# EUROPEAN PATENT SPECIFICATION

(11) **EP 2 091 538 B1**
(45) Date of publication and mention of the grant of the patent: **03.03.2010**
(21) Application number: 07857765.7
(22) Date of filing: 18.12.2007
(51) Int. Cl.: A61K 31/502

(54) **4-BENZYL-1(2H)-PHTHALAZINONES AS H1 RECEPTOR ANTAGONISTS**
4-BENZYL-1(2H)-PHTHALAZINONE ALS H1-REZEPTOR-ANTAGONISTEN
4-BENZYL-1(2H)-PHTHALAZINONES COMME ANTAGONISTES DU RECEPTEUR H1

(30) Priority: 20.12.2006 US 870875 P; 09.10.2007 US 978447 P; 30.11.2007 US 991227 P
(43) Date of publication of application: 26.08.2009
(73) Proprietor: Glaxo Group Limited, Greenford Middlesex UB6 0NN (GB)
(72) Inventor: GORE, Paul Martin, Hertfordshire SG1 2NY (GB); KRANZ, Michael Joachim, Hertfordshire SG1 2NY (GB); LOOKER, Brian Edgar, Hertfordshire SG1 2NY (GB); VILE, Sadie, Hertfordshire SG1 2NY (GB)
(74) Representative: Povey, Alexander W.G.
(86) International application number: PCT/EP2007/064140
(87) International publication number: WO 2008/074803

(56) References cited:
- EP-A- 0 590 551
- WO-A-2007/122156
- DE-A- 3 634 942

## Description

The present invention relates to a class of compounds, processes for their preparation, pharmaceutical compositions containing them and to their use in the treatment of various inflammatory and/or allergic diseases, in particular inflammatory and/or allergic diseases of the respiratory tract.

Allergic rhinitis, pulmonary inflammation and congestion are medical conditions that are often associated with other conditions such as asthma, chronic obstructive pulmonary disease (COPD). In general, these conditions are mediated, at least in part, by inflammation associated with the release of histamine from various cells, in particular mast cells.

Allergic rhinitis, also known as 'hay fever' affects a large proportion of the population worldwide. There are two types of allergic rhinitis, seasonal and perennial. The clinical symptoms of seasonal allergic rhinitis typically include nasal itching and irritation, sneezing and watery rhinorrhea, which is often accompanied by nasal congestion. The clinical symptoms of perennial allergic rhinitis are similar, except that nasal blockage may be more pronounced. Either type of allergic rhinitis may also cause other symptoms, such as itching of the throat and/or eyes, epiphora and oedema around the eyes. The symptoms of allergic rhinitis may vary in intensity from the nuisance level to debilitating.

Allergic rhinitis and other allergic conditions are associated with the release of histamine from various cell types, but particularly mast cells. The physiological effects of histamine are classically mediated by three receptor subtypes, termed H1, H2 and H3. H1 receptors are widely distributed throughout the CNS and periphery, and are involved in wakefulness and acute inflammation. H2 receptors mediate gastric acid secretion in response to histamine. H3 receptors are present on the nerve endings in both the CNS and periphery and mediate inhibition of neurotransmitter release [Hill et al., Pharmacol. Rev., 49:253-278, (1997)]. Recently a fourth member of the histamine receptor family has been identified, termed the H4 receptor [Hough, Mol. Pharmacol., 59:415-419, (2001)]. Whilst the distribution of the H4 receptor appears to be restricted to cells of the immune and inflammatory systems, a physiological role for this receptor remains to be identified.

The activation of H1 receptors in blood vessels and nerve endings are responsible for many of the symptoms of allergic rhinitis, which include itching, sneezing, and the production of watery rhinorrhea. Oral antihistamine compounds which are selective H1 receptor antagonists, such as chlorphenyramine, cetirizine, desloratidine and fexofenadine are effective in treating the itching, sneezing and rhinorrhea associated with allergic rhinitis. Intranasal antihistamines which are selective H1 receptor antagonists, such azelastine and levocabastine, are thought to have similar therapeutic effects to their oral counterparts. However, such compounds generally require twice daily administration and may still cause sedatation despite their local application.

A novel class of compounds have been identified that are H1 receptor antagonists.

Thus, the present invention provides, in a first embodiment, a compound of formula (I) 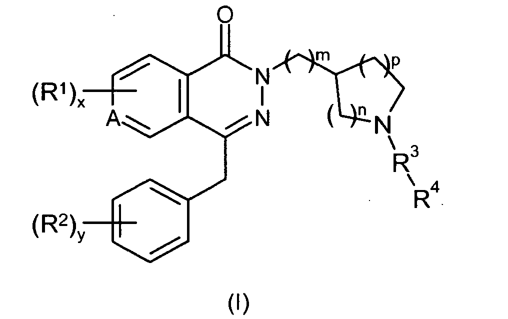 in which:
A represents N or CH;
R¹ and R² each independently represent halogen, C₁₋₃alkyl, C₁₋₃alkoxy, hydroxy or trifluoromethyl;
x and y each independently represent 0, 1 or 2;
m represents 0, n represents 2 and p represents 1; or
m represents 1, n represents 0 and p represents 2;
R³ represents a straight chain C₁₋₆alkylene optionally substituted by one or two C₁₋₃alkyl; and
R⁴ represents a group of formula (i), (ii), (iii) or (iv)

(i) -C(O)N(R⁵)R⁶ in which
   R⁵ represents hydrogen or -C₁₋₆alkyl;
   R⁶ represents hydrogen; -C₁₋₆alkyl (optionally substituted by up to three substituents independently selected from halogen and hydroxy); -C₁₋₆alkyl-O-C₁₋₆alkyl (optionally substituted by up to three substituents independently selected from halogen and hydroxy); C₃₋₇cycloalkyl (optionally substituted by up to three substituents independently selected selected from halogen, hydroxy and C₁₋₃alkyl); -C₁₋₃alkylC₃₋₇cycloalkyl (in which the C₁₋₃alkyl is optionally substituted by up to three substituents independently selected from halogen and hydroxy, and the C₃₋₇cycloalkyl is optionally substituted by up to three substituents independently selected from halogen, hydroxy and C₁₋₃alkyl); aryl (optionally substituted by up to three substituents independently selected from halogen, C₁₋₃alkyl, trifluoromethyl, and cyano); -C₁₋₆alkylaryl (in which the C₁₋₆alkyl is optionally substituted by up to three substituents independently selected from C₁₋₃alkyl, halogen and hydroxy, and the aryl is optionally substituted by up to three substituents independently selected from halogen, C₁₋₃alkyl, trifluoromethyl, and cyano); or -(C₁₋₃alkyl)_{z}Het, in which z represents 0 or 1 and Het represents a 5 to 7 membered saturated heterocyclic ring containing one or two heteroatoms independently selected from O and S;
   or R⁵ and R⁶ together with the N atom to which they are attached represent a 5 to 7 membered saturated heterocyclic ring optionally containing one or two further heteroatoms independently selected from O and S;
(ii) -N(R⁷)C(O)R⁸ in which
   R⁷ represents hydrogen or -C₁₋₆alkyl; and
   R⁸ represents -C₁₋₆alkyl (optionally substituted up to three substituents independently selected from halogen and hydroxy); -C₁₋₆alkyl-O-C₁₋₆alkyl (optionally substituted up to three substituents independently selected from halogen and hydroxy); C₃₋₇cycloalkyl (optionally substituted by up to three substituents independently selected selected from halogen, hydroxy and C₁₋₃alkyl); -C₁₋₃alkylC₃₋₇cycloalkyl (in which the C₁₋₃alkyl is optionally substituted by up to three substituents independently selected from halogen and hydroxy, and the C₃₋₇cycloalkyl is optionally substituted by up to three substituents independently selected from halogen, hydroxy and C₁₋₃alkyl); aryl (optionally substituted by up to three substituents independently selected from halogen, C₁₋₃alkyl, trifluoromethyl, and cyano); - C₁₋₆alkylaryl (in which the C₁₋₆alkyl is optionally substituted by up to three substituents independently selected from C₁₋₃alkyl, halogen and hydroxy, and the aryl is optionally substituted by up to three substituents independently selected from by halogen, C₁₋₃alkyl, trifluoromethyl, and cyano); or -(C₁₋₃alkyl)_{z}Het, in which z represents 0 or 1 and Het represents a 5 to 7 membered saturated heterocyclic ring containing one or two heteroatoms independently selected from O and S; 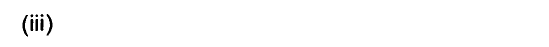 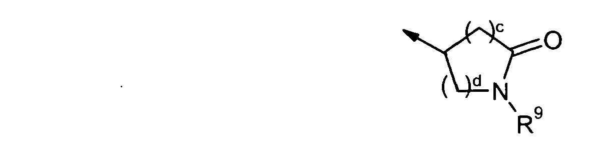 in which c represents 0 or 1 and d represents 2 or 3, or c represents 2 or 3 and d represents 0 or 1; and
   R⁹ represents hydrogen or C₁₋₆alkyl; 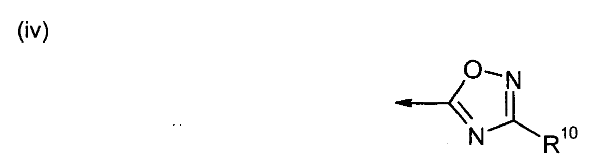 in which R¹⁰ represents hydrogen, or -C₁₋₆alkyl (optionally substituted by up to three substituents independently selected from halogen and hydroxy); -C₁₋₆alkyl-O-C₁₋₆alkyl (optionally substituted by up to three substituents independently selected from halogen and hydroxy); aryl (optionally substituted by up to three substituents independently selected from halogen, C₁₋₃alkyl, trifluoromethyl, and cyano); or -C₁₋₆alkylaryl (in which the C₁₋₆alkyl is optionally substituted by up to three substituents independently selected from C₁₋₃alkyl, halogen and hydroxy, and the aryl is optionally substituted by up to three substituents independently selected from halogen, C₁₋₃alkyl, trifluoromethyl, and cyano);
   or a salt thereof.

The compounds of the invention may be expected to be useful in the treatment of various diseases in particular inflammatory and/or allergic diseases, such as inflammatory and/or allergic diseases of the respiratory tract (for example allergic rhinitis) that are associated with the release of histamine from cells such as mast cells. Further, the compounds may show an improved profile in that they may possess one or more of the following properties:
(i) greater selectivity over the H3 receptor;
(ii) lower CNS penetration;
(iii) prolonged duration of action;
(iv) selectivity over the hERG receptor and/or the alpha receptor; and
(v) lower bioavailability/oral absorption.

Compounds having such a profile may be particularly suitable for intranasal delivery, and/or may be capable of once daily administration and/or further may have an improved side effect profile compared with other existing therapies.

By 'selectivity' it is meant that the compounds may be more potent at the H1 receptor than at the H3 receptor, hERG and/or the alpha receptor, in particular the H3 receptor. The activity at the H1 receptor may be at least about 10 fold greater (e.g. greater than 100 fold) than activity at the H3 receptor, hERG and/or the alpha receptor, in particular the H3 receptor.
In a second embodiment there is provided a compound of formula (I) having the formula (1a) below 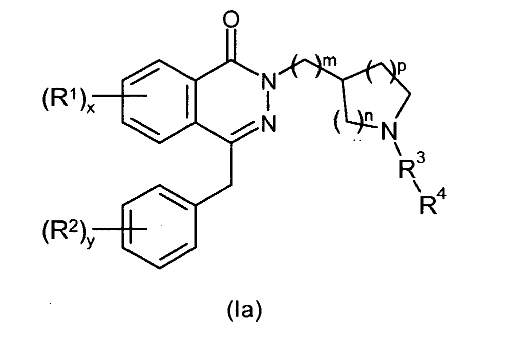 in which:
R¹ and R² each independently represent halogen, C₁₋₃alkyl, C₁₋₃alkoxy, hydroxy or trifluoromethyl;
x and y each independently represent 0, 1 or 2.
m represents 0, n represents 2 and p represents 1; or
m represents 1, n represents 0 and p represents 2;
R³ represents a straight chain C₁₋₆alkylene optionally substituted by C₁₋₃alkyl; and
R⁴ represents a group C(O)NR⁵R⁶ or -N(R⁷)C(O)R⁸ in which
R⁵ represents hydrogen or -C₁₋₆alkyl;
R⁶ represents hydrogen; -C₁₋₆alkyl optionally substituted by halogen; -C₁₋₆alkyl-O-C₁₋₆alkyl optionally substituted by halogen; or -C₁₋₆alkylaryl, in which the alkyl portion is optionally substituted by C₁₋₃alkyl, and the aryl portion is optionally substituted by halogen, C₁₋₃alkyl, trifluoromethyl, or cyano;
or R⁵ and R⁶ together represent a 5 to 7 membered saturated heterocyclic ring optionally containing one or more further heteratoms selected from O and S;
R⁷ represents hydrogen or C₁₋₆alkyl, and
R⁸ represents C₁₋₆alkyl optionally substituted by halogen; C₁₋₆alkoxy optionally substituted by halogen; -C₁₋₆alkyl-O-C₁₋₆alkyl optionally substituted by halogen;
or -C₁₋₆alkylaryl, in which the alkyl portion is optionally substituted by C₁₋₃alky), and the aryl portion is optionally substituted by halogen, C₁₋₃alkyl, trifluoromethyl, or cyano;
or a salt thereof.

It will be understood that C(O)NR⁵R⁶ is C(O)N(R⁵)R⁶.

In another embodiment, R⁴ represents -C(O)N(R⁵)R⁶.

In another embodiment, R⁴ represents -N(R⁷)C(O)R⁸.

In another embodiment, R⁴ represents

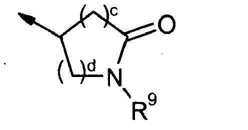

In another embodiment, R⁴ represents 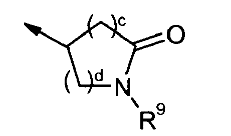 in which c represents 0, d represents 3 and R⁹ represents hydrogen or C₁₋₆alkyl (e.g. C₁₋₃alkyl); in a further embodiment R⁹ represents hydrogen.

In another embodiment, R⁴ represents

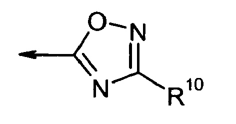

In another embodiment, A represents CH.

In another embodiment, A represents N.

In another embodiment, R² represents halogen; in a further embodiment, R² represents chloro; in yet a further embodiment R² represents chloro substituted in the para position. In another embodiment when y is 1, R² is in the para position.

In another embodiment x is 0.

In another embodiment, y is 1.

In another embodiment m represents 0, n represents 2 and p represents 1.

In another embodiment, m represents 1, n represents 0 and p represents 2.

In another embodiment, R³ represents a straight chain C₁₋₃alkylene optionally substituted by methyl. Representative examples of R³ include methlyene [-(CH₂)-], ethylene [-(CH₂)₂-], and propylene [-(CHO₂)₃-], each of which may be optionally substituted by methyl.

In another embodiment, R⁵ represents hydrogen or -C₁₋₃alkyl, for example -C₁₋₂alkyl.

In another embodiment, R⁶ represents hydrogen; -C₁₋₄alkyl optionally substituted by halogen (for exampte by one or more fluoro atoms, for example by 3 fluoro atoms); -C₁₋₃alkyl-O-C₁₋₂alkyl optionally substituted by halogen (for example by one or more fluoro atoms, for example by 3 fluoro atoms); or -CH₂aryl.

In another embodiment, R⁶ represents hydrogen; -C₁₋₄alkyl optionally substituted by halogen (for example by one or more fluoro atoms, for example by 3 fluoro atoms); -C₁₋₃alkyl-O-C₁₋₃alkyl optionally substituted by halogen (for example by one or more fluoro atoms, for example by 3 fluoro atoms); C₅₋₆cycloalkyl; -C₁₋₃alkyl C₅₋₆cycloalkyl (for example -CH₂C₅₋₆cycloalkyl); or -C₁₋₃alkyl aryl (for example CH₂aryl).

In another embodiment, R⁶ represents -(C₁₋₃alkyl)_{z}Het in which z represents 0 or 1 and Het represents a 5 to 7 (for example a 5 to 6) membered saturated heterocyclic ring containing one O atom.

In another embodiment, R⁵ and R⁶ together represent a 5 or 6 membered saturated heterocyclic ring optionally containing one 0 atom, for example a 6 membered saturated heterocyclic ring optionally containing one O atom.

In another embodiment, R⁷ represents hydrogen or methyl.

In another embodiment, R⁷ represents hydrogen.

In another embodiment, R⁸ represents C₁₋₄alkyl optionally substituted by halogen (for example by one or more fluoro atoms, for example by 3 fluoro atoms); or -C₁₋₃alkyl-O-methyl.

In another embodiment, R⁸ represents C₁₋₄alkyl optionally substituted by halogen (for example by one or more fluoro atoms, for example by 3 fluoro atoms); -C₁₋₃alkyl-O-methyl; C₅₋₆cycloalkyl; -C₁₋₃alkyC₅₋₆cycloalkyl (for example -CH₂C₅₋₆cycloalkyl); or -C₁₋₃alkylaryl (for example CH₂aryl).

In another embodiment, R⁸ represents -(C₁₋₃alkyl)_{z}Het in which z represents 0 or 1 and Het represents a 5 to 7 (for example a 5 to 6) membered saturated heterocyclic ring containing one O atom.

In another embodiment, R⁹ represents hydrogen or C₁₋₃alkyl, for example hydrogen or methyl.

In another embodiment, R¹⁰ represents C₁₋₃alkyl or C₁₋₃alkyl-O-methyl.

Compounds of the invention include the compounds of Examples 1-84 including individual isomers thereof and isomeric mixtures (e.g. a racemate or a racemic mixture), and salts thereof (e.g. a pharmaceutically acceptable salt).

Of particular interest are the compounds:
*N*-[2-((2*R*)-2-{[4-[(4-Chlorophenyl)methyl]-1-oxo-2(1*H*)-phthalazinyl]methyl}-1-pyrrolidinyl)ethyl]-4-(methyloxy)butanamide, or a salt thereof.
*N*-[2-((2R)-2-{[4-[(4-chlorophenyl)methyl]-1-oxo-2(1*H*)-phthalazinyl]methyl)-1-pyrrolidinyl)ethyl]-3,3,3-trifluoropropanamide, or a salt thereof.

Thus, in one embodiment there is provided a compound which is *N*-[2-((2R)-2-{[4-[(4-chlorophenyl)methyl]-1-oxo-2(1*H*)-phthalazinyl]methyl}-1-pyrrolidinyl)ethyl]-4-(methyloxy)butanamide, or a salt thereof.

In another embodiment there is provided a compound which is *N*-[2-((2*R*)2-{[4-[(4-chlorophenyl)methyl]-1-oxo-2(1*H*)-phthalazinyl]methyl}-1-pyrrolidinyl)ethyl]-4-(methyloxy)butanamide, or a pharmaceutically acceptable salt thereof.

In another embodiment there is provided a compound which is *N*-[2-((2*R*)2-{[4-[(4-chlorophenyl)methyl]-1-oxo-2(1*H*)-phthalazinyl]methyl}-1-pyrrolidinyl)ethyl]-4-(methyloxy)butanamide in the form of its free base.

In another embodiment there is provided a compound which is *N*-[2-((2*R*)-2-{[4-[(4-chlorophenyl)methyl]-1-oxo-2(1*H*)-phthalazinyl]methyl}-1-pyrrolidinyl)ethyl]-4-(methyloxy)butanamide in the form of a tartrate salt, particularly an L-tartrate salt.

In another embodiment there is provided a compound of formula (I) or a salt thereof with the proviso that the compound is not *N*-[2-((2*R*)-2-{[4-[(4-chlorophenyl)methyl]-1-oxo-2(1*H*)-phthalazinyl]methyl}-1-pyrrolidinyl)ethyl]-4-(methyloxy)butanamide, or a salt thereof.

C₁₋₆alkyl, whether alone or as part of another group, may be a straight or branched hydrocarbon chain, unless otherwise indicated and C₁₋₆alkoxy shall be interpreted similarly. Representative examples include, but are not limited to methyl, ethyl, propyl, *iso*-propyl, *n*-butyl, *sec*-butyl, *iso*-butyl, *tert*-butyl, *n*-pentyl, *neo*-pentyl and *n*-hexyl. Particular alkyl and alkoxy groups are C₁₋₃alkyl and C₁₋₃ alkoxy. When substituted, C₁₋₆alkyl (e.g. C₁₋₃alkyl) and C₁₋₆alkoxy (e.g. C₁₋₃ alkoxy) may have up to three substituents, for example, one or two substituents, e.g. one substituent. Representative substituents on alkyl and/or alkoxy include, but are not limited to, methyl, ethyl, chloro, and/or fluoro.

Representative examples of C₁₋₆alkylene include methlyene [-(CH₂)-], ethylene [-(CH₂)₂-], propylene, [-(CH₂)₃-], butylene [-(CH₂)₄-], pentylene [-(CH₂)₅-] and hexylene [-(CH₂)₆-].

C₃₋₇cyctoalkyl refers to a non-aromatic cyclic hydrocarbon ring having from three to seven carbon atoms. Representative examples include, but are not limited to, cyclopropyl, cyclobutyl, cyclopentyl and cyclohexyl. When substituted, C₃₋₇cycloalkyl may have up to three substituents, for example, one or two substituents, e.g. one substituent. Representative substituents on cycloalkyl include, but are not limited to, methyl, ethyl, chloro, and fluoro.

The term 'aryl' whether alone or as part of another group is used to describe single and fused aromatic hydrocarbon rings. Representative aryl rings include phenyl and naphthyl. A particular aryl ring is phenyl. When substituted, aryl may have up to three substituents, for example, one or two substituents, e.g. one substituent. Representative substituents on aryl include, but are not limited to, methyl, ethyl, chloro, fluoro, trifluromethyl and/or cyano.

The term "halogen" is used herein to describe, unless otherwise stated, a group selected from fluorine, chlorine, bromine and iodine, for example fluorine and chlorine.

Representative 5 to 7 membered saturated heterocyclic rings containing a nitrogen atom optionally containing one or two (e.g. one) further heteratoms independently selected from O and S include, but are not limited to, pyrrolidine, piperidine, homopiperidine, morpholine and thiomorpholine.

Representative 5 to 7 membered saturated heterocyclic rings containing one or two (e.g. one) heteratoms independently selected from O and S include, but are not limited to, tetrahydrothiophene (or thiolane), tetrahydrothiopyran (or thiane), hexahydrothiepin, hexahydrooxepin, tetrahydrofuran, tetrahydropyran, oxathiolane, thioxane, tetrahydrooxathiepin.

It is to be understood that the invention includes all combinations of embodiments, representative groups and substituents described herein.

It will be appreciated that compounds of formula (I) may possess one or more asymmetric carbon atoms so that optical isomers e.g. enantiomers or diastereoisomers may be formed. The present invention encompasses all optical isomers of the compounds of formula (I) whether as individual isomers isolated such as to be substantially free of the other isomer (i.e. pure) or as mixtures thereof (i.e. racemates and racemic mixtures). An individual isomer isolated such as to be substantially free of the other isomer (i.e. pure) may be isolated such that less than about 10%, particularly less than about 1%, for example less than about 0.1% of the other isomer is present.

Certain compounds of formula (I) may exist in one of several tautomeric forms. It will be understood that the present invention encompasses all tautomers of the compounds of formula (I) whether as individual tautomers or as mixtures thereof.

The compounds of formula (I) may be in crystalline or amorphous form. Furthermore, a compound of formula (I) may exist in one or more polymorphic forms. Thus, the present invention includes within its scope polymorphic forms of the compounds of formula (I). In general, the most thermodynamically stable polymorphic form of a compound of formula (I) is of particular interest.

Polymorphic forms of compounds of formula (I) may be characterized and differentiated using a number of conventional analytical techniques, including but not limited to X-ray powder diffraction (XRPD) patterns, infrared (IR) spectra, Raman spectra, differential scanning calorimetry (DSC), thermogravimetric analysis (TGA) and solid state nuclear magnetic resonance (NMR).

The compounds of the present invention may be in the form of and/or may be administered as a pharmaceutically acceptable salt. For a review on suitable salts see Berge et al., J. Pharm. Sci., 1977, 66, 1-19. Suitable pharmaceutically acceptable salts include acid and base addition salts. As used herein, the term "pharmaceutically acceptable salt", means any pharmaceutically acceptable salt of a compound of the invention, which upon administration to the recipient is capable of providing (directly or indirectly) a compound of the invention, or an active metabolite or residue thereof.

Typically, a pharmaceutically acceptable salt may be readily prepared by using a desired acid or base as appropriate. The salt may precipitate from solution and be collected by filtration or may be recovered by evaporation of the solvent.

A pharmaceutically acceptable acid addition salt can be formed by reaction of a compound of formula (I) with a suitable inorganic or organic acid (such as hydrobromic, hydrochloric, formic, sulfuric, nitric, phosphoric, succinic, maleic, acetic, fumaric, citric, tartaric, benzoic, *p-*toluenesulfonic, methanesulfonic or naphthalenesulfonic acid), optionally in a suitable solvent such as an organic solvent, to give the salt which is usually isolated for example by crystallisation and filtration. Thus, a pharmaceutically acceptable acid addition salt of a compound of formula (I) can be for example a hydrobromide, hydrochloride, formate, sulfate, nitrate, phosphate, succinate, maleate, acetate, fumarate, citrate, tartrate, benzoate, *p*-toluenesulfonate, methanesulfonate or naphthalenesulfonate salt.

Other non-pharmaceutically acceptable salts, eg. oxalates or trifluoroacetates, may be used, for example in the isolation of compounds of the invention, and are included within the scope of this invention. The invention includes within its scope all possible stoichiometric and non-stoichiometric forms of the salts of the compounds of formula (I).

Thus, it is to be understood that the present invention covers the compounds of formula (I) as the free base and as salts thereof, for example, as pharmaceutically acceptable salts.

It will be appreciated that many organic compounds can form solvates with the solvents in which they are reacted or from which they are precipitated or crystallized. For example, a solvate with water is known as a "hydrate". Solvents with high boiling points and/or solvents with a high propensity to form hydrogen bonds such as water, xylene, *N*-methyl pyrrolidinone and methanol may be used to form solvates. Methods for identification of solvates include, but are not limited to, NMR and microanalysis. Thus, solvates of the compounds of formula (I) are within the scope of the invention.

It will be appreciated from the foregoing that included within the scope of the invention are salts e.g. pharmaceutically acceptable salts, solvates e.g. hydrates, isomers and polymorphic forms of the compounds of the invention.

Accordingly, it will be appreciated that references hereinafter to compounds of the invention or to compounds of formula (I) means a compound of formula (I) as the free base, or as a salt e.g. a pharmaceutically acceptable salt, and encompasses solvates, isomers and polymorphic forms of those compounds.

The present invention also provides processes for the preparation of compounds of formula (I) or salts thereof.

According to a first process, A, a compound of formula (I) wherein R⁴ represents a group C(O)N(R⁵)R⁶ may be prepared by reacting a compound of formula (II) 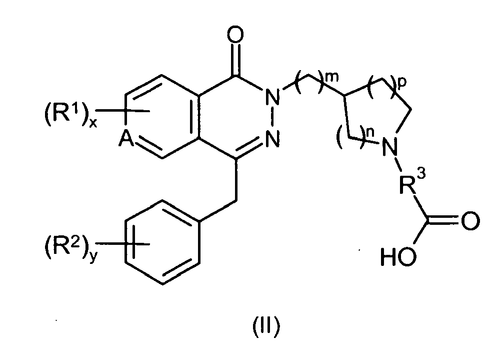 in which A, R¹, R², R³, n, m, p, x and y are as defined above for formula (I) with an amine of formula (III):

HN(R⁵)R⁶ (III)

in which R⁵ and R⁶ are as defined above for formula (I) under suitable coupling conditions.

The coupling reaction may typically be carried out using a suitable activating agent, for example *O*-(1*H*-benzotriazol-1-yl)-*N,N,N',N*-tetramethyluronium tetrafluoroborate (TBTU), 1-[bis(dimethylamino)methylene]-1*H*-benzotriazolium hexafluorophosphate (HBTU), or benzotriazol-1-yl-oxy-trispyrrolidinylphosphonium hexafluorophosphate (PyBOP) in the presence of a suitable base for example triethylamine or *N,N*-diisopropylethylamine (DIPEA) in a suitable solvent such as *N,N*-dimethylformamide (DMF), optionally at an elevated temperature, for example at about 140 to 160 °C for approximately 10 to 40 min, as appropriate.

In one embodiment of process A, A represents CH.

Compounds of formula (II) may be prepared by alkylating a compound of formula (IV) 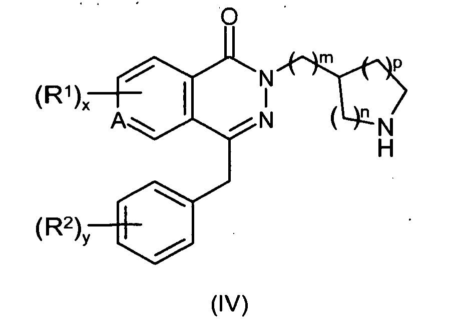 in which A, R¹, R², n, m, p, x and y are as defined above for formula (I), with a compound of formula (V): 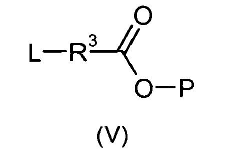 in which R³ is as defined above for formula (I), L is a suitable leaving group [such as a halide (for example, chloro, bromo or iodo) or a sulfonate (for example mesylate, tosylate or triflate)] and P is a protecting group, such as an alkyl group (for example methyl, ethyl or *tert*-butyl) or benzyl; to give a protected derivative (Ila): 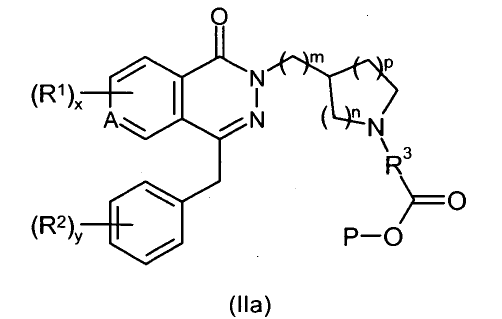 in which A, R¹, R², R³, n, m, p, x and y are as defined above for formula (I), and P is a protecting group, such as an alkyl group (for example methyl, ethyl or *tert*-butyl) or benzyl, followed by deprotection, to give a compound of formula (II).

The alkylation reaction may be carried out in a suitable solvent, for example, DMF in the presence of a suitable base, for example potassium carbonate, optionally at an elevated temperature such as about 80 °C, for example in a microwave oven. The reaction may optionally contain an activating agent, such as sodium iodide. For a particularly reactive compound of formula (V), the reaction may be carried out at room temperature, or lower.

Compounds of formula (IIa) may be isolated, if desired, prior to deprotection to give a compound of formula (II).

Compounds of formula (II) in which R³ is ethylene may also be prepared by reacting a compound of formula (IV) with a compound of formula (VI) 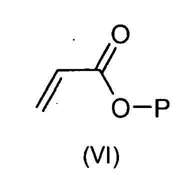 in which P is a protecting group, such as an alkyl group (for example methyl, ethyl or *tert-*butyl) or aralkyl such as benzyl. The alkylation reaction using a compound of formula (VI) may be carried out in a suitable solvent, for example DMF or tetrahydrofuran (THF), optionally at an elevated temperature, for example about 50 to 60 °C.

Deprotection of a compound of formula (IIa) to give a compound of formula (II), when the protecting group P is an alkyl group (for example methyl or ethyl) or benzyl, is typically carried out by hydrolysis of the carboxylic acid ester, for example using sodium hydroxide, potassium hydroxide or lithium hydroxide in a suitable aqueous solvent system, for example methanol/water or THF/water at room temperature (or optionally at an elevated temperature), optionally followed by purification on an ion exchange column (for example an SCX or aminopropyl column).

When the protecting group P is *tert*-butyl, deprotection may also be achieved by acid hydrolysis using a suitable acid such as trifluroactetic acid (TFA) in dichloromethane (DCM) or hydrogen chloride (HCI) in dioxane.

When the protecting group is benzyl, deprotection may also be achieved by hydrogenolysis, for example using hydrogen over a suitable catalyst, such as palladium on carbon.

Compounds of formula (III), (V) and (VI) are commercially available, for example, from Aldrich, ABCR, Rare Chemicals, Sigma, BDH, Apollo, Acros, Berry & Associates, Flurochem, Fluka, Enamine, Chembridge, TCI-Europe and/or GL Synthesis, and/or may be prepared according to methods described herein, and/or may be prepared by methods well-known to those skilled in the art.

Compounds of formula (III) include, but are not limited to, ammonia, methylamine, (*R*)-(-)-2-aminobutane, hexylamine, dimethylamine, dihexylamine, 2-fluoroethylamine, 2,2-difluoroethylamine, 2,2,2-trifluoroethylamine, 2-aminoethanol, cyclopropylamine, cycloheptylamine, *N*-methylcyclohexylamine, *N*-(1-ethylpropyl)cycloheptylamine, (S)-tetrahydrofuran-3-amine, aniline, 2-aminobenzonitrile, 2-fluoroaniline, 2-aminobenzotrifluoride, 3-amino-4-fluorobenzotrffluoride, *N*-hexylaniline, benzylamine, (5-phenylbutyl)methylamine hydrochloride, pyrrolidine, thiazolidine, morpholine, 3-ethoxypropylamine, 1-amino-3-methoxy-propan-2-ol and 6,7-dihydroxy-4-oxaheptylamine.

Compounds of formula (V) include, but are not limited to, ethyl 2-bromoacetate, ethyl 7-bromoheptanoate and benzyl chloroacetate. Alternatively, compounds of formula (V) in which L represents a sulfonate may be prepared by activation of a corresponding hydroxyl compound by methods well known to those skilled in the art, such as by reaction with mesyl chloride or tosyl chloride or with truflic anhydride. Examples of corresponding hydroxyl compounds include (+)-*tert*-butyl D-lactate and benzyl glycolate.

Compounds of formula (VI) include, but are not limited to, methyl acrylate, ethyl acrylate and ethyl crotonate. Compounds of formula (IV) may be prepared according to the methods described herein (see Schemes 1 and 2). Thus, compounds of formula (IV) wherein A represents CH may be prepared according to Scheme 1 below: 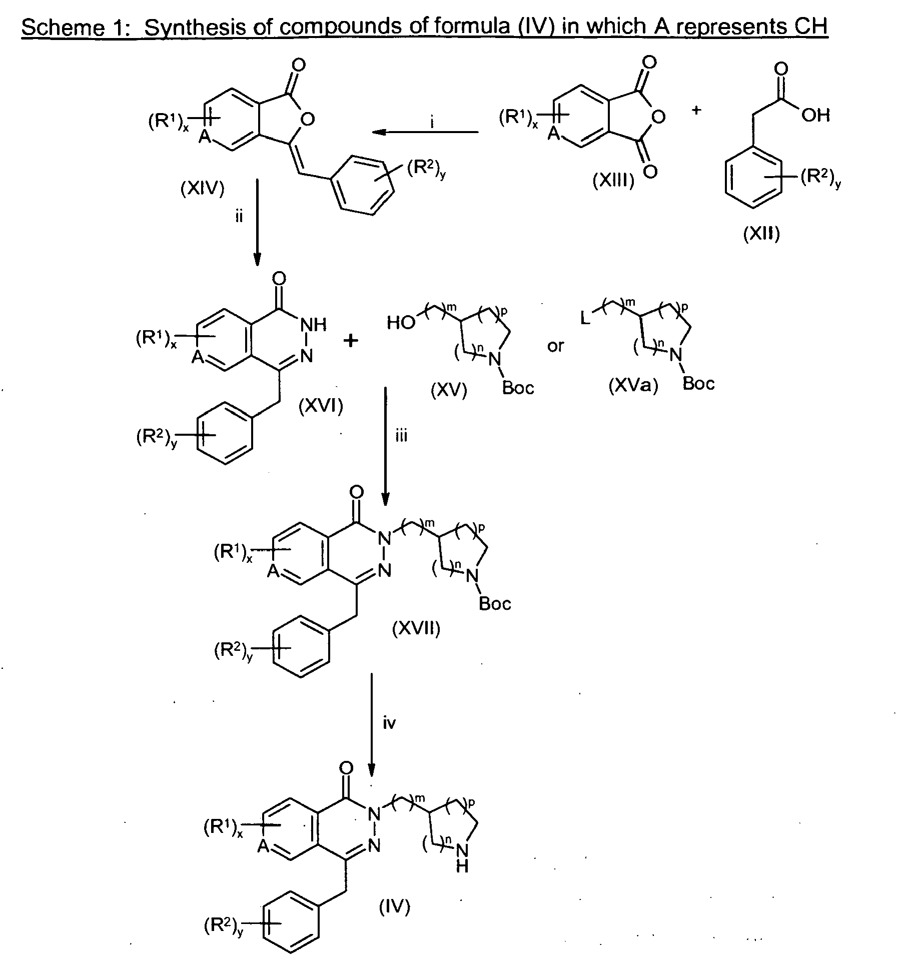 in which A represents CH, R¹, R², n, m, p, x and y are as defined above for formula (I), Boc represents *tert*-butoxycarbonyl and L represents a suitable leaving group such as a sulfonate e.g. mesylate or tosylate.

**Reagents and Conditions:** i) elevated temperature such as about 180 to 250 °C e.g. about 240 °C, suitable base e.g. sodium acetate (NaOAc), suitable solvent such as *N-*methyl-2-pyrrolidinone (NMP); ii) NH₂NH₂, hydrazine sulphate or hydrazine monohydrate and sodium hydroxide (NaOH), in a suitable solvent such as ethanol, optionally at an elevated temperature such as from about 80 °C to 90 °C; iii) when using a compound of formula (XV), reaction conditions are as follows: suitable solvent e.g. THF, appropriate azodicarboxylate e.g. diisopropylazodicarboxylate (DIAD) or other reagent such as di-*tert-*butyl azodicarboxylate (TBAD), suitable phosphine e.g. triphenylphosphine (PPh₃), optionally at a lowered temperature; when using a compound of formula (XVa), reaction conditions are as follows: suitable solvent such as butan-2-ol, suitable base e.g. cesium carbonate, at an elevated temperature such as from about 90 °C to 110 °C; iv) deprotection using an acid catalyst e.g. HCl or TFA, in a suitable solvent e.g. dioxane, iso-propyl alcohol or DCM, optionally at an elevated temperature such as from about 70 °C to 90 °C.

In a modification of the synthesis described above, steps (iii) and (iv) may be performed sequentially, without isolation and purification of the Boc-protected intermediate.

Compounds of formula (XIII) are commercially available from Sigma-Aldrich, Apollo, Fluorochem, Apin, Davos and/or Merck, and include, but are not limited to, phthalic anhydride, 3-chlorophthalic anhydride, 4-fluorophthalic anhydride, 3,6-dichlorophthalic anhydride, 3-hydroxyphthalic anhydride and 4-methylphthalic anhydride and/or may be prepared using methods well known to those skilled in the art. For example 3,6-dihydroxyphthalic anhydride may be prepared from 3,6-diacetoxyphthalic anhydride, which is commercially available from Wako. C₁₋₆alkyl substituted phthalic anhydrides may be prepared using methods well known to those skilled in the art from the commercially available bromide compounds. Such reactions may typically be carried out using the appropriate trialkylborane (for example, triethylborane and tributylborane are available for example from Sigma-Aldrich), with an appropriate palladium catalyst, such as [1,1'-bis(diphenylphosphino)ferrocene] dichloropalladium at an appropriate elevated temperature e.g. 70 - 100 °C, with a suitable base such as K₂CO₃, in a suitable solvent e.g. DMF.

Compounds of formula (XII) are commercially available from Sigma-Aldrich and/or Apollo, and include, but are not limited to, phenylacetic acid, 3-chlorophenylacetic acid, 4-methylphenylacetic acid, 4-methoxyphenylacetic acid, 4-hydroxyphenylacetic acid, 3-(trffluoromethyl)phenylacetic acid and 2-fluoro-3-(trifluoromethyl)phenylacetic acid.

Compounds of formula (XIV) include benzalphthalide, 4-fluorobenzylidene phthalide, 3-(2-Bromo-benzylidene)-3H-isobenzofuran-1-one and 4-chlorobenzylidene phthalide which are commercially available, for example, from Honeywell and/or Aldrich and/or Aurora Chemicals.

Compounds of formula (XV) are commercially available from Sigma-Aldrich and/or Fluka, and include (*R*)-1-BOC-2-pyrrolidinemethanol, (*S*)-1-BOC-2-pyrrolidinemethanol and 1-BOC-4-hydroxypiperidine. Compounds of formula (XVa) may be prepared from the commercially available alcohol (XV) by activation, using methyl sulfonyl chloride or tosyl chloride. The reaction is typically carried out in a suitable solvent such as methyl *iso*-butyl ketone, with a suitable base e.g. triethylamine, optionally at a lowered temperature for example at about 0 °C.

Compounds of formula (XVI) in which A represents CH, are disclosed in US patent US4,841,047, Uspatent US1,377,231 and by G. Scheffer et al., in Arch. Pharm., 321:205-208 (1988), (see compound 4) or may be prepared by the methods described herein.

Compounds of formula (IV) in which A represents N may be prepared according to Scheme 2 below. 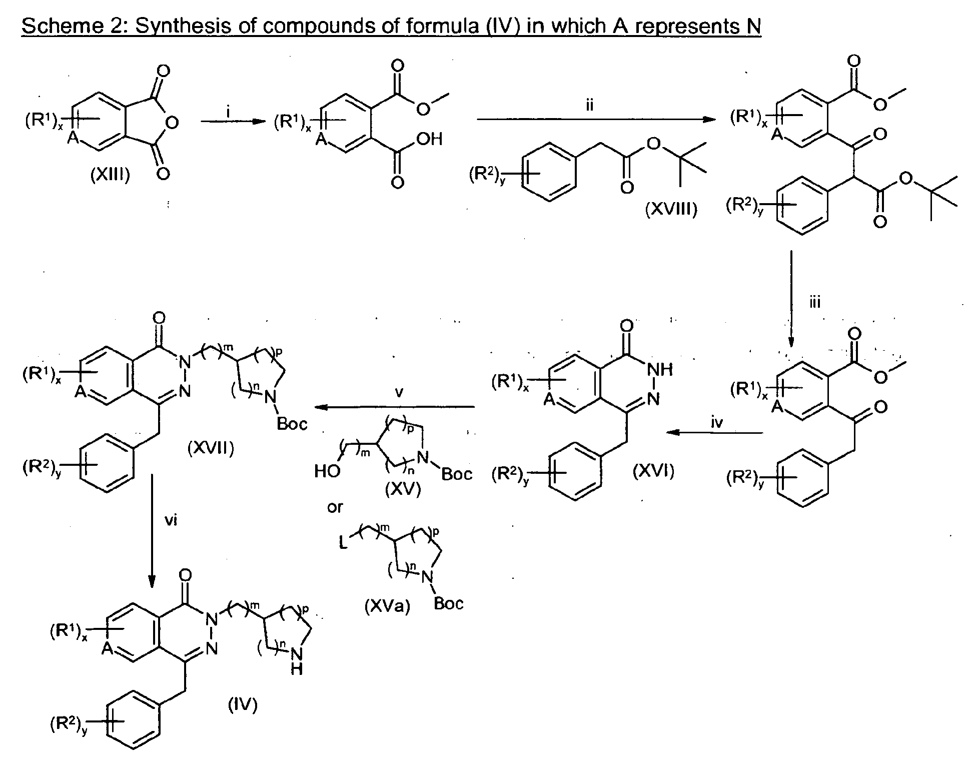 wherein A represents N, R¹, R², y and z are as described hereinabove for formula (I), Boc represents *tert*-butoxycarbonyl and L represents a suitable leaving group such as a sulfonate e.g. mesylate or tosylate.

**Reagents and conditions:** i) Sodium methoxide, THF/methanol(MeOH); ii) a) suitable activating agent such as carbonyl diimidazole or oxalyl chloride, suitable solvent such as DMF, appropriate elevated temperature such as at approximately 50 °C, b) appropriate base for example NaH, c) compound of formula (XVIII); iii) suitable acid catalyst for example TFA, appropriate solvent such as DCM; iv) H₂NNH₂.H₂O, in an appropriate solvent for example ethanol, catalytic amount of acid such as acetic acid; v) when using a compound of formula (XV), reaction conditions are as follows: suitable solvent e.g. THF, appropriate azodicarboxylate e.g. DIAD or other reagent such as TBAD, suitable phosphine e.g. PPh₃, optionally at a lowered temperature; when using a compound of formula (XVa), reaction conditions are as follows: suitable solvent such as butan-2-ol, suitable base e.g. cesium carbonate, at an elevated temperature such as from about 90 °C to 110 °C; vi) deprotection using an acid catalyst e.g. HCl or TFA in a suitable solvent e.g. dioxane, *iso*-propyl alcohol or DCM, optionally at an elevated temperature such as from about 70 °C to 90 °C.

Compounds of formula (XIII) in which A represents N are commercially available, or may be prepared from known methods. For example, pyridine-3,4-dicarboxylic anhydride is available from Sigma-Aldrich. 2-methyl-pyridine-4,5-dicarboxylic anhydride may be prepared according to the methods described by Werner, W. Graefe, U., Ihn, W., Tresselt, D., Winter, S., Paulus, E., Tetrahedron, 53(1):109-118 (1997), see compound 4. 3-Methoxypyridine-4,5-dicarboxylic anhydride may be prepared according to the methods disclosed by Krapcho, A. P., Maresch, M. J., Gallagher, C. E., Hacker, M. P., J. Het. Chem., 32(6):1693-702, (1995), see compound 10. 2-Methyl-3,4-pyridinedicarboxylic anhydride may be prepared according to the methods described by Moriconi, E. J. and Spano, F. A., J. Amer. Chem. Soc., 86(1):38-46, (1964), see compound 14.

Compounds of formula (XVIII) may be prepared by the methods described in Scheme 3, below, or by the methods described in WO 2002/079143 (see Preparation 149). 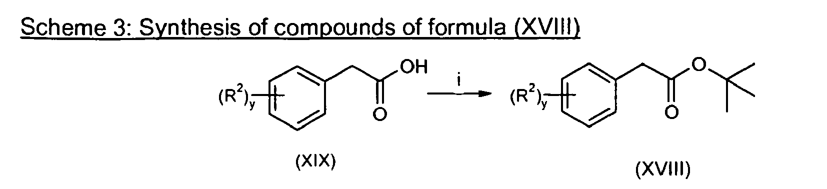 wherein R² and y are as described hereinabove for formula (I).

**Reagents and conditions:** i) dimethylformamide di-*tert*-butyl acetal, suitable solvent such as toluene, elevated temperatue, e.g. about 80 °C, for approximately 18 hours.

Dimethylformamide di-*tert*-butyl acetal is commercially available, for example, from Sigma-Aldrich.

Compounds of formula (XIX) are commercially available from Sigma-Aldrich and/or Apollo, and include, but are not limited to, phenylacetic acid, 3-chlorophenylacetic acid, 4-methylphenylacetic acid, 4-methoxyphenylacetic acid, 4-hydroxyphenylacetic acid, 3-(trifluoromethyl)phenylacetic acid, 2-fluoro-3-(trifluoromethyl)phenylacetic acid, and 4-hydroxy-3-methoxyphenylacetic acid.

According to a second process, B, a compound of formula (I) wherein R⁴ represents N(R⁷)C(O)R⁸ may be prepared by reacting a compound of formula (VII) 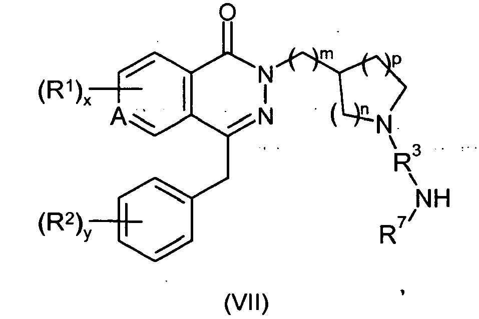 in which A, R¹, R², R³, R⁷, n, m, p, x and y are as defined above for formula (I) with a compound of formula (VIII): 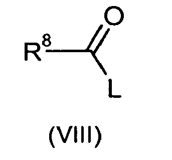 in which R⁸ is as defined above for formula (I) and L is a suitable leaving group, for example hydroxy or chloro. When L is chloro, the reaction may typically be carried out in a suitable solvent such as DCM in the presence of a suitable base such as triethylamine, at room temperature. When L represents hydroxy, the reaction may typically be carried out in a suitable solvent such as *N,N*-dimethylformamide (DMF), with an appropriate base, e.g. triethylamine or *N,N*'-diisopropylehtylamine (DIPEA) and in the presence of a suitable activating agent such as *O*-(benzotriazol-1-yl)*N,N,N',N'-*tetramethyluronium tetrafluoroborate (TBTU), benzotriazol-1-yl-oxytrispyrrolidinophosphonium hexafluorophosphate (PyBop) or 2-(1*H*-7-azabenzotriazol-1-yl)-1,1,3,3-tetramethyl uronium hexafluorophosphate (HATU). Alternative activating agents are T3P® (propane phosphonic acid anhydride) and 1,1'-carbonyldiimidazole (CDI). The reaction is optionally heated, for example using a microwave oven at an appropriate elevated temperature, for example at about 140 to 160 °C for approximately 10 to 40 min, as appropriate.

In one embodiment of process B, A represents CH.

Compounds of formula (VII) may be prepared by alkylating a compound of formula (IV) 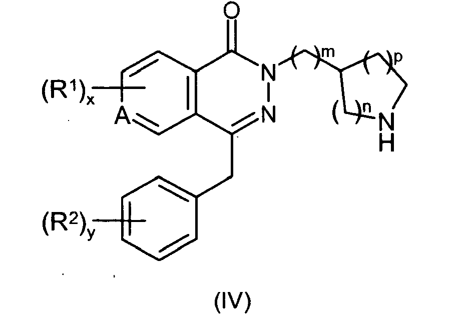 in which A, R¹, R², n, m, p, x and y are as defined above for formula (I) with a compound of formula (IX) 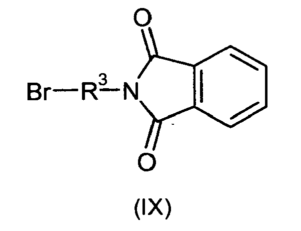 in which R³ is as defined above for formula (I), to form the compound (IVa) 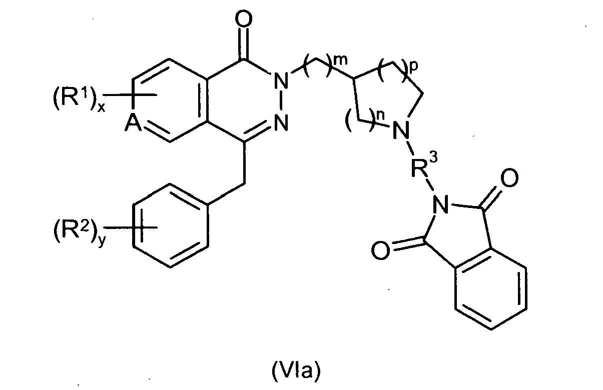 which may be isolated if desired, followed by deprotection to give a compound of formula (VII), and for compounds of formula (VII) in which R⁷ represents C₁₋₆alkyl, alkylation with a compound of formula (X)

L-R⁷ (X)

in which L is a suitable leaving group (for example, chloro, bromo or iodo) or a sulfonate (for example mesylate, tosylate or triflate).

The alkylation reaction may be carried out in a suitable solvent, for example methyl ethyl ketone or 3-pentanone, in the presence of a suitable base, for example potassium carbonate or triethylamine, optionally at an elevated temperature, for example from about 80 °C to 100 °C, to give the phthalimide intermediate compound (IVa), which may be isolated if desired.

Deprotection may then be achieved by hydrazine or hydrazine monohydrate in a suitable solvent for example ethanol, optionally at elevated temperature, for example at from about 80 °C to 120 °C. Alternatively, ethanolamine may be used, optionally in a suitable solvent such as 2-methyl tetrahydrofuran.

Alternatively, a compound of formula (VII) may be prepared by reacting a compound of formula (IV) with a compound of formula (XI): 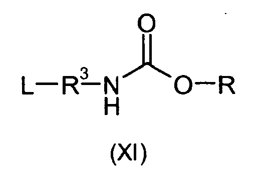 in which L is a suitable leaving group as defined above for formula (X) and R³ is as defined above for formula (I) and R represents C₁₋₆alkyl (e.g. *tert*-butyl) or benzyl.

Suitable reaction conditions include a suitable solvent, for example methyl ethyl ketone, in the presence of a suitable base, for example potassium carbonate, optionally at an elevated temperature, for example about 80 °C.

Compounds of formula (VIII), (IX), (X) and (XI) are either commercially available for example from Aldrich, ABCR, Chembridge, Milestone Pharmtech, Toronto, Timtec, TCl-Europe, Lancaster, Alfa Aesar, Apollo, Matrix, Fluka and/or Acros or may be prepared by known methods or the methods described herein.

Compounds of formula (VIII) in which L represents chloro are commercially available and include, but are not limited to, acetyl chloride, 3,3,3-trirfluoropropionyl chloride, hexanoyl chloride, 2-bromopropionyl chloride, methoxyacetyl chloride, cyclopropanecarbonyl chloride, cyclohexanecarbonyl chloride, benzoyl chloride, 3-fluorobenzoyl chloride, m-toluoyl chloride, 4-(trifluoromethyl)benzoyl chloride, 4-cyanobenzoyl chloride, 3,5-bis(trifluoromethyl)benzoyl chloride, 3-cyano-4-fluorobenzoyl chloride, 4-phenylbutyryl chloride and 2-(4-chlorophenyl)-3-methylbutyryl chloride.

Compounds of formula (VIII) in which L represents hydroxy which are commercially available include, but are not limited to, formic acid, 3-hydroxy-2,2-dimethylpropanoic acid, 4-methylcyclohexaneacetic acid, tetrahydro-2*H*-pyran-3-carboxylic acid, methoxyacetic acid, 3-methoxypropionic acid; 4-(2,4-dimethylphenyl)-4-hydroxybutanoic acid, 4-(4-fluorophenyl)-4-hydroxybutanoic acid, 3-(trifluoromethyl)butyric acid, 4,4,4-trifluorobutyric acid, 7-phenylheptanoic acid, and 3-(4-cyanophenyl)propanoic acid.

Compounds of formula (VIII) in which L represents hydroxyl may also be prepared by methods well-known to those skilled in the art, for example, by hydrolysis of a corresponding ester. The reaction may typically be carried out using an appropriate acid e.g. sodium hydroxide in a suitable solvent such as methanol or ethanol. Examples of commercially available corresponding esters include ethyl 3-ethoxypropionate, ethyl 4-ethoxybutyrate and methyl 4-(methyloxy)butanoate.

Compounds of formula (IX) are commercially available, and include 2-(2-bromoethyl)-1*H-*isoindole-1,3(2*H*)-dione, *N*-(bromomethyl)phthalimide, *N*-(3-bromopropyl)phthalimide, *N-*(4-bromobutyl)phthalimide, *N*-(5-bromopentyl)phthalimide and *N*-(6-bromohexyl)phthalimide.

Compounds of formula (X) are commercially available, and include methyl iodide, iodoethane, 1-iodopropane, 1-iodobutane, 2-iodobutane, 1-iodopentane and 1-iodohexane.

Compounds of formula (XI) are commercially available, and include 2-(Boc-amino)ethyl bromide, 3-(Boc-amino)propyl bromide, 4-(Boc-amino)butyl bromide, 5-(Boc-amino)pentyl bromide and 6-(Boc-amino)hexyl bromide.

According to a third process C, a compound of formula (I), wherein R⁴ represents a group 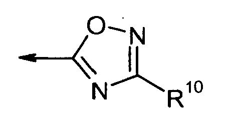 may be prepared by reacting a compound of formula (II): 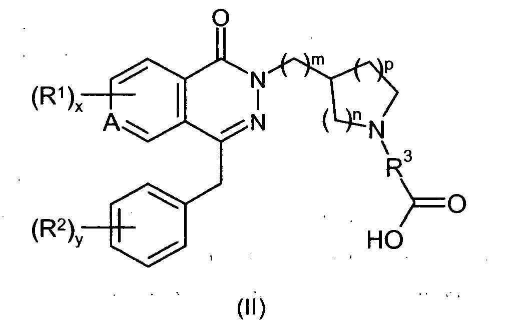 in which A, R¹, R², R³, n, m, p, x and y are as defined above for formula (I), with a compound of formula (XX): 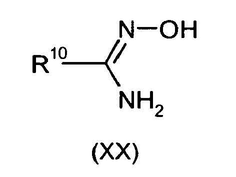 in which R¹⁰ is as defined above for formula (I).

The reaction may typically be carried out in a suitable solvent such as DMF by first reacting a compound of formula (II) with a suitable activating agent such as TBTU or oxalyl chloride optionally at an elevated temperature, followed by reaction with a compound of formula (XX) in the presence of an appropriate base such as triethylamine or diisoproplethylamine at an elevated temperature for example at about 80 to 100 °C for example using a microwave oven.

Compounds of formula (XX) are commercially available for example, from Apollo, Alfa Aesar, Maybridge, Tuner Scientific and/or Fulcrum Scientific and include *N*-hydroxy-3-(methyloxy)propanimidamide, *N*-hydroxy-2-methylpropanimidamide, *N-*hydroxypropanimidamide, *N*-hydroxy-2-(methoxyloxy)ethanimidamide, *N-*hydroxybutanimidamide.

Alternatively compounds of formula (XX) may be prepared according to Scheme 4 below: 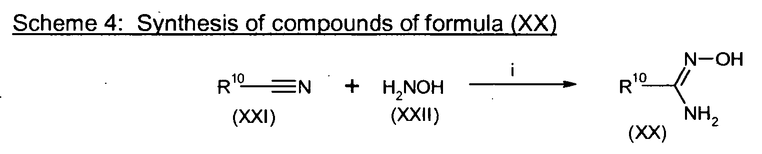 wherein R¹⁰ is as defined hereinabove.

Reagents and Conditions: i) suitable solvent such as ethanol, toluene or THF, optionally with the addition of an appropriate base such as aqueous sodium carbonate, triethylamine, or sodium methoxide when the compound (XXII) is in the form of a salt.

Compounds of formula (XXI) are commercially available, for example, from Aldrich and/or Apollo and/or Alfa Aesar, and/or ABCR and/or Betapharma and include, but are not limited to, acetonitrile, hexanenitrile, 3-hydroxypropionitrile, trifluoroacetonitrile, 3-methoxypropionitrile, 3-(2,2,2-trifluoroethoxy)propionitrile benzonitrile, 2-chlorobenzonitrile, 2-chloro-6-methylbenzonitrile, 4-pentylbenzonitrile, 4-(trifluoromethyl)benzonitrile, isophthalonitrile, 4-phenylbutyronitrile and 3-(3-chlorophenyl)-propionitrile.

The compound of formula (XXII) (hydroxylamine, hydroxylamine hydrochloride and hydroxylamine hydrate) is commercially available, for example, from Aldrich.

According to a fourth process D, a compound of formula (I) in which R⁴ represents 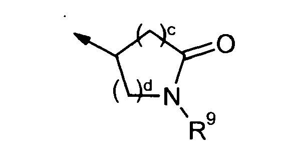 may be prepared by reacting a compound of formula (IV): 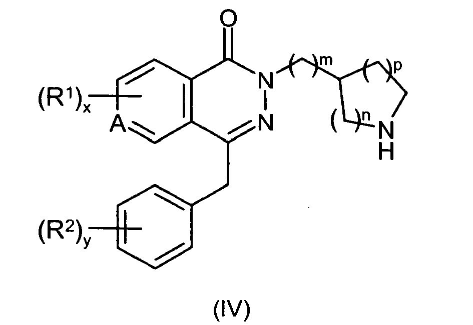 with a compound of formula (XXIII): 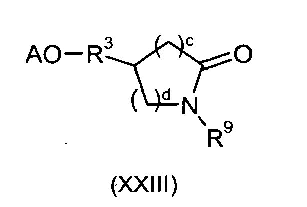 wherein AO represents an activated hydroxyl group such as mesylate or tosylate.

The reaction may typically be carried out in a suitable solvent, such as DMF, with an appropriate base, e.g. sodium bicarbonate (sodium hydrogen carbonate) and in the presence of a suitable activating agent such as sodium iodide. The reaction is usually heated, for example using a microwave oven at an appropriate elevated temperature, for example at about 140 to 160 °C for approximately 10 to 40 min, as appropriate.

Compounds of formula (IV) may be prepared according to Scheme 1 and Scheme 2, above.

Compounds of formula (XXIII) may be prepared according to Scheme 5 below. 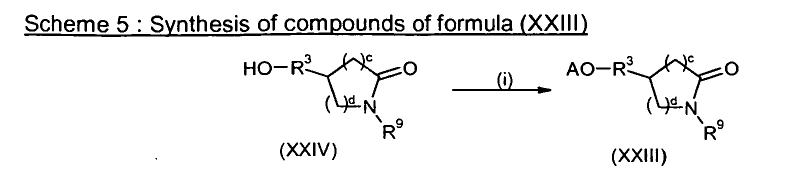 wherein c, d, R³ and R⁹ are as defined hereinabove, and AO represents an activated hydroxyl group such as mesylate or tosylate.

**Reagents and Conditions:** i) suitable solvent such as DCM, appropriate base e.g. triethylamine, activating agent for example methanesulfonyl chloride or tosylsulfonyl chloride (both commercially available, for example, from Aldrich).

Compounds of formula (XXIV) are commercially available and/or may be prepared according to methods well-known to those skilled in the art. Examples of compounds which are commercially available include (*R*)-(-)-5-(hydroxymethyl)-2-pyrrolidinone and (*S*)-(+)-5-(hydroxymethyl)-2-pyrrolidinone are available, for example, from Aldrich. 5-(2-hydroxyethyl)-2-pyrrolidinone may be prepared according to methods disclosed in European Patent EP 537606 B1, see Example 2. 4-(hydroxymethyl)-2-pyrrolidinone may be prepared according to methods disclosed in German Patent Application DE 2557335 A1, see Example 1. (S)-4-(2-hydroxyethyl)-2-pyrrolidinone may be prepared according to methods disclosed by Hanessian, S., et al., J. Org. Chem., 58(19):5032-5034, (1993), see Chart 1. 3-(2-hydroxyethyl)-2-pyrrolidinone may be prepared according to methods disclosed by Otto, A., et al., Tetrahedron Asymmetry, 10(17):3381-3389, (1999), see compound 7a. 6-(hydroxymethyl)-piperidin-2-one may be prepared according to methods disclosed by Synthetic Comm., 26(4):687-696, (1996). 6-(2-hydroxyethyl)-2-piperidinone may be prepared according to methods disclosed by Mohammad, T., et al., J. Label Compds. and Radiopharmaceuticals, 28(9):1087-1092, (1990). 5-(hydroxymethyl)-2-piperidinone may be prepared according to methods disclosed by Lerchner, A., et al., Chemistry - A European Journal, 12(32):8208-8219, (2006), see compound 30. 3-(hydroxymethyl)-2-piperidinone may be prepared according to methods disclosed by Smith, R. D., et al., J. Med. Chem., 24:104, (1981), see compound 2a. 7-hydroxymethyl-azepan-2-one may be prepared according to methods disclosed in International Patent Application WO 2006/103255 A1, see Compound F1. Hexahydro-7-(2-hydroxyethyl)-2H-azepin-2-one may be prepared according to methods disclosed in Can. J. Chem., 49(10):1648-1658, (1971). Hexahydro-3-(2-hydroxyethyl)-2*H*-azepin-2-one may be prepared according to methods disclosed by Cummings, W. A. W. et al., J. Chem. Soc., 4591 - 4604, (1964), see compound (VIII).

According to a fifth process, E, a compound of formula (I) may be prepared by reacting a compound of formula (IV) 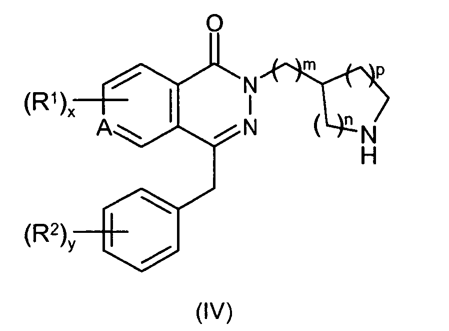 in which A, R¹, R², n, m, p, x and y are as defined above for formula (I), with a compound of formula (XXIV)

R⁴-R-³L (XXV)

in which R³ and R⁴ are as defined about for formula (I) and L is a suitable leaving group (for example, chloro, bromo or iodo) or a sulfonate (for example mesylate, tosylate or triflate).

The reaction may be carried out in a suitable solvent, for example methyl ethyl ketone, in the presence of a suitable base, for example potassium carbonate, triethylamine, or *N,N'-*diisopropylethylamine, optionally at an elevated temperature, for example at about 80 °C.

Compounds of formula (XXV) may be prepared by methods well known to those skilled in the art and/or by methods described herein, and/or are commercially available. Examples of compounds of formula (XXV) which are commercially available, for example from Apollo, Aldrich, include 2,2,2-trifluoro-*N*-(2-iodoethyl)acetamide, *N*1-(3-chloropropyl)-3-chloro-2,2-dimethylpropanamide, *N*-(2-chloroethyl)benzamide, *N*1-(2-chloroethyl)-4-methylbenzamide, *N*-(bromoacetyl)-2'-chloroacetaniline, *N*1-[2-chloro-4-(trifluoromethyl) phenyl]-2-bromoacetamide, *N*1-(2,6-dimethylphenyl)-3-bromopropanamide and *N*1-(2-chloro-6-fluorobenzyl)-3-bromopropanamide. Alternatively, compounds of formula (XXV) in which L represents a sulfonate may be prepared by activation of a corresponding hydroxyl compound by methods well known to those skilled in the art, such as by reaction with mesyl chloride or tosyl chloride. Examples of corresponding hydroxyl compounds include 4-(trifluoroacetamido)-1-butanol, 5-(trifluoroacetamido)-1-pentanol, 6-(trifluoroacetamido)-1-hexanol, which are commercially available, for example, from Aldrich.

According to a sixth process, F, a compound of formula (I) in which R³ is ethylene and R⁴ represents -C(O)N(R⁵)R⁶ may be prepared by reacting a compound of formula (IV) 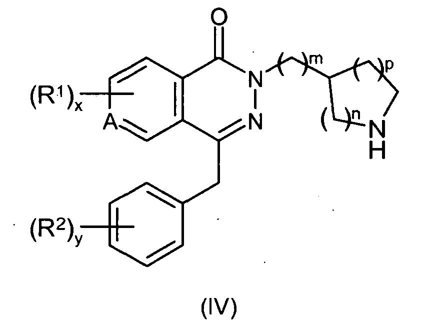 in which A, R¹, R², n, m, p, x and y are as defined above for formula (I), with a compound of formula (XXVI) 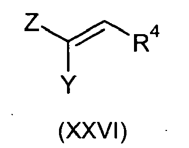 in which R⁴ represents -C(O)N(R⁵)R⁶ and Y and Z each independently represent C₁₋₃alkyl.

The reaction may be carried out in a suitable solvent, such as DMF or THF at an elevated temperature, for example about 70 to 90 °C.

Compounds of formula (XXVI) may be prepared by methods well known to those skilled in the art and/or by methods described herein, and/or are commercially available. Examples of compounds of formula (XXVI) which are commercially available, for example, from Apollo, Aldrich, and/or ABCR chemicals, include crotonamide, 3-methyl-but-2-enoicacid-(4-chloro-phenyl)-amide, 3-methyl-but-2-enoicacid-(5-chloro-2-methyl-phenyl)-amide, 3-methyl-but-2-enoicacid-(2-trifluoromethyl-phenyl)-amide, *N*-benzylacrylamide, *N-*(hydroxymethyl)acrylamide, *N*-*tert*-butylacrylamide, *N*-isopropylacrylamide, *N-*(isobutoxymethyl)acrylamide, *N*-methyl acrylamide, and *N*-(*n*-butoxymethyl)acrylamide.

According to a seventh process, G, a compound of formula (I), may be prepared by interconversion from other compounds of formula (I).

Interconversions include, but are not limited to alkylation and deprotection, under standard conditions well known to those skilled in the art.

Thus, typically, an alkylation reaction may be carried out between a compound of formula (I) and a C₁₋₆alkyl, activated to substitution by means of a leaving group such as halogen or an activated hydroxyl group, such as mesylate or tosylate. The reaction usually takes place in the presence of a suitable base such as triethylamine, *N,N*-diisopropylethylamine or sodium carbonate, in an appropriate solvent such as 2-butanone or DMF, optionally at an appropriate elevated temperature such as at about 80 °C.

According to an eighth process, H, a salt of a compound of formula (I) may be prepared by exchange of counterions, or precipitation of said salt from the free base.

Examples of protecting groups that may be employed in the synthetic routes descrbied and the means for their removal can be found in T. W. Greene 'Protective Groups in Organic Synthesis' (3rd edition, J. Wiley and Sons, 1999). Suitable amine protecting groups include sulphonyl (e.g. tosyl), acyl (e.g. acetyl, 2',2',2'-trichloroethoxycarbonyl, benzyloxycarbonyl or *t*-butoxycarbonyl) and arylalkyl (e.g. benzyl), which may be removed using an acid such as hydrogen chloride in dioxane or trifluoroacetic acid in dichloromethane or reductively by hydrogenolysis of a benzyl group or reductive removal of a 2',2',2'-trichloroethoxycarbonyl group using zinc in acetic acid, as appropriate. Other suitable amine protecting groups include trifluoroacetyl (-COCF₃), which may be removed by base catalysed hydrolysis or a solid phase resin bound benzyl group, such as a Merrifield resin bound 2,6-dimethoxybenzyl group (Ellman linker), which may be removed by acid catalysed hydrolysis, for example with trifluoroacetic acid.

It will be appreciated that all novel intermediates used to prepare compounds of the invention form yet a further aspect of the present invention.

Compounds of formula (I) or a pharmaceutical acceptable salt thereof may be useful for the treatment of various inflammatory and/or allergic diseases. Compounds of formula (I) or a pharmaceutically acceptable salt thereof may also be useful for the prophylaxis of various inflammatory and/or allergic diseases.

Examples of diseases in which a compound of formula (I), or a pharmaceutically acceptable salt thereof, may have potentially beneficial anti-inflammatory and/or anti-allergic effects include diseases of the respiratory tract such as bronchitis (including chronic bronchitis), asthma (including allergen-induced asthmatic reactions), chronic obstructive pulmonary disease (COPD), sinusitis and allergic rhinitis (seasonal and perennial).

Furthermore, the compounds of the invention may be of use in the treatment of nephritis, skin diseases such as psoriasis, eczema, allergic dermatitis and hypersensitivity reactions. Also, the compounds of the invention may be useful in the treatment of insect bites and stings.

The compounds of the invention may also be of use in the treatment of nasal polyposis, conjunctivitis or pruritis.

A disease of particular interest is allergic rhinitis.

Other diseases in which histamine may have a pathophysiological role include non-allegic rhinitis, and also diseases of the gastrointestinal tract such as intestinal inflammatory diseases including inflammatory bowel disease (e.g.Crohn's disease or ulcerative colitis) and intestinal inflammatory; diseases secondary to radiation exposure or allergen exposure.

As mentioned above, compounds of formula (I) may be useful as therapeutic agents. There is thus provided, as a further aspect of the invention, a compound of formula (I) or a pharmaceutically acceptable salt thereof for use in therapy.

In another embodiment, there is provided a compound which is *N*-[2-((2*R*)2-{[4-[(4-chlorophenyl)methyl]-1-oxo-2(1*H*)-phthalazinyl]methyl}-1-pyrrolidinyl)ethyl]-4-(methyloxy)butanamide or a pharmaceutically acceptable salt thereof for use in therapy.

According to another aspect of the invention, there is provided the use of a compound of formula (I) or a pharmaceutically acceptable salt thereof for the manufacture of a medicament for the treatment and/or prophylaxis of any of the above diseases.

In another embodiment, there is provided a compound which is *N*-[2-((2*R*)-2-{[4-[(4-chlorophenyl)methyl]-1-oxo-2(1*H*)-phthalazinyl]methyl}-1-pyrrolidinyl)ethyl]-4-(methyloxy) butanamide or a pharmaceutically acceptable salt thereof for the manufacture of a medicament for the treatment and/or prophylaxis of any of the above diseases.

According to yet another aspect of the invention, there is provided a compound of formula (I) or a pharmaceutically acceptable salt thereof for use in the treatment and/or prophylaxis of any of the above diseases.

In another embodiment, there is provided a compound which is *N*-[2-((2*R*)-2-{[4-[(4-chlorophenyl)methyl]-1-oxo-2(1*H*)-phthalazinyl]methyl}-1-pyrrolidinyl)ethyl]-4-(methyloxy) butanamide or a pharmaceutically acceptable salt thereof for use in the treatment and/or prophylaxis of any of the above diseases.

In a further aspect, there is provided a method for the treatment and/or prophylaxis of inflammatory and/or allergic diseases (such as any of the above diseases) which comprises administering to a patient in need thereof an effective amount of a compound of formula (I) or a pharmaceutically acceptable salt thereof.

In another embodiment, there is provided a method for the treatment and/or prophylaxis of inflammatory and/or allergic diseases (such as any of the above diseases) which method comprises administering to a patient in need thereof an effective amount of a compound which is *N*-[2-((2*R*)2-{[4-[(4-chlorophenyl)methyl]-1-oxo-2(1*H*)-phthalazinyl]methyl}-1-pyrrolidinyl)ethyl]-4-(methyloxy)butanamide or a pharmaceutically acceptable salt thereof.

When used in therapy, the compounds of formula (I) are usually formulated in a suitable pharmaceutical composition. Such pharmaceutical compositions can be prepared using standard procedures.

Thus, the present invention further provides a pharmaceutical composition which comprises a compound of formula (I) or a pharmaceutically acceptable salt thereof optionally with one or more pharmaceutically acceptable carriers and/or excipients.

In another embodiment, there is provided a composition which comprises a compound which is *N*-[2-((2*R*)2-{[4-[(4-chlorophenyl)methyl]-1-oxo-2(1*H*)-phthalazinyl]methyl}-1-pyrrolidinyl)ethyl]-4-(methyloxy)butanamide or a pharmaceutically acceptable salt thereof optionally with one or more pharmaceutically acceptable carriers and/or excipients.

A composition comprising a compound of formula (I) or a pharmaceutically acceptable salt thereof, which may be prepared by admixture, suitably at ambient temperature and atmospheric pressure, may be suitable for topical administration (which includes epicutaneous, inhaled, intranasal or ocular administration), enteral administration (which includes oral or rectal administration) or parenteral administration (such as by injection or infusion). Of interest are compositions comprising a compound of formula (I) or a pharmaceutically acceptable salt thereof, suitable for topical administration, particularly suitable for intranasal administration.

Generally, compositions may be in the form of solutions or suspensions (aqueous or non-aqueous), tablets, capsules, oral liquid preparations, powders, granules, lozenges, lotions, creams, ointments, gels, foams, reconstitutable powders or suppositories as required by the route of administration.

Generally, the compositions comprising a compound of formula (I) or a pharmaceutically acceptable salt thereof may contain from about 0.1 % to 99% (w/w), such as from about 10 to 60% (w/w) (based on the total weight of the composition), of the compound of formula (I) or the pharmaceutically acceptable salt thereof, depending on the route of administration. The dose of the compound used in the treatment of the aforementioned diseases will vary in the usual way with the seriousness of the diseases, the weight of the sufferer, and other similar factors. However, as a general guide, suitable unit doses may be about 0.05 to 1000 mg, for example about 0.05 to 200 mg, and such unit doses may be administered more than once a day, for example two or three times a day or as desired. Such therapy may extend for a number of weeks or months.

The proportion of the compound of formula (I) or a pharmaceutically acceptable salt thereof in a topical composition will depend on the precise type of composition to be prepared and the particular route of administration, but will generally be within the range of from about 0.001 to 10% (w/w), based on the total weight of the composition. Generally, however for most types of preparations the proportion used will be within the range of from about 0.005 to 1% (w/w), such as about 0.01 to 1% (w/w), for example about 0.01 to 0.5% (w/w), based on the total weight of the composition. However, in powders for inhalation the proportion used will generally be within the range of from about 0.1 to 5% (w/w), based on the total weight of the composition.

Generally, compositions suitable for intranasal or inhaled administration may conveniently be formulated as aerosols, solutions, suspensions, drops, gels or dry powders, optionally with one or more pharmaceutically acceptable carriers and/or excipients such as aqueous or non-aqueous vehicles, thickening agents, isotonicity adjusting agents, antioxidants and/or preservatives.

For compositions suitable for intranasal or inhaled administration, the compound of formula (I) or a pharmaceutically acceptable salt thereof may typically be in a particle-size-reduced form, which may be prepared by conventional techniques, for example, micronisation and milling. Generally, the size-reduced (e.g. micronised) compound of formula (I) or a pharmaceutically acceptable salt thereof can be defined by a D₅₀ value of about 0.5 to 10 microns, such as of about 2 to 4 microns (for example as measured using laser diffraction).

In one embodiment, compositions comprising a compound of formula (I) or a pharmaceutically acceptable salt thereof are suitable for intranasal administration. Intranasal compositions comprising a compound of formula (I) or a pharmaceutically acceptable salt thereof may permit the compound(s) to be delivered to all areas of the nasal cavities (the target tissue) and further, may permit the compound(s) to remain in contact with the target tissue for longer periods of time. A suitable dosing regime for intranasal compositions would be for the patient to inhale slowly through the nose subsequent to the nasal cavity being cleared. During inhalation the composition would be administered to one nostril (for example, as a spray or drops) while the other is manually compressed. This procedure would then be repeated for the other nostril. Typically, one or two sprays per nostril would be administered by the above procedure up to two or three times each day, ideally once daily. Of particular interest are intranasal compositions suitable for once daily administration.

Intranasal compositions may optionally contain one or more suspending agents, one or more preservatives, one or more wetting agents and/or one or more isotonicity adjusting agents as desired. Compositions suitable for intranasal administration may optionally further contain other excipients, such as antioxidants (for example sodium metabisulphite), taste-masking agents (such as menthol) and sweetening agents (for example dextrose, glycerol, saccharin and/or sorbitol).

The suspending agent, if included, will typically be present in the intranasal composition in an amount of between about 0.1 and 5% (w/w), such as between about 1.5% and 2.4% (w/w), based on the total weight of the composition. Examples of suspending agents include Avicel®, carboxymethylcellulose, veegum, tragacanth, bentonite, methylcellulose and polyethylene glycols, e.g. microcrystalline cellulose or carboxy methylcellulose sodium. Suspending agents may also be included in compositions suitable for inhaled, ocular and oral administration as appropriate.

For stability purposes, intranasal compositions comprising a compound of formula (I) or a pharmaceutically acceptable salt thereof may be protected from microbial or fungal contamination and growth by inclusion of a preservative. Examples of pharmaceutically acceptable anti-microbial agents or preservatives may include quaternary ammonium compounds (e.g. benzalkonium chloride, benzethonium chloride, cetrimide and cetylpyridinium chloride), mercurial agents (e.g. phenylmercuric nitrate, phenylmercuric acetate and thimerosal), alcoholic agents (e.g. chlorobutanol, phenylethyl alcohol and benzyl alcohol), antibacterial esters (e.g. esters of para-hydroxybenzoic acid), chelating agents such as disodium ethylenediaminetetraacetate (EDTA) and other anti-microbial agents such as chlorhexidine, chlorocresol, sorbic acid and its salts (such as potassium sorbate) and polymyxin. Examples of pharmaceutically acceptable anti-fungal agents or preservatives may include sodium benzoate. The preservative, if included, may be present in an amount of between about 0.001 and 1% (w/w), such as about 0.015% (w/w), based on the total weight of the composition. Preservatives may be included in compositions suitable for other routes of administration as appropriate.

Compositions which contain a suspended medicament may include a pharmaceutically acceptable wetting agent which functions to wet the particles of medicament to facilitate dispersion thereof in the aqueous phase of the composition. Typically, the amount of wetting agent used will not cause foaming of the dispersion during mixing. Examples of wetting agents include fatty alcohols, esters and ethers, such as polyoxyethylene (20) sorbitan monooleate (Polysorbate 80). The wetting agent may be present in intranasal compositions in an amount of between about 0.001 and 0.05% (w/w), for example about 0.025% (w/w), based on the total weight of the composition. Wetting agents may be included in compositions suitable for other routes of administration, e.g. for inhaled and/or ocular administration, as appropriate.

An isotonicity adjusting agent may be included to achieve isotonicity with body fluids e.g. fluids of the nasal cavity, resulting in reduced levels of irritancy. Examples of isotonicity adjusting agents include sodium chloride, dextrose, xylitol and calcium chloride. An isotonicity adjusting agent may be included in intranasal compositions in an amount of between about 0.1 and 10% (w/w), such as about 5.0% (w/w), based on the total weight of the composition. Isotonicity adjusting agents may also be included in compositions suitable for other routes of administration, for example in compositions suitable for inhaled, ocular, oral liquid and parenteral administration, as appropriate.

Further, the intranasal compositions comprising a compound of formula (I) or a pharmaceutically acceptable salt thereof may be buffered by the addition of suitable buffering agents such as sodium citrate, citric acid, phosphates such as disodium phosphate (for example the dodecahydrate, heptahydrate, dihydrate and anhydrous forms) or sodium phosphate and mixtures thereof. Buffering agents may also be included in compositions suitable for other routes of administration as appropriate.

Compositions for administration topically to the nose or lung for example, for the treatment of rhinitis, include pressurised aerosol compositions and aqueous compositions delivered to the nasal cavities by pressurised pump. Compositions which are non-pressurised and adapted to be administered topically to the nasal cavity are of particular interest. Suitable compositions contain water as the diluent or carrier for this purpose. Aqueous compositions for administration to the lung or nose may be provided with conventional excipients such as buffering agents, tonicity modifying agents and the like. Aqueous compositions may also be administered to the nose by nebulisation.

A fluid dispenser may typically be used to deliver a fluid composition to the nasal cavities. The fluid composition may be aqueous or non-aqueous, but typically aqueous. Such a fluid dispenser may have a dispensing nozzle or dispensing orifice through which a metered dose of the fluid composition is dispensed upon the application of a user-applied force to a pump mechanism of the fluid dispenser. Such fluid dispensers are generally provided with a reservoir of multiple metered doses of the fluid composition, the doses being dispensable upon sequential pump actuations. The dispensing nozzle or orifice may be configured for insertion into the nostrils of the user for spray dispensing of the fluid composition into the nasal cavity. A fluid dispenser of the aforementioned type is described and illustrated in WO05/044354 the entire content of which is hereby incorporated herein by reference. The dispenser has a housing which houses a fluid discharge device having a compression pump mounted on a container for containing a fluid composition. The housing has at least one finger-operable side lever which is movable inwardly with respect to the housing to cam the container upwardly in the housing to cause the pump to compress and pump a metered dose of the composition out of a pump stem through a nasal nozzle of the housing. In one embodiment, the fluid dispenser is of the general type illustrated in Figures 30-40 of WO05/044354.

In one embodiment, there is provided an intranasal composition comprising a compound of formula (I) or a pharmaceutically acceptable salt thereof.

In another embodiment, there is provided an intranasal composition comprising a compound which is *N*-[2-((2*R*)2-{[4-[(4-chlorophenyl)methyl]-1-oxo-2(1*H*)-phthalazinyl]methyl}-1-pyrrolidinyl)ethyl]-4-(methyloxy)butanamide or a pharmaceutically acceptable salt thereof: In another embodiment, such an intranasal composition is benzalkonium chloride-free.

Inhaled administration involves topical administration to the lung, such as by aerosol or dry powder composition.

Aerosol compositions suitable for inhaled administration may comprise a solution or fine suspension of the compound in a pharmaceutically acceptable aqueous or non-aqueous solvent. Aerosol compositions suitable for inhalation can be either a suspension or a solution and generally contain a compound of formula (I) or a pharmaceutically acceptable salt thereof and a suitable propellant such as a fluorocarbon or hydrogen-containing chlorofluorocarbon or mixtures thereof, such as hydrofluoroalkanes, e.g. 1,1,1,2-tetrafluoroethane, 1,1,1,2,3,3,3-heptafluoro-n-propane or a mixture thereof. The aerosol composition may optionally contain additional excipients well known in the art such as surfactants or cosolvents. Examples of surfactants include, but are not limited to oleic acid, lecithin, an oligolactic acid or derivative e.g. as described in WO94/21229 and WO98/34596. An example of a cosolvent includes, but is not limited to ethanol. Aerosol compositions may be presented in single or multidose quantities in sterile form in a sealed container, which may take the form of a cartridge or refill for use with an atomising device or inhaler. Alternatively, the sealed container may be a unitary dispensing device such as a single dose nasal inhaler or an aerosol dispenser fitted with a metering valve (metered dose inhaler), which is intended for disposal once the contents of the container have been exhausted.

Dry powder inhalable compositions may take the form of capsules and cartridges of, for example, gelatine, or blisters of, for example, laminated aluminium foil, for use in an inhaler or insufflator. Such compositions may be formulated comprising a powder mix of a compound of formula (I) or a pharmaceutically acceptable salt thereof and a suitable powder base such as lactose or starch.

Optionally, for dry powder inhalable compositions, a composition suitable for inhaled administration may be incorporated into a plurality of sealed dose containers (e.g. comprising the dry powder composition) mounted longitudinally in a strip or ribbon inside a suitable inhalation device. The container is rupturable or peel-openable on demand and the dose of e.g. the dry powder composition may be administered by inhalation via the device such as the DISKUS^{™} device, marketed by GlaxoSmithKline. The DISKUS ^{™} inhalation device is for example described in GB 2242134 A, and in such a device, at least one container for the composition in powder form (the container or containers may, for example, be a plurality of sealed dose containers mounted longitudinally in a strip or ribbon) is defined between two members peelably secured to one another; the device comprises: a means of defining an opening station for the said container or containers; a means for peeling the members apart at the opening station to open the container; and an outlet, communicating with the opened container, through which a user can inhale the composition in powder form from the opened container.

Aerosol compositions are typically arranged so that each metered dose or "puff' of aerosol contains about 20 µg - 2000 µg, particularly about 20 µg - 500 µg of a compound of formula (I) or a pharmaceutically acceptable salt thereof. Administration may be once daily or several times daily, for example 2, 3, 4 or 8 times, giving for example 1, 2 or 3 doses each time. The overall daily dose with an aerosol will be within the range of about 100 µg - 10 mg, such as between about 200 µg - 2000 µg. The overall daily dose and the metered dose delivered by capsules and cartridges in an inhaler or insufflator will generally be double those with aerosol compositions.

In another embodiment, there is provided a composition comprising a compound of formula (I) or a pharmaceutically acceptable salt thereof which is suitable for epicutaneous administration. An epicutaneous composition to be applied to the affected area e.g. the skin, by one or more application per day, may be in the form of, for example, an ointment, a cream, an emulsion, a lotion, a foam, a spray, an aqueous gel, or a microemulsion. Such compositions may optionally contain one or more solubilising agents, skin-penetration-enhancing agents, surfactants, fragrances, preservatives or emulsifying agents.

Ointments, creams and gels, may, for example, be formulated with an aqueous or oily base with the addition of suitable thickening and/or gelling agent and/or solvents. Such bases may thus, for example, include water and/or an oil such as liquid paraffin or a vegetable oil such as arachis oil or castor oil, or a solvent such as polyethylene glycol. Thickening agents and gelling agents which may be used according to the nature of the base include soft paraffin, aluminium stearate, cetostearyl alcohol, polyethylene glycols, woolfat, beeswax, carboxypolymethylene and cellulose derivatives, and/or glyceryl monostearate and/or non-ionic emulsifying agents. Lotions may be formulated with an aqueous or oily base and will in general also contain one or more emulsifying agents, stabilising agents, dispersing agents, suspending agents or thickening agents.

In another embodiment, there is provided a composition comprising a compound of formula (I) or a pharmaceutically acceptable salt thereof which is suitable for ocular administration. Such compositions may optionally contain one or more suspending agents, one or more preservatives, one or more wetting/lubricating agents and/or one or more isotonicity adjusting agents. Examples of ophthalmic wetting/lubricating agents may include cellulose derivatives; dextran 70, gelatin, liquid polyols, polyvinyl alcohol and povidone such as cellulose derivatives and polyols.

In another embodiment, there is provided a composition comprising a compound of formula (I) or a pharmaceutically acceptable salt thereof which is suitable for oral administration. Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colorants.

In another embodiment, there is provided a composition comprising a compound of formula (I) or a pharmaceutically acceptable salt thereof which is suitable for parenteral administration. Fluid unit dosage forms suitable for parenteral administration may be prepared utilising a compound of formula (I) or pharmaceutically acceptable salt thereof and a sterile vehicle which may be aqueous or oil based. The compound, depending on the vehicle and concentration used, may be either suspended or dissolved in the vehicle. In preparing solutions, the compound may be dissolved for injection and filter sterilised before filling into a suitable vial or ampoule and sealing. Optionally, adjuvants such as a local anaesthetic, preservatives and buffering agents may be dissolved in the vehicle. To enhance the stability, the composition may be frozen after filling into the vial and the water removed under vacuum. The lyophilised parenteral composition may be reconstituted with a suitable solvent just prior to administration. Parenteral suspensions may be prepared in substantially the same manner, except that the compound is suspended in the vehicle instead of being dissolved, and sterilisation cannot be accomplished by filtration. The compound may be sterilised by exposure to ethylene oxide before suspension in a sterile vehicle. A surfactant or wetting agent may be included in the composition to facilitate uniform distribution of the compound.

The compounds and pharmaceutical compositions according to the invention may also be used in combination with or include one or more other therapeutic agents, for example other antihistaminic agents for example H4 or H3 receptor antagonists, anticholinergic agents, anti-inflammatory agents such as corticosteroids (e.g. fluticasone propionate, fluticasone furoate, beclomethasone dipropionate, mometasone furoate, triamcinolone acetonide, budesonide and the steroid disclosed in WO02/12265); or non-steroidal anti-inflammatory drugs (NSAIDs) (e.g. sodium cromoglycate, nedocromil sodium), PDE-4 inhibitors, leukotriene antagonists, lipoxygenase inhibitors, chemokine antagonists (e.g. CCR3, CCR1, CCR2, CCR4, CCR8, CXCR1, CXCR2), IKK antagonists, iNOS inhibitors, tryptase and elastase inhibitors, beta-2 integrin antagonists and adenosine 2a agonists; or beta adrenergic agents (e.g. salmeterol, salbutamol, formoterol, fenoterol, terbutaline, and the beta agonists described in WO 02/66422, WO 02/270490, WO02/076933, WO03/024439 and WO03/072539 and salts thereof); or antiinfective agents e.g. antibiotic agents and antiviral agents.

It will be clear to a person skilled in the art that, where appropriate, the other therapeutic agent(s) may be used in the form of salts, (e.g. as alkali metal or amine salts or as acid addition salts), or prodrugs, or as esters (e.g. lower alkyl esters), or as solvates (e.g. hydrates) to optimise the activity and/or stability and/or physical characteristics (e.g. solubility) of the therapeutic agent. It will be clear also that where appropriate, the therapeutic agents may be used in optically pure form.

There is provided, in another embodiment, a combination comprising a compound of formula (I) or a pharmaceutically acceptable salt thereof together with one or more (such as one or two, e.g. one) other therapeutically active agents, optionally with one or more pharmaceutically acceptable carriers and/or excipients.

In another embodiment, there is provided a combination comprising a compound which is *N*-[2-((2*R*)-2-{[4-[(4-chlorophenyl)methyl]-1-oxo-2(1*H*)-phthalazinyl]methyl}-1-pyrrolidinyl)ethyl]-4-(methyloxy)butanamide or a pharmaceutically acceptable salt thereof, together with one or more (such as one or two, e.g. one) other therapeutically active agents (such as those described herein), optionally with one or more pharmaceutically acceptable carriers and/or excipients.

In another embodiment, there is provided a combination comprising a compound of formula (I) or a pharmaceutically acceptable salt thereof and an H3 and/or H4 antagonist.

Other histamine receptor antagonists which may be used alone, or in combination with an H1 receptor antagonist include antagonists (and/or inverse agonists) of the H4 receptor, for example, the compounds disclosed in Jablonowski et al., J. Med. Chem. 46:3957-3960 (2003), and antagonists (and/or inverse agonists) of the H3 receptor, for example the compounds described in WO2004/035556, the compounds described in WO2006/125665 and the compounds described in WO2006/090142.

In another embodiment, there is provided a combination comprising a compound of formula (I) or a pharmaceutically acceptable salt thereof and a β₂-adrenoreceptor agonist.

Examples of β₂-adrenoreceptor agonists include salmeterol (which may be a racemate or a single enantiomer, such as the *R*-enantiomer), salbutamol (which may be a racemate or a single enantiomer such as the *R*-enantiomer), formoterol (which may be a racemate or a single diastereomer such as the *R,R*-diastereomer), salmefamol, fenoterol, carmoterol, etanterol, naminterol, clenbuterol, pirbuterol, flerbuterol, reproterol, bambuterol, indacaterol, terbutaline and salts thereof, for example the xinafoate (1-hydroxy-2-naphthalenecarboxylate) salt of salmeterol, the sulfate salt or free base of salbutamol or the fumarate salt of formoterol. In one embodiment, combinations containing a compound of formula (I) may include longer-acting β₂-adrenoreceptor agonists, for example, compounds which provide effective bronchodilation for about 12 h or longer.

Other β₂-adrenoreceptor agonists include those described in WO 02/066422, WO 02/070490, WO 02/076933, WO 03/024439, WO 03/072539, WO 03/091204, WO 04/016578, WO 2004/022547, WO 2004/037807, WO 2004/037773, WO 2004/037768, WO 2004/039762, WO 2004/039766, WO01/4219 and WO03/042160.

Examples of β₂-adrenoreceptor agonists include:
3-(4-{[6-({(2*R*)-2-hydroxy-2-[4-hydroxy-3-(hydroxymethyl)phenyl]ethyl}amino)hexyl]oxy} butyl)benzenesulfonamide;
3-(3-{[7-{[(2*R*)2-hydroxy-2-[4-hydroxy-3-hydroxymethyl) phenyl] ethyl}-amino)heptyl]oxy} propyl)benzenesulfonamide;
4-{(1*R*)-2-[(6-{2-[(2,6-dichlorobenzyl)oxy]ethoxy}hexyl)amino]-1-hydroxyethyl}-2-(hydroxyl methyl) phenol;
4-{(1*R*)-2-[(6-{4-[3-(cyclopentylsulfonyl)phenyl]butoxy}hexyl)amino]-1-hydroxyethyl}-2-(hydroxylmethyl)phenol;
N-[2-hydroxyl-5-[(1*R*)-1-hydroxy-2-[[2-4-[[(2*R*)-2-hydroxy-2-phenylethyl]amino]phenyl] ethyl]amino]ethyl]phenyl]formamide;
N-2{2-[4-(3-phenyl-4-methoxyphenyl)aminophenyl]ethyl}-2-hydroxy-2-(8-hydroxy-2(1*H*)-quinolinon-5-yl)ethylamine; and
5-[(*R*)-2-(2-{4-[4-(2-amino-2-methyl-propoxy)-phenylamino]-phenyl}ethylamino)-1-hydroxy -ethyl]-8-hydroxy-1H-quinolin-2-one.

The β₂-adrenoreceptor agonist may be in the form of a salt formed with a pharmaceutically acceptable acid selected from sulfuric, hydrochloric, fumaric, hydroxynaphthoic (for example 1- or 3-hydroxy-2-naphthoic), cinnamic, substituted cinnamic, triphenylacetic, sulfamic, sulfanilic, naphthaleneacrylic, benzoic, 4-methoxybenzoic, 2- or 4-hydroxybenzoic, 4-chlorobenzoic and 4-phenylbenzoic acid.

In another embodiment, there is provided a combination comprising a compound of formula (I) or a pharmaceutically acceptable salt thereof and an anti-inflammatory agent.

Anti-inflammatory agents include corticosteroids. Suitable corticosteroids which may be used in combination with the compounds of formula (I) are those oral and inhaled corticosteroids and their pro-drugs which have anti-inflammatory activity. Examples include methyl prednisolone, prednisolone, dexamethasone, fluticasone propionate, 6α,9α-difluoro-11β-hydroxy-16α-methyl-17α-[(4-methyl-1,3-thiazole-5-carbonyl)oxy]-3-oxo-androsta-1,4-diene-17β-carbothioic acid *S*-fluoromethyl ester, 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester (fluticasone furoate), 6α,9α-difluoro-11β-hydroxy-16α-methyl-3-oxo-17α-propionyloxy- androsta-1,4-diene-17β-carbothioic acid *S*-(2-oxo-tetrahydro-furan-3S-yl) ester, 6α,9α-difluoro-11β-hydroxy-16α-methyl-3-oxo-17α-(2,2,3,3- tetramethycyclo propylcarbonyl)oxy-androsta-1,4-diene-17β-carbothioic acid *S*-cyanomethyl ester and 6α,9α-difluoro-11β-hydroxy-16α-methyl-17α-(1-methycylopropylcarbonyl)oxy-3-oxo-androsta-1,4-diene-17β-carbothioic acid *S*-fluoromethyl ester, beclomethasone esters (for example the 17-propionate ester or the 17,21-dipropionate ester), budesonide, flunisolide, mometasone esters (for example mometasone furoate), triamcinolone acetonide, rofleponide, ciclesonide (16α,17-[[(*R*)-cyclohexylmethylene]bis(oxy)]-11β,21-dihydroxy-pregna-1,4-diene-3,20-dione), butixocort propionate, RPR-106541, and ST-126. Corticosteroids of particular interest may include fluticasone propionate, 6α,9α-difluoro-11 β-hydroxy-16α-methyl-17α-[(4-methyl-1,3-thiazole-5-carbonyl)oxy]-3-oxo-androsta-1,4-diene-17β-carbothioic acid *S*-fluoromethyl ester, 6α,9α-difluoro-17α-[(2-furanylcarbonyl)oxy]-11β-hydroxy-16a-methyl-3-oxo-androsta-1,4-diene-17β-carbothioic acid *S*-fluoromethyl ester, 6α,9α-difluoro-11β-hydroxy-16α-methyl-3-oxo-17α-(2,2,3,3-tetramethycyclopropylcarbonyl)oxy-androsta-1,4-diene-17β-carbothioic acid *S*-cyano methylester, 6α,9α-difluoro-11β-hydroxy-16α-methyl-17α-(1-methycyclopropylcarbonyl) oxy-3-oxo-androsta-1,4-diene-17β-carbothioic acid *S*-fluoromethyl ester and mometasone furoate. In one embodiment the corticosteroid is 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester (fluticasone furoate) or mometasone furoate.

There is provided, in a further embodiment, a combination comprising a compound of formula (I) or a pharmaceutically acceptable salt thereof and a corticosteroid, such as fluticasone propionate or 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester (fluticasone furoate) or mometasone furoate, in particular 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester (fluticasone furoate). Such combinations may be of particular interest for intranasal administration.

In another embodiment there is provided a combination comprising a compound which is *N*-[2-((2*R*)-2-{[4-[(4-chlorophenyl)methyl]-1-oxo-2(1*H*)-phthalazinyl]methyl}-1-pyrrolidinyl)ethyl]-4-(methyloxy)butanamide or a pharmaceutically acceptable salt thereof and 6α,9α-difluoro-17α-[(2-furanylcarbonyl)oxy]-11β-hydroxy-16α-methyl-3-oxo-androsta-1,4-diene-17β-carbothioic acid S-fluoromethyl ester (fluticasone furoate).

In another embodiment, there is provided a combination comprising a compound of formula (I) or a pharmaceutically acceptable salt thereof and a glucocorticoid agonist.

Non-steroidal compounds having glucocorticoid agonism that may possess selectivity for transrepression over transactivation and that may be useful in combination therapy include those covered in the following patent application and patents: WO03/082827, WO98/54159, WO04/005229, WO04/009017, WO04/018429, WO03/104195, WO03/082787, WO03/082280, WO03/059899, WO03/101932, WO02/02565, WO01/16128, WO0O/6659 WO03/086294, WO04/026248, WO03/061651, WO03/08277, WO06/000401, WO06/000398 and WO06/015870.

Anti-inflammatory agents include non-steroidal anti-inflammatory drugs (NSAID's).

NSAID's include sodium cromoglycate, nedocromil sodium, phosphodiesterase (PDE) inhibitors (e.g. theophylline, PDE4 inhibitors or mixed PDE3/PDE4 inhibitors), leukotriene antagonists, inhibitors of leukotriene synthesis (eg. montelukast), iNOS (inducible nitric oxide synthase) inhibitors (e.g. oral iNOS inhibitors), IKK antagonists, tryptase and elastase inhibitors, beta-2 integrin antagonists and adenosine receptor agonists or antagonists (e.g. adenosine 2a agonists), cytokine antagonists (e.g. chemokine antagonists, such as a CCR1, CCR2, CCR3, CCR4, or CCR8 antagonists) or inhibitors of cytokine synthesis, or 5-lipoxygenase inhibitors. iNOS inhibitors include those disclosed in WO93/13055, WO98/30537, WO02/50021, WO95/34534 and WO99/62875.

In another embodiment there is provided the use of the compounds of formula (I) or a pharmaceutically acceptable salt thereof in combination with a phosphodiesterase 4 (PDE4) inhibitor. The PDE4-specific inhibitor useful in this embodiment may be any compound that is known to inhibit the PDE4 enzyme or which is discovered to act as a PDE4 inhibitor, and which are only PDE4 inhibitors, not compounds which inhibit other members of the PDE family, such as PDE3 and PDE5, as well as PDE4.

Compounds which may be of interest include 6-({3-[(dimethylamino)carbonyl]phenyl}sulfonyl)-8-methyl-4-{[3-(methyloxy)phenyl]amino}-3-quinolinecarboxamide (Example 399 of International Patent Application WO04/103998), *cis*-4-cyano-4-(3-cyclopentyloxy-4-methoxyphenyl)cyclohexan-1-carboxylic acid, 2-carbomethoxy-4-cyano-4-(3-cyclopropylmethoxy-4-difluoromethoxyphenyl)cyclohexan-1-one and *cis*-[4-cyano-4-(3-cyclopropylmethoxy-4-difluoromethoxyphenyl)cyclohexan-1-ol]. Also, *cis*-4-cyano-4-[3-(cyclopentyloxy)-4-methoxyphenyl]cyclohexane-1-carboxylic acid (also known as cilomilast) and its salts, esters, pro-drugs or physical forms, which is described in U.S. patent 5,552,438 issued 03 September, 1996.

Other PDE4 inhibitors include AWD-12-281 from Elbion (Hofgen, N. et al., 15th EFMC Int. Symp. Med. Chem., (Sept 6-10, Edinburgh) 1998, Abst. P. 98; CAS reference No. 247584020-9); a 9-benzyladenine derivative nominated NCS-613 (INSERM); D-4418 from Chiroscience and Schering-Plough; a benzodiazepine PDE4 inhibitor identified as CI-1018 (PD-168787) and attributed to Pfizer; a benzodioxole derivative disclosed by Kyowa Hakko in WO99/16766; K-34 from Kyowa Hakko; V-11294A from Napp (Landells, L.J. et al., Eur. Resp. J. [Ann. Cong. Eur. Resp. Soc. (Sept 19-23, Geneva) 1998] 1998, 12 (Suppl. 28): Abst P2393); roflumilast (CAS reference No 162401-32-3) and a pthalazinone (WO99/47505 from Byk-Gulden; Pumafentrine, (-)-p-[(4aR*,10*b*S*)-9-ethoxy-1,2,3,4,4a,10b-hexahydro-8-methoxy-2-methylbenzo[c][1,6]naphthyridin-6-yl]-*N,N-*diisopropylbenzamide which is a mixed PDE3/PDE4 inhibitor which has been prepared and published on by Byk-Gulden, now Altana; arofylline under development by Almirall-Prodesfarma; VM554/UM565 from Vernalis; or T-440 (Tanabe Seiyaku; Fuji, K. et al., J. Pharmacol. Exp. Ther., 284(1):162, (1998)), and T2585.

Further compounds which may be of interest are disclosed in the published international patent applications WO04/024728 (Glaxo Group Ltd), WO04/056823 (Glaxo Group Ltd) and WO04/103998 (Glaxo Group Ltd).

In another embodiment, there is provided a combination comprising a compound which is *N*-[2-((2*R*)-2-{[4-[(4-chlorophenyl)methyl]-1-oxo-2(1*H*)-phthalazinyl]methyl}-1-pyrrolidinyl) ethyl]-4-(methyloxy)butanamide or a pharmaceutically acceptable salt thereof and 6-({3-[(dmethylamino)carbonyl]phenyl}sulfonyl)-8-methyl-4-{[3-(methyloxy)phenyl]amino}-3-quinolinecarboxamide or a pharmaceutically acceptable salt thereof.

In another embodiment, there is provided a combination comprising a compound of formula (I) or a pharmaceutically acceptable salt thereof and an anticholinergic agent.

Anticholinergic agents are those compounds that act as antagonists at the muscarinic receptors, in particular those compounds which are antagonists of the M₁ or M₃ receptors, dual antagonists of the M₁/M₃ or M₂/M₃, receptors or pan-antagonists of the M₁/M₂/M₃ receptors. Exemplary compounds for administration *via* inhalation include ipratropium (for example, as the bromide, CAS 22254-24-6, sold under the name Atrovent), oxitropium (for example, as the bromide, CAS 30286-75-0) and tiotropium (for example, as the bromide, CAS 136310-93-5, sold under the name Spiriva). Also of interest are revatropate (for example, as the hydrobromide, CAS 262586-79-8) and LAS-34273 which is disclosed in WO01/04118. Exemplary compounds for oral administration include pirenzepine (for example, CAS 28797-61-7), darifenacin (for example, CAS 133099-04-4, or CAS 133099-07-7 for the hydrobromide sold under the name Enablex), oxybutynin (for example, CAS 5633-20-5, sold under the name Ditropan), terodiline (for example, CAS 15793-40-5), tolterodine (for example, CAS 124937-51-5, or CAS 124937-52-6 for the tartrate, sold under the name Detrol), otilonium (for example, as the bromide, CAS 26095-59-0, sold under the name Spasmomen), trospium chloride (for example, CAS 10405-02-4) and solifenacin (for example, CAS 242478-37-1, or CAS 242478-38-2, or the succinate also known as YM-905 and sold under the name Vesicare).

Other anticholinergics may be found in WO 2004/012684; WO2004/091482; WO2005/009439; WO2005/009362; WO2005/009440; W02005/037280; WO2005/037224; WO2005/046586; WO2005/055940; WO2005/055941; WO2005/067537; WO2005/087236; WO2005/086873; WO2005/094835; WO2005/094834; WO2005/094251; WO2005/095407; WO2005/099706; WO2005/104745; WO2005/112644; WO2005/118594; WO2006/005057; WO2006/017768; WO2006/017767; WO2006/050239; WO2006/055553; WO2006/055503; WO2006/065755; WO2006/065788; WO2007/018514; WO2007/018508; WO2007/016650; WO2007/016639; and WO2007/022351 .

Specific compounds include:
(3-*endo*)3-(2,2-di-2-thienylethenyl)-8,8-dimethyl-8-azoniabicyclo[3.2.1]octane bromide;
(3-*endo*)-3-(2,2-diphenylethenyl)8,8-dimethyl-8-azoniabicyclo[3.2.1]octane bromide;
(3-*endo*)3-(2,2-diphenylethenyl)-8,8-dimethyl-8-azoniabicyclo[3.2.1]octane 4-methylbenzenesulfonate;
(3-*endo*)-8,8-dimethyl-3-[2-phenyl-2-(2-thienyl)ethenyl]-8-azoniabicyclo[3.2.1]octane bromide; and/or
(3-*endo*)-8,8-dimethyl-3-[2-phenyl-2-(2-pyridinyl)ethenyl]-8-azoniabicyclo[3.2.1]octane bromide;
(Endo)-3-(2-methoxy-2,2-di-thiophen-2-yl-ethyl)-8,8-dimethyl-8-azonia-bicyclo[3.2.1]octane iodide;
3-((Endo)-8-methyl-8-aza-bicyclo[3.2.1]oct-3-yl)-2,2-diphenyl-propionitrile;
(Endo)-8-methyl-3-(2,2,2-triphenyl-ethyl)-8-aza-bicyclo[3.2.1]octane;
3-((Endo)-8-methyl-8-aza-bicyclo[3.2.1]oct-3-yl)-2,2-diphenyl-propionamide;
3-((Endo)-8-methyl-8-aza-bicyclo[3.2.1]oct-3-yl)-2,2-diphenyl-propionic acid;
(Endo)-3-(2-cyano-2,2-diphenyl-ethyl)-8,8-dimethyl-8-azonia-bicyclo[3.2.1]octane iodide;
(Endo)-3-(2-cyano-2,2-diphenyl-ethyl)-8,8-dimethyl-8-azonia-bicyclo[3.2.1]octane bromide;
3-((Endo)-8-methyl-8-aza-bicyclo[3.2.1]oct-3-yl)-2,2-diphenyl-propan-1-ol;
*N*-Benzyl-3-((endo)-8-methyl-8-aza-bicyclo[3.2.1]oct-3-yl)-2,2-diphenyl-propionamide;
(Endo)-3-(2-carbamoyl-2,2-diphenyl-ethyl)-8,8-dimethyl-8-azonia-bicyclo[3.2.1]octane iodide;
1-Benzyl-3-[3-((endo)-8-methyl-8-aza-bicyclo[3.2.1]oct-3-yl)-2,2-diphenyl-propyl]-urea;
1-Ethyl-3-[3-((endo)-8-methyl-8-aza-bicyclo[3.2.1]oct-3-yl)-2,2-diphenyl-propyl]-urea;
*N*-[3-((Endo)-8-methyl-8-aza-bicyclo[3.2.1]oct-3-yl)-2,2-diphenyl-propyl]-acetamide;
*N*-[3-((Endo)-8-methyl-8-aza-bicycla[3.2.1]oct-3-yl)-2,2-diphenyl-propyl]-benzamide;
3-((Endo)-8-methyl-8-aza-bicyclo[3.2.1]oct-3-yl)-2,2-di-thiophen-2-yl-propionitrile;
(Endo)-3-(2-cyano-2,2-di-thiophen-2-yl-ethyl)-8,8-dimethyl-8-azonia-bicyclo[3.2.1]octane iodide;
*N*-[3-((Endo)-8-methyl-8-aza-bicyclo[3.2.1]oct-3-yl)-2,2-diphenyl-propyl]-benzenesulfonamide;
[3-((Endo)-8-methyl-8-aza-bicyclo[3.2.1]oct-3-yl)-2,2-diphenyl-propyl]-urea;
*N*-[3-((Endo)-8-methyl-8-aza-bicyclo[3.2.1]oct-3-yl)-2,2-diphenyl-propyl]-methanesulfonamide; and/or
(Endo)-3-{2,2-diphenyl-3-[(1-phenyl-methanoyl)-amino]-propyl}-8,8-dimethyl-8-azonia-bicyclo[3.2.1]octane bromide.
(Endo)-3-(2-methoxy-2,2-di-thiophen-2-yl-ethyl)-8,8-dimethyl-8-azonia-bicyclo[3.2.1]octane iodide;
(Endo)-3-(2-cyano-2,2-diphenyl-ethyl)-8,8-dimethyl-8-azonia-bicyclo[3.2.1]octane iodide;
(Endo)-3-(2-cyano-2,2-diphenyl-ethyl)-8,8-dimethyl-8-azonia-bicyclo[3.2.1]octane bromide;
(Endo)-3-(2-carbamoyl-2,2-diphenyl-ethyl)-8,8-dimethyl-8-azonia-bicyclo[3.2.1]octane iodide;
(Endo)-3-(2-cyano-2,2-di-thiophen-2-yl-ethyl)-8,8-dimethyl-8-azonia-bicyclo[3.2.1]octane iodide; and/or
(Endo)-3-{2,2-diphenyl-3-[(1-phenyl-methanoyl)-amino]-propyl}-8,8-dimethyl-8-azonia-bicyclo[3.2.1]octane bromide.

The combinations referred to above may conveniently be presented for use in the form of a pharmaceutical composition and thus pharmaceutical compositions comprising a combination as defined above optionally together with a pharmaceutically acceptable carrier and/or excipient.

The individual compounds of such combinations may be administered either sequentially in separate pharmaceutical compositions as well as simultaneously in combined pharmaceutical compositions. Additional therapeutically active ingredients may be suspended in the composition together with a compound of formula (I). Appropriate doses of known therapeutic agents will be readily appreciated by those skilled in the art.

Compounds of the invention may be prepared by the methods described below or by similar methods. Thus the following Intermediates and Examples illustrate the preparation of the compounds of the invention, and are not to be considered as limiting the scope of the invention in any way.

### GENERAL EXPERIMENTAL

### Abbreviations

| | |
|---|---|
| BOC (Boc): | *tert*-butoxycarbonyl |
| CV: | Column volumes |
| DCM: | Dichloromethane |
| DIPEA: | *N,N*-Diisopropylethylamine |
| DMF: | *N*,*N*-Dimethylformamide |
| DMSO: | Dimethylsulfoxide |
| EtOAc: | Ethylacetate |
| EtOH: | Ethanol |
| HCl: | Hydrogen chloride |
| HPLC: | High Performance Liquid Chromatography |
| h: | Hours |
| LCMS: | Liquid Chromatography - Mass Spectroscopy |
| MDAP HPLC: | Mass-Directed Autopreparative HPLC |
| MeCN: | Acetonitrile |
| MeOH: | Methanol |
| MgSO₄: | Magnesium Sulfate |
| min: | Minutes |
| NaOH: | Sodium hydroxide |
| Na₂SO₄: | Sodium sulphate |
| NEt₃: | Triethylamine |
| NMR: | Nuclear Magnetic Resonance |
| Pd/C: | Palladium on activated carbon |
| PyBOP: | Benzotriazol-1-yl-oxy-trispyrrolidinophosphonium hexafluorophospate |
| RT: | Retention time |
| s.g | Specific gravity |
| TBTU: | *O*-(1*H*-benzotriazol-1-yl)-*N*,*N*,*N'*,*N'*-tetramethyluronium tetrafluoroborate |
| TFA: | Trifluoroacetic acid |
| THF: | Tetrahydrofuran |
| TLC: | Thin Layer Chromatography |

### General Procedures

Flash silica gel refers to Merck Art No. 9385; silica gel refers to Merck Art No. 7734. SCX cartridges are Ion Exchange SPE columns where the stationary phase is polymeric benzene sulfonic acid. These are used to isolate amines.

SCX2 cartridges are Ion Exchange SPE columns where the stationary phase is polymeric propylsulfonic acid. These are used to isolate amines.

LCMS was conducted on a Supelcosil LCABZ+PLUS column (3.3 cm × 4.6 mm ID) eluting with 0.1 % formic acid and 0.01 M ammonium acetate in water (solvent A) and 0.05% formic acid 5% water in MeCN (solvent B), using the following elution gradient 0.0 - 7 min 0% B, 0.7 - 4.2 min 100% B, 4.2 - 5.3 min 0% B, 5.3 - 5.5min 0% B at a flow rate of 3 mlmin⁻¹. The mass spectra were recorded on a Fisons VG Platform spectrometer using electrospray positive and negative mode (ES+ve and ES-ve).

The Flashmaster II is an automated multi-user flash chromatography system, available from Argonaut Technologies Ltd, which utilises disposable, normal phase, SPE cartridges (2 g to 100 g). It provides quaternary on-line solvent mixing to enable gradient methods to be run. Samples are queued using the multi-functional open access software, which manages solvents, flow-rates, gradient profile and collection conditions. The system is equipped with a Knauer variable wavelength UV-detector and two Gilson FC204 fraction-collectors enabling automated peak cutting, collection and tracking.

Mass directed autopreparative (MDAP) HPLC was conducted on a Waters FractionLynx system comprising of a Waters 600 pump with extended pump heads, Waters 2700 autosampler, Waters 996 diode array and Gilson 202 fraction collector on a 10 cm × 2.54 cm internal diameter ABZ+ column, eluting with 0.1 % formic acid in water (solvent A) and 0.1 % formic acid in MeCN (solvent B), using an appropriate elution gradient over 15 min at a flow rate of 20 mlmin⁻¹ and detecting at 200 - 320 nm at room temperature. Mass spectra were recorded on Micromass ZMD mass spectrometer using electro spray positive and negative mode, alternate scans. The software used was MassLynx 3.5 with OpenLynx and FractionLynx options.

The ¹H NMR spectra were recorded on a Bruker AV400 operating at 400 MHz. Standard deuterated solvents were used. Tetramethylsilane may have been used as internal standard.

Reactions are routinely monitored by methods well known to those skilled in the art, such as TLC, LCMS and/or HPLC. Such methods are used to assess whether a reaction has gone to completion, and reaction times may be varied accordingly.

The XRPD method which is employed to analyse crystalline forms of compounds is as follows:

| Manufacturer | PANalytical - The Netherlands |
|---|---|
| Instrument | X'Pert Pro |
| Diffractometer Type | DY1850 |
| Tube anode | Cu |
| K-Alpha1 wavelength (A°) | 1.54056 |
| K-Alpha2 wavelength (A°) | 1.54439 |
| Ration Alpha 1:2 | 0.50000 |
| Divergence slit | Prog.Div.Slit |
| Receiving slit | Prog.Rec.Slit |
| Generator voltage (kV) | 40 |
| Tube Current (mA) | 45 |
| Detector | X'celerator |
| Data Angle range (°2θ) | 2.000-39.997 |
| Scan type | Continuous |
| Scan step size | 0.0167 |
| Scan step time (seconds) | 31.75 |
| Sample preparation | Flush Silicon wafer |

XRPD analysis is performed on a PANalytical X'Pert Pro X-ray powder diffractometer, model X' Pert Pro PW3040/60, serial number DY1850 using an X'Celerator detector. The acquisition conditions are: radiation: Cu K, generator tension: 40 kV, generator current: 45 mA, start angle: 2.000°2θ, end angle: 39.997 °2θ, step size: 0.0167°2θ, time per step: 31.75 seconds. The sample is prepared using flush Silicon wafer. The margin of error is approximately ±1 °2θ for each of the peak assignments.

Differential Scanning Calorimetry (DSC) is performed on a TA instruments Q1000 Differential Scanning Calorimeter equipped with a refrigerated cooling system by weighing compound into an aluminium pan and crimping a pan lid onto the top of the pan. Slight variations in the observed peaks may be expected based on the specific instrument and pan configuration employed, the analyst's sample preparation technique, and the sample size. Some margin of error is present in the peak assignment reported above. The margin of error is approximately ±5 °C for the peak maximum and ±10 J/g for the heat of fusion.

Compounds were named using ACD/Name PRO 6.02 chemical naming software Advanced Chemistry Developments Inc.; Toronto, Ontario, M5H2L3, Canada.

### Intermediates

### Intermediate 1

### 1,1-Dimethylethyl(2R)-2-{[4-[(4-chlorophenyl)methyl]-1-oxo-2(1H)-phthalazinyl]methyl}-1-pyrrolidinecarboxylate

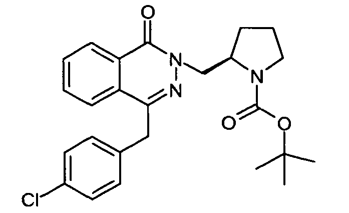

To a solution of triphenylphosphine (1.86 g, 7.09 mmol) in dry THF (6 ml) was added diisopropyl azodicarboxylate (1.12 ml, 5.69 mmol) at -15 °C. The resulting pale yellow thick suspension was stirred at -15 °C for 2 min. To aid stirring more dry THF (2 ml) was added. The reaction mixture was then treated with a suspension of 4-[(4-chlorophenyl)methyl]-1(2*H*)-phthalazinone, (as disclosed in US patent 3,813,384, Example 10, Step 1) (0.571 g, 2.11 mmol) and *N*-*tert*-butoxycarbonyl-D-prolinol (commercially available, for example, from Fluka), (0.650 g, 3.23 mmol) in dry THF (10 ml) at -15 °C. The reaction mixture was allowed to warm to room temperature and stirred at 20 °C for 23 h. MeOH (20 ml) was then added and the solvents were removed *in vacuo.* The resultant residue was purified by Flashmaster II chromatography (70 g silica cartridge) eluted with 0-50% EtOAc-cyclohexane gradient over 40 min. The solvents were removed *in vacuo* to afford the *title compound* (1.05 g). LCMS RT = 3.71 min.

### Intermediate 2

### 4-[(4-Chlorophenyl)methyl]-2-[(2R)-2-pyrrolidinylmethyl]-1(2H)-phthalazinone

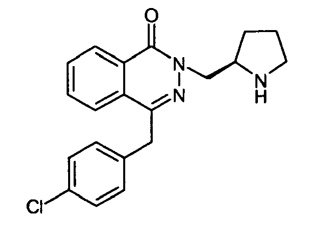

To a solution of 1,1-dimethylethyl (2*R*)-2-{[4-[(4-chlorophenyl)methyl]-1-oxo-2(1*H*)-phthalazinyl]methyl}-1-pyrrolidinecarboxylate (for example, as prepared for Intermediate 1) (1.05 g, 2.31 mmol) in dry dioxane (12 ml) was added a solution of HCl in 1,4-dioxane (4.0 M, 6 ml). The solution was stirred at 20 °C for 2 h. TFA (4 × about 1 ml) was added at 10 minute intervals until deprotection was completed. The solvent was removed *in vacuo* and the residue applied onto an SCX-2 cartridge (20 g, pre-conditioned with MeOH), washed with MEOH (× 2) and then eluted with 10% aqueous ammonia in MeOH (2 × 50 ml). The solvents were removed *in vacuo* and the resultant residue purified by Flashmaster II chromatography (50 g silica cartridge) eluted with 0-30% MeOH + 1% triethylamine - DCM gradient over 40 min to afford the *title compound* (0.351 g). LCMS RT = 2.45 min, ES+ve *m*/*z* 354/356 (M+H)⁺.

### Intermediate 3

### Methyl 3-((2R)-2-{[4-[(4-chlorophenyl)methyl]-1-oxo-2(1H)-phthalazinyl]methyl}-1-pyrrolidinyl)propanoate

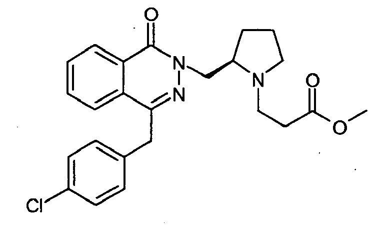

To a solution of 4-[(4-chlorophenyl)methyl]-2-[(2*R*)-2-pyrrolidinylmethyl]-1(2*H*)-phthalazinone (for example, as prepared for Intermediate 2) (2.40 g, 6.78 mmol) in tetrahydrofuran (10 ml) was added methyl acrylate (commercially available, for example, from Aldrich) (2.0 ml, 22 mmol). The solution was heated to 50 °C for 20 h. The solvent was removed *in vacuo* to leave an orange gum. The residue was purified by silica chromatography (flashmaster, 100 g cartridge) eluting with 0-15% MeOH in DCM containing 1% triethylamine over 40 min. Appropriate fractions were combined, and the solvent was removed *in vacuo* to give the *title compound* (2.59 g, 87%). LCMS RT = 2.51 min, ES+ve *m*/*z* 440/442 (M+H)⁺.

### Intermediate 4

### 3-((2R)-2-{[4-[(4-Chlorophenyl)methyl]-1-oxo-2(1H)-phthalazinyl]methyl}-1-pyrrolidinyl)propanoic acid, triethylamine salt (1:1)

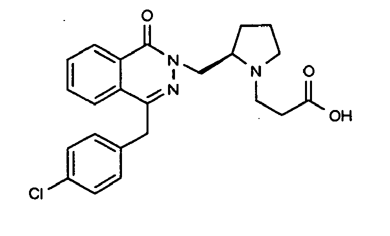

To a solution of methyl 3-((2*R*)2-{[4-[(4-chlorophenyl)methyl]-1-oxo-2(1*H*)-phthalazinyl]methyl}-1-pyrrolidinyl)propanoate (for example, as prepared for Intermediate 3) (2.50 g , 5.68 mmol) in MeOH (25 ml) was added 2 M aqueous sodium hydroxide (5 ml, 10 mmol). The solution was stirred at ambient temperature for 45 min. A further amount of 2 M aqueous sodium hydroxide (5 ml, 10 mmol) was added to the solution. The solution was stirred for a further 2.25 h. The solvent was removed *in vacuo* to leave a white solid. The residue was dissolved in water (25 ml) and carefully neutralised with 2 M aqueous hydrochloric acid (approximately 10 ml) until pH 6-5 was reached. The resulting oily suspension was applied to a SCX cartridge (70 g, pre-conditioned with MeOH and then water). The cartridge was washed with water (1.5 column volumes) and then MeOH (1.5 column volumes). The cartridge was eluted with 10% triethylamine in MeOH (1.5 column volumes). The combined basic fractions were concentrated *in vacuo* to give the *title compound* (2.393 g, 85%). LCMS RT = 2.68 min, ES+ve *m*/*z* 426/428 (M+H)⁺.

### Intermediate 5

### Methyl 3-((2R)-2-{(4-[(4-chlorophenyl)methyl]-1-oxo-2(1H)-phthalazinyl]methyl}-1-pyrrolidinyl)-2-methylpropanoate

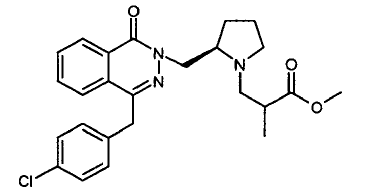

4-[(4-Chlorophenyl)methyl]-2-[(2*R*)-2-pyrrolidinylmethyl]-1(2*H*)-phthalazinone (for example, as prepared for Intermediate 2) (100 mg, 0.28 mmol) was stirred under reflux with methyl methacrylate (commercially available, for example, from Aldrich) (60 µl, 0.56 mmol) in tetrahydrofuran (10 ml) for three days. The solution was heated, evaporated to dryness and the residue was dissolved in DMF (1ml) and more methyl methacrylate (60 µl, 0.56 mmol) was added. The reaction was heated at 80 °C under nitrogen for a further 24 h. Further methyl methacrylate (180 µl, 1.66 mmol) was added and heating continued for 6 h at 100°C. More methyl methacrylate (4 × 0.4 ml) was added over two days and after a further 24 h, heating stopped and the reaction was diluted with MeOH. The mixture was poured onto.a 10 g SCX-2 cartridge that had been pre-conditioned with MeOH. The cartridge was washed through with MeOH and then the product was eluted with 10% 0.880 s.g. aqueous ammonia solution in MeOH to give the *title compound* (26 mg). LCMS RT = 2.65 min, ES+ve *m*/*z* 454/456 (M+H)⁺.

### Intermediate 6

### 3-((2R)-2-{[4-[(4-chlorophenyl)methyl]-1-oxo-2(1H)-phthalazinyl]methyl}-1-pyrrolidinyl)-2-methylpropanoic acid, formate salt (1:1)

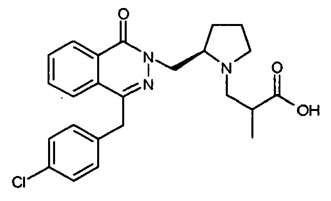

Methyl 3-((2*R*)-2-{[4-[(4-chlorophenyl)methyl]-1-oxo-2(1*H*)-phthalazinyl]methyl}-1-pyrrolidinyl)-2-methylpropanoate (for example, as prepared for Intermediate 5) (26 mg, 0.057 mmol) was dissolved in MeOH (1 ml) and 2 N aqueous sodium hydroxide (0.2 ml, 0.4 mmol) was added. After 24 hours, 2 M hydrochloric acid (0.2 ml, 0.4 mmol) was added and the mixture was evaporated to dryness. The residue was leached with MeOH, the MeOH solution was concentrated and the product was purified by MDAP HPLC to give the *title compound* (13.6 mg). LCMS RT = 2.60 min, ES+ve *m*/*z* 440/442 (M+H)⁺.

### Intermediate 7

### 1,1-Dimethylethyl {2-[(2,2,2-trifluoroethyl)oxy]ethyl}carbamate

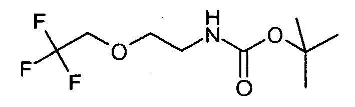

1,1'-(Azodicarbonyl)dipiperidine (commercially available, for example, from Fluka) (2.52 g, 10 mmol) was dissolved in toluene (50 ml) with stirring. 1,1,1-Trifluoroethanol (commercially available, for example, from Aldrich) (2.50 g, 25 mmol) and 1,1-dimethylethyl(2-hydroxyethyl)carbamate (commercially available, for example, from Aldrich) (773 µl, 5 mmol) were added, and the stirring was continued at room temperature under a nitrogen atmosphere. Tributyl phosphine (commercially available, for example, from Fluka) (2.5 ml, 10.0 mmol) was added dropwise, causing precipitation of solid material. Further toluene (20 ml) was added, but precipitate remained. The resulting slurry was stirred at room temperature under nitrogen overnight. The reaction mixture was treated with MeOH (10 ml), causing dissolution of the remaining solid. The mixture was concentrated *in vacuo* to give a white solid (approximately 6.4g).

A portion of the crude material (approximately 650 mg) was purified by chromatography on silica (20 g, eluting with EtOAc-cyclohexane 10%, 25%, 50%). Following MS analysis of the fractions, the appropriate one was concentrated *in vacuo* to afford the *title compound* (103 mg), ¹H NMR (400 MHz, CDCl₃) δ 4.88 (br s, 1H), 3.85 (q, *J*=9 Hz, 2H), 3.68 (t, *J*=5 Hz, 2H), 3.39-3.32 (m, 2H), 1.45 (s, 9H).

The remainder of the crude material was also purified by chromatography on silica (Flashmaster, 100 g, eluting with EtOAc-cyclohexane, 0-50% over 40 min). The appropriate fractions were concentrated *in vacuo* to afford the *title compound* (936 mg), identical by NMR to the previous sample, ES+ve *m*/*z* 244 (M+H)⁺.

### Intermediate 8

### {2-[(2,2,2-Trifluoroethyl)oxy]ethyl}amine, hydrochloride salt

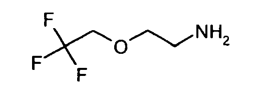

1,1-Dimethylethyl {2-[(2,2,2-trifluoroethyl)oxy]ethyl}carbamate, (for example, as prepared for Intermediate 7) (97 mg, 0.40 mmol) was dissolved in dioxane with stirring, and the solution was treated with HCl in dioxane (4M, 1 ml). After stirring for 1 h at room temperature, the reaction mixture was concentrated *in vacuo* to afford the *title compound* (75 mg, 0.42 mmol), LCMS RT = 0.35 min (detected by ELSD), ES+ve *m*/*z* 144 (M+H)⁺.

### Intermediate 9

### Ethyl 4-((2R)-2-{[4-[(4-chlorophenyl)methyl]-1-oxo-2(1H)-phthalazinyl]methyl}-1-pyrrolidinyl)butanoate

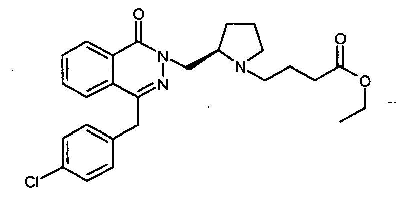

4-[(4-Chlorophenyl)methyl]-2-[(2*R*)-2-pyrrolidinylmethyl]-1(2*H*)-phthalazinone (107 mg, 0.3 mmol) (for example, as prepared for Intermediate 2) was stirred with ethyl 4-bromobutyrate (commercially available, for example form Aldrich)(86 µL, 0.6 mmol) and potassium carbonate (84 mg, 0.6 mmol) in DMF (1 ml) in a Smith Creator microwave at 150 °C for 15 min. The reaction was poured onto a SCX-2 cartridge (10 g) that had been pre-conditioned with MeOH. The cartridge was flushed through with MeOH (approximately 80 ml) and the product was then eluted with 10% 0.880 s.g. aqueous ammonia solution in MeOH to give the *title compound* (106 mg). LCMS RT = 2.77 min, ES+ve *m*/*z* 468/470 (M+H)⁺, plus approximately 25% methyl ester; LCMS RT = 2.65 min, ES+ve *m*/*z* 454/456 (M+H)⁺.

### Intermediate 10

### 4-((2R)-2-{[4-[(4-chlorophenyl)methyl]-1-oxo-2(1H)-phthalazinyl]methyl}-1-pyrrolidinyl)butanoic acid, formate salt (1:1)

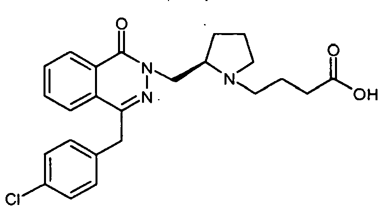

Ethyl 4-((2*R*)-2-{[4-[(4-chlorophenyl)methyl]-1-oxo-2(1*H*)-phthalazinyl]methyl}-1-pyrrolidinyl)butanoate (containing approximately 25% methyl ester) (for example, as prepared for Intermediate 9) (106 mg, approximately 0.23 mmol) was stirred with 2 M aqueous sodium hydroxide (0.5 ml, 1 mmol) in MeOH (2 ml) at room temperature for 65 min. 2 M aqueous hydrochloric acid (0.5 ml) was added and the mixture was evaporated to dryness. The residue was leached with 1:1 MeOH-DMSO (1 ml) and the solution was purified by MDAP HPLC to give the *title compound* (67 mg). LCMS RT = 2.55 min, ES+ve *m*/*z* 440/442 (M+H)⁺.

### Intermediate 11

### 1,1-Dimethylethyl ((2R)-2-{[4-[(4-chlorophenyl)methyl]-1-oxo-2(1H)-phthalazinyl]methyl}-1-pyrrolidinyl)acetate

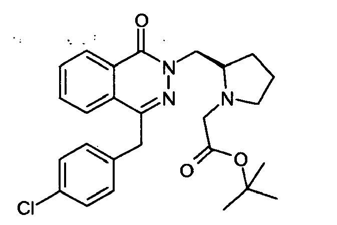

To a solution of 4-[(4-chlorophenyl)methyl]-2-[(2*R*)-2-pyrrolidinylmethyl]-1(2*H*)-phthalazinone (for example, as prepared for Intermediate 2) (0.527 g, 1.49 mmol) in dry dimethylformamide (5 ml) was added potassium carbonate (0.411 g, 2.97 mmol) followed by *tert*-butyl bromoacetate (commercially available, for example, from Aldrich) (0.330 ml, 2.23 mmol). The resultant reaction mixture was stirred at room temperature under nitrogen overnight. More *tert*-butyl bromoacetate (0.220 ml, 1.49 mmol) was added and the reaction was heated to 80 °C for 1 h. The reaction mixture was then filtered and applied onto a pre-conditioned (MeOH) SCX-2 cartridge (20 g), washed with MeOH (× 3) and then eluted with 10% aqueous ammonia in MeOH (x 4). The solvents were removed *in vacuo* to afford the *title compound* (0.547 g). LCMS RT = 2.87 min, ES+ve *m*/*z* 468/470 (M+H)⁺.

### Intermediate 12

### ((2R)-2-{[4-[(4-Chlorophenyl)methyl]-1-oxo-2(1H)-phthalazinyl]methyl}-1-pyrrolidinyl)acetic acid, trifluoroacetate salt

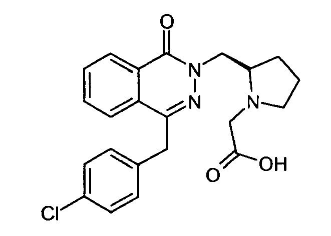

To a solution of 1,1-dimethylethyl ((2*R*)-2-{[4-[(4-chlorophenyl)methyl]-1-oxo-2(1*H*)-phthalazinyl]methyl}-1-pyrrolidinyl)acetate (for example, as prepared for Intermediate 11) (0.540 g, 1.15 mmol) in DCM (5 ml) was added TFA (4 ml). The resultant reaction mixture was stirred for 3 days and then concentrated *in vacuo* to afford the *title compound* (0.925 g). LCMS RT = 2.60 min ES+ve *m*/*z* 412/414 (M+H)⁺.

### Intermediate 13

### 1,1-Dimethylethyl 4-[4-[(4-chlorophenyl)methyl]-1-oxo-2(1H)-phthalazinyl]-1-piperidinecarboxylate

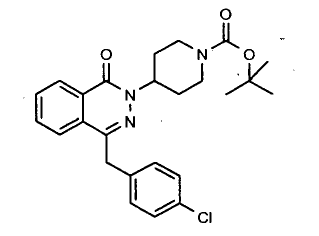

To a solution of triphenylphosphine (3.80 g, 14.5 mmol) (commercially available, for example, from Aldrich) in anhydrous tetrahydrofuran (12 ml) at -5 °C to -10 °C was added diisopropyl azodicarboxylate (2.4 ml, 11.58 mmol) (commercially available, for example, from Aldrich). The resulting suspension set solid and was left to stand for 10-15 min. To the suspension at -5 °C to 0 °C was added a suspension of 4-[(4-chlorophenyl)methyl]-1(2*H*)-phthalazinone (1.50 g, 5.54 mmol) (as described in US Patent 3,813,384, Example 10, Step 1) and 1,1-dimethylethyl 4-hydroxy-1-piperidinecarboxylate (commercially available, for example, from Aldrich)(1.50 g, 7.45 mmol) in anhydrous THF (30 ml). The resulting light suspension was stirred at 20 °C for 1.5 h and then quenched with MeOH (10 ml). The solvent was removed *in vacuo* to leave a brown oil (10.5 g) which was purified by silica chromatography (flashmaster, 100 g × 3 cartridges) eluting with 0-50 % ethyl acetate in cyclohexane over 40 min. The appropriate fractions were combined, and the solvent was removed *in vacuo* to give the *title compound* (4.3 g). LCMS RT = 3.80 min, ES+ve *m*/*z* 454/456 (M+H)⁺.

### Intermediate 14

### 4-[(4-Chlorophenyl)methyl]-2-(4-piperidinyl)-1(2H)-phthalazinone

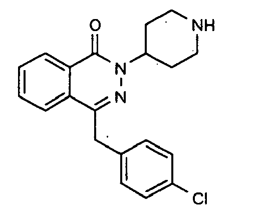

To a solution of crude 1,1-dimethylethyl 4-[4-[(4-chlorophenyl)methyl]-1-oxo-2(1*H)-*phthalazinyl]-1-piperidinecarboxylate (for example, as prepared for Intermediate 13) (4.30 g) in anhydrous 1,4-dioxane (20 ml) was added 4.0 M hydrochloric acid in 1,4-dioxane (20 ml) (commercially available, for example, from Aldrich). The solution was stirred at 20 °C for 4 h. The solvent was removed *in vacuo* and residue applied to a SCX cartridge (50 g, pre-conditioned with MeOH). The cartridge was washed with MeOH (2 column volumes) and then eluted with 10% 0.880 s.g. ammonia in MeOH (2 column volumes). The basic fractions were combined and the solvent removed *in vacuo* to leave a purple oil (2.80 g). The residue was purified by silica chromatography (flashmaster, 70 g cartridge) eluting with 0-30% MeOH in DCM containing 1% triethylamine over 30 min, appropriate fractions were combined, and the solvent was removed *in vacuo* to give the *title compound* (2.59 g, 96 % over two steps). LCMS RT = 2.52 min, ES+ve *m*/*z* 354/356 (M+H)⁺.

### Intermediate 15

### Methyl 3-{4-[(4E,5E)-3-[(4-chlorophenyl)methyl]-4,5-diethylidene-6-oxo-5,6-dihydro-1(4H)-pyridazinyl]-1-piperidinyl}propanoate

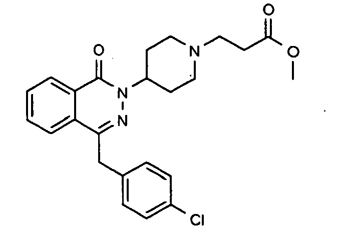

Synthesised using 4-[(4-chlorophenyl)methyl]-2-(4-piperidinyl)-1(2*H*)-phthalazinone (for example, as prepared for Intermediate 14) by a method generally similar to that described herein in Intermediate 3. LCMS RT = 3.66 min, ES+ve *m*/*z* 440/442 (M+H)⁺.

### Intermediate 16

### 3-{4-[4-[(4-Chlorophenyl)methyl]-1-oxo-2(1H)-phthalazinyl]-1-piperidinyl}propanoic acid, triethylamine salt (1:1)

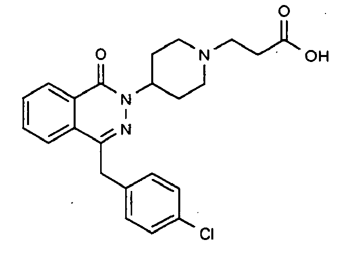

Synthesised using methyl 3-{4-[(4*E*,5*E*)-3-[(4-chlorophenyl)methyl]-4,5-diethylidene-6-oxo-5,6-dihydro-1(4*H*)-pyridazinyl]-1-piperidinyl}propanoate (for example, as prepared for Intermediate 15) by a method generally similar to that described herein in Intermediate 4. LCMS RT = 2.53min, ES+ve *m*/*z* 426/428 (M+H)⁺.

### Intermediate 17

### 4-(Methyloxy)butanoic acid

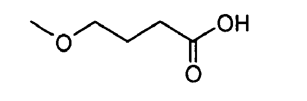

Methyl 4-(methyloxy)butanoate (commercially available, for example, from Aldrich) (8.2 ml, 60 mmol) was dissolved in MeOH (60 ml) and treated with 2 M aqueous sodium hydroxide solution (60 ml), and the resulting mixture was stirred at room temperature overnight. The mixture was concentrated *in vacuo* to remove the MeOH. The aqueous mixture was partitioned between DCM (100 ml) and water (40 ml). The layers were separated and the aqueous washed with further DCM (100 ml). The aqueous layer was acidified to pH 1-2 using 5 M HCl (24 ml), and extracted with DCM (2 × 100 ml). These latter organic extracts were combined and concentrated *in vacuo* to give the product as a colourless mobile oil (4.12 g, 34.8 mmol, 58%), ¹H NMR (400 MHz, CDCl₃) δ 11.0, (br s, 1H), 3.44 (t, *J* = 6 Hz, 2H), 3.34 (s, 3H), 2.46 (t, *J* = 7.5 Hz, 2H), 1.95-1.86 (m, 2H).

### Intermediate 18

### 2-[2-((2R)-2-([4-[(4-Chlorophenyl)methyl]-1-oxo-2(1H)-phthalazinyl]methyl)-1-pyrrolidinyl)ethyl]-1H-isoindole-1,3(2H)-dione

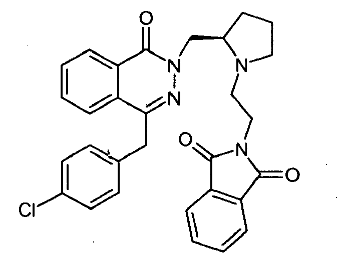

4-[(4-Chlorophenyl)methyl]-2-[(2*R*)-2-pyrrolidinylmethyl]-1(2*H*)-phthalazinone (for example, as prepared for Intermediate 2) (3.05 g, 8.59 mmol) was stirred with 2-(2-bromoethyl)-1*H*-isoindole-1,3(2*H*)-dione (4.82 g, 19 mmol) (commercially available, for example, from Acros) and potassium carbonate (5.9 g, 43 mmol) in 2-butanone (75 ml) under nitrogen at 80 °C for 18 h. Further 2-(2-bromoethyl)-1*H*-isoindole-1,3(2*H*)-dione (2.4 g, 9.4 mmol) and potassium carbonate (3.0 g, 22 mmol) were added and the heating and stirring were continued for a further day. After three further days at room temperature, the mixture was partitioned between water and DCM. The aqueous layer was extracted with more DCM and the combined organic layers were washed with water, dried (MgSO₄) and evaporated to a thick oil. This oil was redissolved in DCM and loaded onto a column of silica gel (250 g) that had been pre-conditioned with 40% ethyl acetate in 40-60 petroleum ether. The column was eluted with this mixture, then 50%, 70%, 80% and 100% ethyl acetate. Appropriate fractions were combined and concentrated to give the *title compound* (3.96 g, 7.51 mmol). LCMS RT = 3.19 min, ES+ve *m*/*z* 527/529 (M+H)⁺.

### Intermediate 19

### 2-{[(2R)-1-(2-Aminoethyl)-2-pyrrolidinyl]methyl}-4-[(4-chlorophenyl)methyl]-1(2H)-phthalazinone

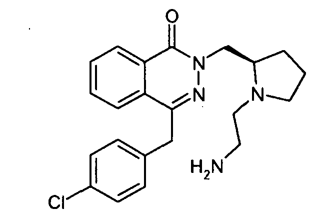

2-[2-((2*R*)-2-{[4-[(4-Chlorophenyl)methyl]-1-oxo-2(1*H*)-phthalazinyl]methyl}-1-pyrrolidinyl)ethyl]-1*H*-isoindole-1,3(2*H*)-dione (for example, as prepared for Intermediate 18) (3.96 g, 7.51 mmol) was dissolved in ethanol (50 ml) at 80 °C with stirring and hydrazine monohydrate (0.91 ml, 19 mmol) (commercially available, for example, from Aldrich) was added. The mixture was heated with stirring for 1.25 h. The reaction was cooled with ice-water and the white solid was removed by filtration. The filter-cake was leached with ethanol and the combined filtrates were evaporated to a white solid. This solid was mixed with 2M hydrochloric acid (10 ml) and water (approximately 100 ml). The opaque solution was washed successively with ethyl acetate and DCM. The aqueous layer was then made alkaline with 2M sodium hydroxide solution and the product was extracted with DCM (× 4). The combined organic layers were washed with water and dried (MgSO₄) and evaporated to give the *title compound* as a white solid, (2.29 g) LCMS RT = 2.74 min, ES+ve *m*/*z* 397/399 (M+H)⁺.

### Intermediate 20

### 1,1-Dimethylethyl [3-((2R)-2-{[4-[(4-chlorophenyl)methyl]-1-oxo-2(1H)-phthalazinyl]methyl}-1-pyrrolidinyl)propyl]carbamate

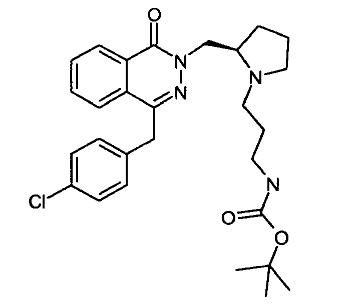

4-[(4-Chlorophenyl)methyl]-2-[(2*R*)-2-pyrrolidinylmethyl]-1(2*H*)-phthalazinone (for example, as prepared for Intermediate 2) (495 mg, 1.40 mmol) was stirred with 1,1-dimethylethyl (3-bromopropyl)carbamate (404 mg, 1.70 mmol) (commercially available, for example, from Fluka), potassium carbonate (293 mg, 2.12 mmol) and sodium iodide (41 mg, 0.27 mmol) in 2-butanone (20 ml) under nitrogen at 80 °C overnight. The solvent was removed *in vacuo,* and the residue was dissolved in a mixture of DCM and water. The layers were separated, and the DCM solution was passed through a hydrophobic frit, and then concentrated *in vacuo* to give the *title compound* (737 mg, approximately 100%). LCMS RT = 2.74 min, ES+ve *m*/*z* 511/513 (M+H)⁺.

### Intermediate 21

### 2-{[(2R)-1-(3-Aminopropyl)-2-pyrrolidinyl]methyl}-4-[(4-chlorophenyl)methyl]-1(2H)-phthalazinone

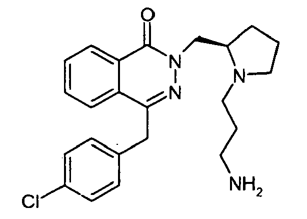

1,1-Dimethylethyl [3-((2*R*)-2-{[4-[(4-chlorophenyl)methyl]-1-oxo-2(1*H*)-phthalazinyl]methyl}-1-pyrrolidinyl)propyl]carbamate (737 mg, 1.4 mmol) (for example, as prepared for Intermediate 20) was dissolved in a solution of HCl in dioxane (2.4 M, 25 ml), and the mixture was stirred at room temperature under nitrogen overnight. The reaction mixture was concentrated *in vacuo,* and the residue was dissolved in acetonitrile and applied to a SCX cartridge (20 g, pre-conditioned with MeOH and then acetonitrile). The cartridge was washed with acetonitrile and then eluted with 10% 0.880 s.g. ammonia in acetonitrile. The appropriate basic fractions were combined and the solvent removed *in vacuo,* to give the *title compound* (508 mg, 88%). LCMS RT = 2.11 min, ES+ve *m*/*z* 411/413 (M+H)⁺.

### Intermediate 22

### 4-[(4-chlorophenyl)methyl]-2-({(2R)-1-[2-(methylamino)ethyl]-2-pyrrolidinyl}methyl)-1(2H)-phthalazinone

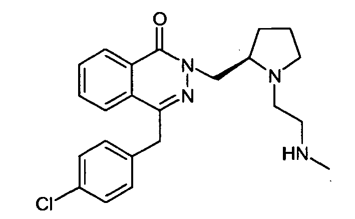

*N*-[2-((2R)-2-{[4-[(4-Chlorophenyl)methyl]-1-oxo-2(*H*)-phthalaziny)]methyl}-1-pyrrolidinyl)ethyl]-2,2,2-trifluoro-*N*-methylacetamide, (for example, as prepared for Example 48) (53 mg, max 0.10 mmol) was dissolved in MeOH (2 ml). A solution of potassium carbonate (48 mg, 0.34 mmol) in water (1 ml) was added and the reaction was stirred at room temperature for 7 h. The reaction was concentrated under a stream of nitrogen, and the residue was applied to an SCX-2 cartridge (10 g). The cartridge was washed with MeOH and then eluted with 10% aqueous 0.880 s.g. ammonia in MeOH. The appropriate basic fractions were combined and the solvent removed *in vacuo*. Purification by MDAP HPLC gave *the title compound* as the formate salt. This was applied to an SCX-2 cartridge (5 g), washing with MeOH and eluting with 10% aqueous 0.880 s.g. ammonia in MeOH. The basic fractions were combined and concentrated to give the *title compound* as a brown gum (12 mg, 29% over two steps); LCMS RT = 2.81 min, ES+ve *m*/*z* 425/427 (M+H)⁺.

### Intermediate 23

### 4-[(Methyloxy)carbonyl]-3-pyridinecarboxylic acid

To a suspension of pyridine-3,4-dicarboxylic acid anhydride (commercially available, for example, from Aldrich) (26.73 g, 180 mmol) in dry THF (250 ml) at -70 °C under nitrogen was added a suspension of sodium methoxide (11.2 g, 2.01 mol) in dry MeOH (50 ml). The reaction mixture was allowed to warm to room temperature and stirred for 18 h. The solvents were removed *in vacuo* and the residue was dissolved in water (350 ml). This was acidified to approximately pH 2 using concentrated hydrochloric acid. The resultant solid was collected by filtration and washed with water. The solid was dried *in vacuo* at 45 °C to give *the title compound* (14.6 g, 45%) as a white solid. LCMS RT = 0.98 min, ES+ve *m*/*z* 182 (M+H)⁺.

### Intermediate 24

### Methyl 3-{2-(4-chlorophenyl)-3-[(1,1-dimethylethyl)oxy]-3-oxopropanoyl}-4-pyridine carboxylate

To a solution of 4-[(methyloxy)carbonyl]-3-pyridinecarboxylic acid (for example, as prepared for Intermediate 23) (1.81 g, 10 mmol) in dry DMF (90 ml) under nitrogen was added carbonyl diimidazole (1.7 g, 10.5 mmol). The reaction mixture was heated at 50 °C for 90 min and then cooled to -5 °C in a salt/ice bath. To this was added 1,1-dimethylethyl 4-chlorophenylacetate (for example, as prepared for Intermediate 28) (2.38 g, 10.5 mmol), followed by the portionwise addition of sodium hydride (1.4 g of 60% dispersion in mineral oil, 35 mmol) over 15 min. The reaction mixture was stirred at -5 °C for 10 min and then warmed to room temperature. After 2 h, the reaction mixture was poured into a saturated solution of ammonium chloride (100 ml). This was extracted using EtOAc (3 × 100 ml). The combined organics were washed with water (2 × 100 ml) and brine (2 × 100 ml). The organic phase was dried (MgSO₄) and the solvent removed *in vacuo*. The residue was dissolved in DCM (5 ml and applied to a silica cartridge (100 g). This was eluted using a gradient of 0-50% EtOAc in cyclohexane over 60 min. The required fractions were evaporated *in vacuo* to give the *title compound* (2.94 g, 75%, mixture of ketone and enol purity 99%) as a pale brown oil. LCMS RT = 3.41 and 3.63 (U-shaped peak) min ES+ve *m*/*z* 390/392 (M+H)⁺.

### Intermediate 25

### Methyl 3-[(4-chlorophenyl)acetyl]-4-pyridinecarboxylate

Methyl 3-{2-(4-chlorophenyl)-3-[(1,1-dimethylethyl)oxy]-3-oxopropanoyl}-4-pyridine carboxylate (for example, as prepared for Intermediate 24) (2.94 g, 7.5 mmol) was dissolved in dry DCM (12 ml) and to this was added TFA (5 ml). The reaction mixture was stirred at room temperature under nitrogen for 20 h. The solvent was removed *in vacuo* and the residue was dissolved in DCM (5 ml). This was applied to a silica cartridge (100 g) and eluted with a gradient of 0-100% EtOAc in cyclohexane over 60 min. The required fractions were combined and evaporated *in vacuo* to give *the title compound* (1.59 g, 73%) as a pale orange oil. LCMS RT = 3.02 min ES+ve *m*/*z* 290/292 (M+H)⁺.

### Intermediate 26

### 4-[(4-Chlorophenyl)methyl]pyrido[3,4-d]pyridazin-1(2H)-one

Methyl 3-[(4-chlorophenyl)acetyl]-4-pyridinecarboxylate (for example, as prepared for Intermediate 25) (1.59 g, 5.5 mmol) was dissolved in EtOH (60 ml) and to this was added hydrazine hydrate (commercially available, for example, from Aldrich) (0.3 ml, 6 mmol) and a few drops of acetic acid. The reaction mixture was heated at reflux for 3 h. The reaction mixture was allowed to cool and the solid was collected by filtration and washed with EtOH (10 ml). The solid was dried *in vacuo* to give *the title compound* (1.17 g, 78%) as a white solid. LCMS RT = 2.73 min, ES+ve *m*/*z* 272/274 (M+H)⁺.

### Intermediate 27

### 4-[(4-Chlorophenyl)methyl]-2-[(2R)-2-pyrrolidinylmethyl]pyrido[3,4-d]pyridazin-1(2H)-one

To a solution of triphenylphosphine (10.42 g, 40 mmol) in anhydrous THF (80 ml) at -10 °C was added a solution of di-*tert*-butyl azodicarboxylate (8.38 g, 36 mmol) (commercially available, for example, from Aldrich) in anhydrous THF (60 ml). The solution was allowed to warm to 15 °C and then cooled to 0-5 °C. To the slight suspension was added a suspension of 4-[(4-chlorophenyl)methyl]pyrido[3,4-d]pyridazin-1(2*H*)-one (for example, as prepared for Intermediate 26) and *N*-Boc-D-prolinol (commercially available, for example from Aldrich) (5.14 g, 25.6 mmol) in anhydrous THF (100 ml). The suspension was allowed to warm to ambient temperature and stirred for 23 h. The solvent was removed *in vacuo* to leave an oil (30 g). LCMS RT = 3.48 min, ES+ve *m*/*z* 455/457. To a solution of the crude product (30 g) in 1,4-dioxane (80 ml) was added 4.0 M HCl in 1,4-dioxane (80 ml, 320 mmol). The solution was stirred at ambient temperature for 5 h. The solvent was removed *in vacuo* and the residue was partitioned between 1 M aqueous hydrochloric acid (400 ml) and EtOAc (200 ml). The phases were separated and the aqueous phase washed with EtOAc (200 ml). The combined organic extracts were washed with 1 M aqueous hydrochloric acid (200 ml). The combined aqueous extracts were basified to pH 10 using 2 aqueous NaOH (300-350 ml) and the resulting suspension extracted with EtOAc (2 × 400 ml, 1 × 200 ml). The combined organic extracts were concentrated *in vacuo* to give the *title compound* (8.0 g). LCMS RT = 2.15 min, ES+ve *m*/*z* 355/357 (M+H)⁺.

### Intermediate 28

### 1,1-Dimethylethyl (4-chlorophenyl)acetate

(4-Chlorophenyl)acetic acid (commercially available, for example from Aldrich) (13.76 g, 81 mmol) was suspended in toluene (100 ml) under nitrogen. To this was added di-*tert-*butyl dimethylacetal (commercially available, for example, from Aldrich) (50 ml) and the reaction mixture was heated at 80 °C for 18 h. The solvent was removed *in vacuo* and the residue was dissolved in EtOAc (200 ml). The solution was washed with saturated sodium bicarbonate solution (2 × 200 ml) and brine (2 × 200 ml). The organic phase was dried over anhydrous MgSO₄ and evaporated *in vacuo* to give the *title compound* (6.68 g, 36%) as pale brown oil. ¹H NMR (CDCl₃) 7.28 (2H, d, J=8.5 Hz), 7.19 (2H, d, J=8.5 Hz), 3.48 (2H, s), 1.43 (9H, s).

### Intermediate 29

### Methyl 3-((2R)-2-{[4-[(4-chlorophenyl)methyl]-1-oxopyrido[3,4-d]pyridazin-2(1H)-yl]methyl}-1-pyrrolidinyl)propanoate

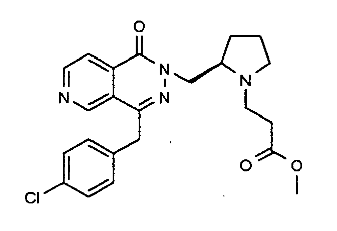

To a solution of 4-[(4-chlorophenyl)methyl]-2-[(2R)-2-pyrrolidinylmethyl]pyrido[3,4-*d*]pyridazin-1(2*H*)-one (for example, as prepared for Intermediate 27) (2.87g, 8.09 mmol) in anhydrous THF (20 ml) was added methyl acrylate (commercially available, for example, from Aldrich) (2.75 ml, 30.6 mmol). The suspension was heated to 50°C under nitrogen for 21 h. The solvent was removed *in vacuo* and the residue was purified by flashmaster II (100 g cartridge, 0-30% MeOH containing 1% triethylamine - dichloromethane over 60 min) and the appropriate fractions combined. The solvent was removed *in vacuo* to teave the *title compound* as a pale pink solid (2.988 g, 84%). LCMS RT = 2.65 min, ES+ve *m*/*z* 441/443 (M+H)⁺.

### Intermediate 30

### 3-((2R)-2-{[4-[(4-Chlorophenyl)methyl]-1-oxopyrido[3,4-d]pyridazin-2(1H)-yl]methyl}-1-pyrrolidinyl)propanoic acid, partial triethylamine salt

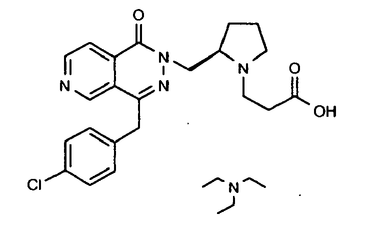

To a solution of methyl 3-((2*R*)-2-{[4-[(4-chlorophenyl)methyl]-1-oxopyrido[3,4-*d*]pyridazin-2(1*H*)-yl]methyl}-1-pyrrolidinyl)propanoate (for example, as prepared for Intermediate 29) (2.5 g, 5.7 mmol) in MeOH (25 ml) was added 2M aqueous sodium hydroxide (10 ml, 20 mmol). The solution was stirred at ambient temperature for 2 h. The solvent was removed *in vacuo* and the residue was dissolved in water (25 ml) and then carefully neutralised with 2M hydrochloric acid (approximately 27 ml) to pH 4-5. The solution was applied to a SCX cartridge (70 g, pre-washed with MeOH and then water). The cartridge was washed with water (1 column volume) and then MeOH (1 column volume). The cartridge was eluted with 10% triethylamine in MeOH (2 column volumes) and the basic fractions concentrated *in vacuo* to leave the *title compound* as a yellow foam (2.55 g, 90%). LCMS RT = 2.34 min, ES+ve *m*/*z* 427/429 (M+H)⁺.

### Intermediate 31

### 2-[2-((2R)-2-{[4-[(4-Chlorophenyl)methyl]-1-oxopyrido[3,4-d]pyridazin-2(1H)-yl]methyl}-1-pyrrolidinyl)ethyl]-1H-isoindole-1,3(2H)-dione

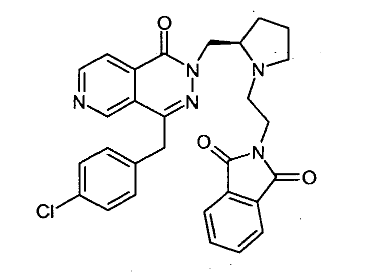

4-[(4-Chlorophenyl)methyl]-2-[(2*R*)-2-pyrrolidinylmethyl]pyrido[3,4-*d*]pyridazin-1(2*H*)-one (for example, as prepared for Intermediated 27) (840 mg, 2.37 mmol) was stirred with 2-(2-bromoethyl)-1*H*-isoindole-1,3(2*H*)-dione (1.51 g, 5.93 mmol) (commercially available, for example, from Aldrich) and potassium carbonate (1.64 g, 11.9 mmol) in 2-butanone (25 ml) under nitrogen at 80 °C overnight. Further potassium carbonate (0.17 g, 1.2 mmol) was added and the heating and stirring were continued for 4 h further. The butanone was removed *in vacuo*. The residue was partitioned between water and DCM. The aqueous layer was extracted with further DCM (x2), and the combined organic layers were washed with water, dried (MgSO₄) and concentrated *in vacuo*. Purification on silica (100 g), eluting with 0-100% EtOAc-cyclohexane over 60 min, gave the *title compound* as a pale yellow foam (996 mg, 80%). LCMS RT = 2.57 min, ES+ve *m*/*z* 528/530 (M+H)⁺.

### Intermediate 32

### 2-{[(2R)-1-(2-Aminoethyl)-2-pyrrolidinyl]methyl}-4-[(4-chlorophenyl)methyl]pyrido[3,4-d]pyridazin-1(2H)-one

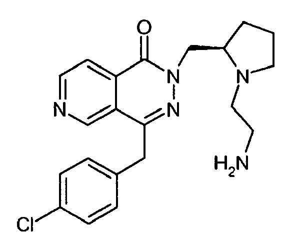

2-[2-((2*R*)-2-{[4-[(4-Chlorophenyl)methyl]-1-oxopyrido[3,4-*d*]pyridazin-2(1*H*)-yl]methyl}-1-pyrrolidinyl)ethyl]-1*H*-isoindole-1,3(2*H*)dione (for example, as prepared for Intermediate 31) (996 mg, 1.89 mmol) was dissolved in EtOH (20 ml) and hydrazine monohydrate (commercially available, for example, from Aldrich) (0.23 ml, 4.73 mmol) was added. The mixture was heated at 80 °C with stirring for 4 h. The reaction was allowed to cool and the solid was removed by filtration, washing with excess EtOH. The combined filtrate and washings were concentrated *in vacuo* to give the *title compound* as a yellow gum (636 mg, 85%) LCMS RT = 2.44 min, ES+ve *m*/*z* 398/400 (M+H)⁺.

### Intermediate 33

### 1,1-Dimethylethyl [3-((2R)-2-{[4-[(4-chlorophenyl)methyl]-1-oxopyrido[3,4-d]pyridazin-2(1H)-yl]methyl}-1-pyrrolidinyl)propyl]carbamate

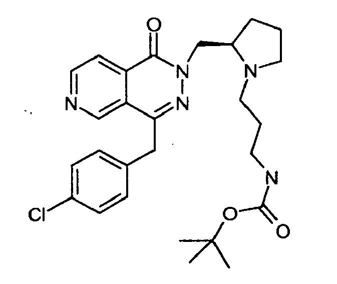

4-[(4-Chlorophenyl)methyl]-2-[(2*R*)-2-pyrrolidinylmethyl]pyrido[3,4-*d*]pyridazin-1(2*H*)-one (for example, as prepared for Intermediate 27) (605 mg, 1.71 mmol) was stirred with 1,1-dimethylethyl(3-bromopropyl)carbamate (488 mg, 2.04 mmol) (commercially available, for example, from Fluka), potassium carbonate (353 mg, 2.56 mmol) and sodium iodide (51 mg, 0.34 mmol) in 2-butanone (20 ml) under nitrogen at 80 °C overnight. Further potassium carbonate (118 mg, 0.85 mmol) was added and the mixture was again heated under nitrogen at 80 °C with stirring for 6 h. The solvent was removed *in vacuo*, and the residue was partitioned between DCM and water. The layers were separated, and the aqueous was extracted with DCM (x2). The combined DCM solution was passed through a hydrophobic frit, and concentrated *in vacuo*. Purification on silica (70 g), eluting with 0-15%(1% aqueous 0.880 ammonia in MeOH) - DCM over 40 min, gave the *title compound* (519 mg, 59%). LCMS RT = 2.57 min, ES+ve *m*/*z* 512/514 (M+H)⁺.

### Intermediate 34

### 2-{[(2R)-1-(3-Aminopropyl)-2-pyrrolidinyl]methyl}-4-[(4-chlorophenyl)methyl]pyrido[3,4-d]pyridazin-1(2H)-one

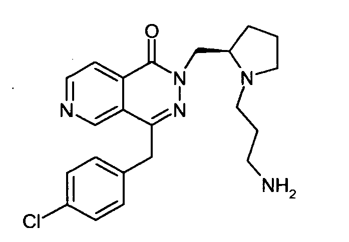

1,1-Dimethylethyl [3-((2*R*)-2-{[4-[(4-chlorophenyl)methyl]-1-oxopyrido[3,4-*d*]pyridazin-2(1*H*)-yl]methyl}-1-pyrrolidinyl)propyl]carbamate, (for example, as prepared for Intermediate 33) (519 mg, 1.0 mmol) was dissolved in dioxane (10 ml) and treated with a solution of HCl in dioxane (4M, 10 ml). The mixture was stirred at room temperature under nitrogen for 1 h. The reaction mixture was concentrated *in vacuo*, and the residue was applied to a SCX-2 cartridge (20 g, pre-washed with MeOH). The cartridge was washed with MeOH and then eluted with 10% aqueous 0.880 ammonia in MeOH. The appropriate basic fractions were combined and the solvent removed *in vacuo*, to give the *title compound* (347 mg, 83%). LCMS RT = 1.95 min, ES+ve *m*/*z* 412/414 (M+H)⁺.

### Intermediate 35

### (2-Oxo-3-piperidinyl)methyl methanesulfonate

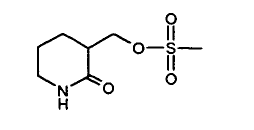

3-(Hydroxymethyl)-2-piperidinone (prepared as disclosed by R. D. Smith et al., J. Med. Chem., 1981, 24, (1) 104-109, compound 2a) (129 mg, 1 mmol) was stirred with triethylamine (0.28 ml, 2 mmol) in DCM (2 ml) at room temperature and methanesulphonyl chloride (commercially available, for example, from Aldrich) (0.086 ml, 1.1 mmol) was added under nitrogen. After 2 h, the mixture was partitioned between DCM and water acidified by addition of 2 M hydrochloric acid. The organic layer washed with water, saturated sodium bicarbonate and water in succession, each time back extracting with DCM. The combined organic solutions were dried (MgSO₄) and evaporated to dryness to give the *title compound* (50mg), LCMS RT = 1.19 min, ES+ve *m*/*z* 208 (M+H)⁺. All the aqueous layers from the above extractions and washings were combined and extracted with DCM (x 3). The combined organic layers were dried (MgSO₄) and evaporated to give further *title compound* (67 mg), LCMS RT = 1.19 min, ES+ve *m*/*z* 208 (M+H)⁺

### Examples

### Example 1

### 3-((2R)-2-{[4-[(4-Chlorophenyl)methyl]-1-oxo-2(1H)-phthalazinyl]methyl}-1-pyrrolidinyl)-N-(2-fluoroethyl)propanamide, trifluoroacetate

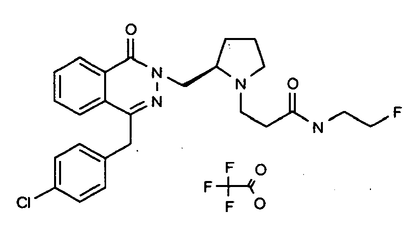

To a solution of 3-((2*R*)-2-{[4-[(4-chlorophenyl)methyl]-1-oxo-2(1*H*)-phthalazinyl]methyl}-1-pyrrolidinyl)propanoic acid -triethylamine (1:1) (for example, as prepared for Intermediate 4) (80 mg, 0.15 mmol) in *N*,*N*-dimethylformamide (1.0 ml) was added triethylamine (0.1 ml, 0.7 mmol) and then a solution of TBTU (commercially available, for example, from Novabiochem) (80 mg, 0.24 mmol) in *N*,*N*-dimethylformamide (0.5 ml). The solution was stirred for 3 to 5 min. To the solution was added 2-fluoroethylamine hydrochloride (commercially available, for example, from Rare Chemicals). (40 mg, 0.40 mmol) The mixture was stirred overnight. The mixture was applied to a SCX cartridge (20 g, pre-washed with MeOH). The cartridge washed with MeOH (2 column volumes) and then eluted with 10% 0.880 ammonia in MeOH (2 column volumes). The appropriate basic fractions were combined and the solvent removed *in vacuo*. The residue was purified by MDAP HPLC and the appropriate fractions combined. The solvent was removed *in vacuo* and the residue dissolved in MeOH (2 to 3 ml). To the methanolic solution was added trifluoroacetic acid (0.2 ml). The solvent and excess trifluoroacetic acid were removed using a stream of nitrogen to give the *title compound* (36 mg, 40%). LCMS RT = 2.44 min, ES+ve *m*/*z* 471/473 (M+H)⁺.

### Example 2

### 3-((2R)-2-{[4-[(4-chlorophenyl)methyl]-1-oxo-2(1H)-phthalazinyl]methyl}-1-pyrrolidinyl)-2-methyl-N-[2-(methyloxy)ethyl]propanamide, hydrochloride

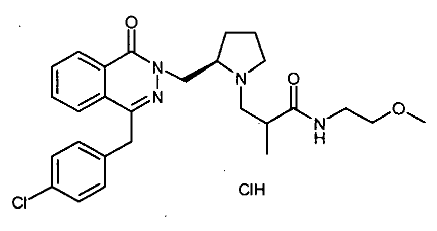

3-((2*R*)-2-{[4-[(4-chlorophenyl)methyl]-1-oxo-2(1*H*)-phthaiazinyl]methyl}-1-pyrrolidinyl)-2-methylpropanoic acid - formate salt (1:1) (for example, as prepared for Intermediate 6) 13.5 mg, 0.028 mmol) was stirred with TBTU (commercially available, for example from Novabiochem) (56 mg, 0.18 mmol) and 2-methoxyethylamine (commercially available, for example, from Aldrich)(13 µl, 0.15 mmol) in DMF (1 ml) containing triethylamine (0.082 ml, 0.59 mmol) at room temperature. After two hours the mixture was applied to a SCX-2 cartridge (10 g, pre-conditioned with MeOH). The cartridge was washed through with MeOH and then the product was eluted with 10% aqueous 0.880 ammonia in MeOH. The appropriate eluate was evaporated to dryness and the residue was dissolved in MeOH (approximately 10 ml) and 2 M hydrochloric acid (5 drops) was added. The solution was evaporated to dryness and the residue was re-evaporated to dryness with MeOH and thoroughly pumped to give the *title compound* (12.1 mg). LCMS RT = 2.53 min, ES+ve *m*/*z* 497/499 (M+H)⁺.

### Example 3

### Formic acid - 3-((2R)-2-{[4-[(4-chlorophenyl)methyl]-1-oxo-2(1H)-phthalazinyl]methyl}-1-pyrrolidinyl)-N-(phenylmethyl)propanamide (1:1)

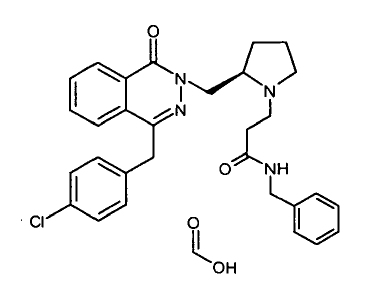

4-[(4-Chlorophenyl)methyl]-2-[(2*R*)-2-pyrrolidinylmethyl]-1(2*H*)-phthalazinone (for example, as prepared for Intermediate 2) (35 mg, 0.1 mmol) was heated at 80 °C for three days with *N*-benzylacrylamide (commercially available, for example, from ABCR Chemicals), (24 mg, 0.15 mmol) in DMF (0.1 ml). The solution was poured onto a SCX-2 cartridge (2 g) which had been pre-conditioned with MeOH. The reaction mixture was washed on with MeOH. It was then washed through with MeOH and the crude product was eluted with 10% aqueous 0.880 ammonia solution in MeOH. This was purified using MDAP HPLC and evaporation of the appropriate eluate gave the *title compound* (15.9 mg). LCMS RT = 2.86 min, ES+ve *m*/*z* 515/517 (M+H)⁺.

### Examples 4 - 23

The following examples were prepared by a generally similar method to that described herein in Example 1 using 3-((2*R*)-2-{[4-[(4-Chlorophenyl)methyl]-1-oxo-2(1*H*)-phthalazinyl]methyl}-1-pyrrolidinyl)propanoic acid -triethylamine (1:1) (for example, as prepared for Intermediate 4) and the appropriate amine.

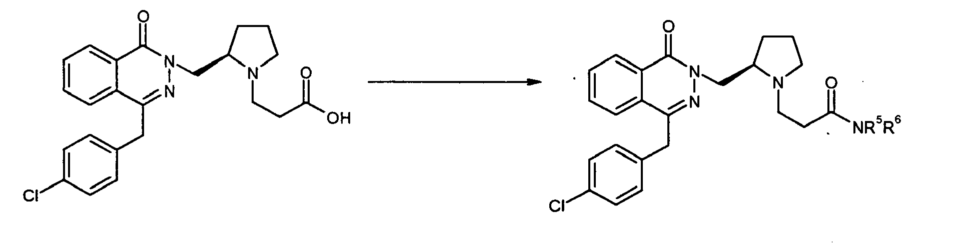

The following amines were used:
2-Fluoroethylamine hydrochloride, (commercially available for example from Rare Chemicals)
Methylamine hydrochloride, (commercially available for example from Sigma)
Ammonia, (commercially available for example from BDH, as 0.880 specific gravity)
2,2-Difluoroethylamine, (commercially available for example from Apollo)
2,2,2-Trifluoroethylamine, (commercially available for example from Acros)
Piperidine, (commercially available for example from Lancaster)
*N*-(2-Methoxyethyl)methylamine, (commercially available for example from Fluorochem)
{2-[(2,2,2-Trifluoroethyl)oxy]ethyl}amine, (for example, as prepared for Intermediate 8)
2-Aminoethyl ethyl ether, (commercially available for example from TCI)
*N*-Ethyl-*N*-methylamine, Morpholine, Ethylamine (as 2M solution in THF), Diethylamine,
Isopropylamine, Propylamine, *tert*-Butylamine, 2-Methoxyethylamine and 3-Methoxypropylamine (all commercially available for example from Aldrich)

| Ex No | Compound Name | Structure | Amine | LCMS RT (min) | ES+ve *m*/*z* (M+H)+ |
|---|---|---|---|---|---|
| 4 | 3-((2*R*)-2-{[4-[(4-chlorophenyl)methyl]-1-oxo-2(1*H*)-phthalazinyl]methyl}-1-pyrrolidinyl)-*N*-(2,2,2-trifluoroethyl)propanamide | 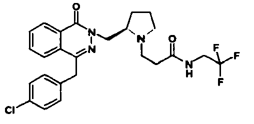 | 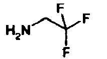 | 2.64 | 507 and 509 |
| 5 | 3-((2*R*)-2-{[4-[(4-chlorophenyl)methyl]-1-oxo-2(1*H*)-phthalazinyl]methyl}-1-pyrrolidinyl)-*N*-(2,2,2-trifluoroethyl)propanamide, trifluoroacetate | 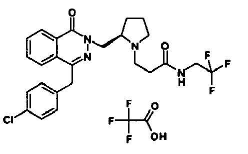 | 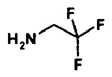 | 2.62 | 507 and 509 |
| 6 | formic acid - 3-((2*R*)-2-{[4-[(4-chlorophenyl)methyl]-1-oxo-2(1*H*)-phthalazinyl]methyl}-1-pyrrolidinyl)-*N*-(2,2-difluoroethyl)propanamide (1:1) | 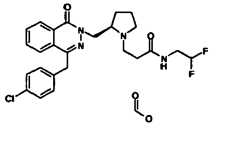 | 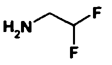 | 2.55 | 489 and 491 |
| 7 | 3-((2*R*)-2-{[4-[(4-chlorophenyl)methyl]-1-oxo-2(1*H*)-phthalazinyl]methyl}-1-pyrrolidinyl)-*N*-(2,2-difluoroethyl)propanamide, trifluoroacetate | 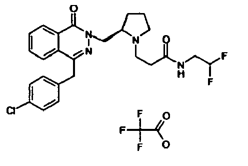 | 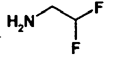 | 2.54 | 489 and 491 |
| 8* | 3-((2*R*)-2-{[4-[(4-chlorophenyl)methyl]-1-oxy-2(1*H*)-phthalazinyl]methyl}-1-pyrrolidinyl)-*N*-(2-fluoroethyl)propanamide, trifluoroacetate | 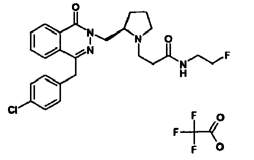 | 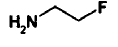 | 2.44 | 471 and 473 |
| 9 | 3-((2*R*)-2-{[4-[(4-chlorophenyl)methyl]-1-oxo-2(1*H*)-phthalazinyl]methyl}-1-pyrrolidinyl)-*N*-methylpropanamide, trifluoroacetate | 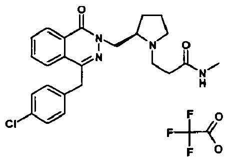 | 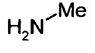 | 2.37 | 439 and 441 |
| 10 | 3-((2*R*)-2-{[4-[(4-chlorophenyl)methyl]-1-oxo-2(1*H*)-phthalazinyl]methyl}-1-pyrrolidinyl)propanamide, trifluoroacetate | 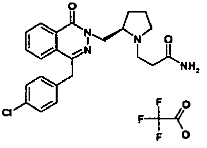 | NH₃ | 2.32 | 425 and 427 |
| 11 | 3-((2*R*)-2-{[4-[(4-chlorophenyl)methyl]-1-oxo-2(1*H*)-phthalazinyl]methyl}-1-pyrrolidinyl)-*N*-ethyl-*N*-methylpropanamide, trifluoroacetate | 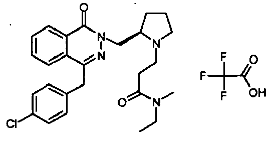 | 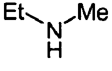 | 2.55 | 467 and 469 |
| 12 | 4-[(4-chlorophenyl)methyl]-2-({(2*R*)-1-[3-oxo-3-(1-piperidinyl)propyl]-2-pyrrolidinyl}methyl)-1(2*H*)-phthalazinone, trifluoroacetate | 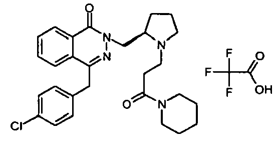 | 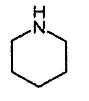 | 2.65 | 493 and 495 |
| 13 | 4-[(4-chlorophenyl)methyl]-2-({(2*R*)-1-[3-(4-morpholinyl)-3-oxopropyl]-2-pyrrolidinyl}methyl-1(2*H*)-phthalazinone, trifluoroacetate | 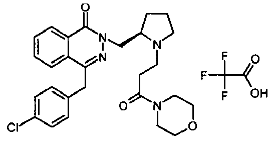 | 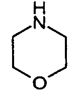 | 2.46 | 495 and 497 |
| 14 | 3-((2*R*)-2-{[4-[(4-chlorophenyl)methyl]-1-oxo-2(1*H*)-phthalazinyl]methyl}-1-pyrrolidinyl)-*N*-methyl-*N*-[2-(methyloxy)ethyl]propanam ide, trifluoroacetate | 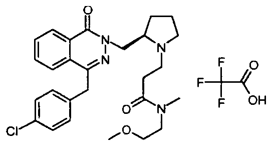 | 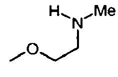 | 2.45 | 497 and 499 |
| 15 | 3-((2*R*)-2-{[4-[(4-chlorophenyl)methyl]-1-oxo-2(1*H*)-phthalazinyl]methyl}-1-pyrrolidinyl)-*N*-{2-[(2,2,2-trifluoroethyl)oxy]ethyl}prop anamide, trifluoroacetate | 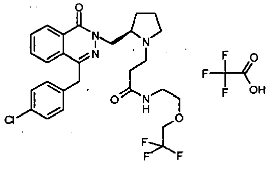 | 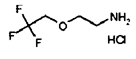 | 2.67 | 551 and 553 |
| 16 | 3-((2*R*)-2-{[4-[(4-chlorophenyl)methyl]-1-oxo-2(1*H*)-phthalazinyl]methyl}-1-pyrrolidinyl)-*N*-ethylpropanamide, trifluoroacetate | 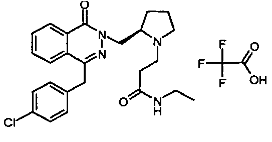 | 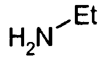 | 2.49 | 453 and 455 |
| 17 | 3-((2*R*)-2-{[4-[(4-chlorophenyl)methyl]-1-oxo-2(1*H*)-phthalazinyl]methyl}-1-pyrrolidinyl)-*N*-ethylpropanamide, trifluoroacetate | 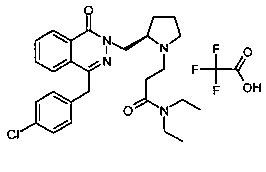 | 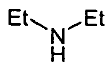 | 2.64 | 481 and 483 |
| 18 | 3-((2*R*)-2-{[4-[(4-chlorophenyl)methyl]-1-oxo-2(1*H*)-phthalazinyl]methyl}-1-pyrrolidinyl)-*N*-(1-methylethyl)propanamide, trifluoroacetate | 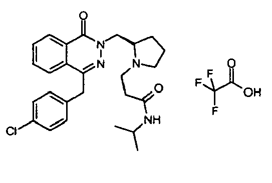 | 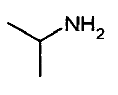 | 2.97 | 467 and 469 |
| 19 | 3-((2*R*)-2-{[4-[(4-chlorophenyl)methyl]-1-oxo-2(1*H*)-phthalazinyl]methyl}-1-pyrrolidinyl)-*N*-propylpropanamide, trifluoroacetate | 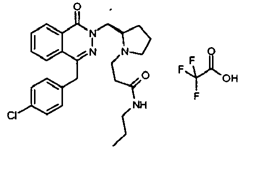 | 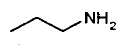 | 3.01 | 467 and 469 |
| 20 | 3-((2*R*)-2-{[4-[(4-chlorophenyl)methyl]-1-oxo-2(1*H*)-phthalazinyl]methyl}-1-pyrrolidinyl)-*N*-(1,1-dimethylethyl)propanamide trifluoroacetate | 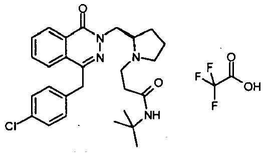 | 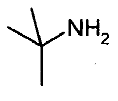 | 3.16 | 482 and 484 |
| 21 | 3-((2*R*)-2-{[4-[(4-chlorophenyl)methyl]-1-oxo-2(1*H*)-phthalazinyl]methyl}-1-pyrrolidinyl)-*N*-[2-(methyloxy)ethyl]propanam ide, trifluoroacetate | 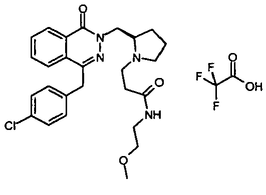 | 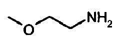 | 2.84 | 483 and 485 |
| 22 | 3-((2*R*)-2-{[4-[(4-chlorophenyl)methyl]-1-oxo-2(1*H*)-phthalazinyl]methyl}-1-pyrrolidinyl)-*N*-[2-(ethyloxy)ethyl]propanamid e, trifluoroacetate | 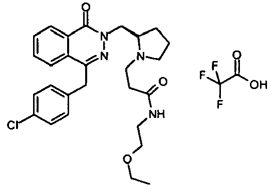 | 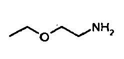 | 2.91 | 497 and 499 |
| 23 | 3-((2*R*)-2-{[4-[(4-chlorophenyl)methyl]-1-oxo-2(1*H*)-phthalazinyl]methyl}-1-pyrrolidinyl)-*N*-[3-(methyloxy)propyl]propana mide, trifluoroacetate | 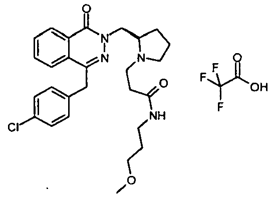 | 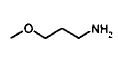 | 2.57 | 497 and 499 |

Example 8 is the same compound as Example 1

### Examples 24 - 26

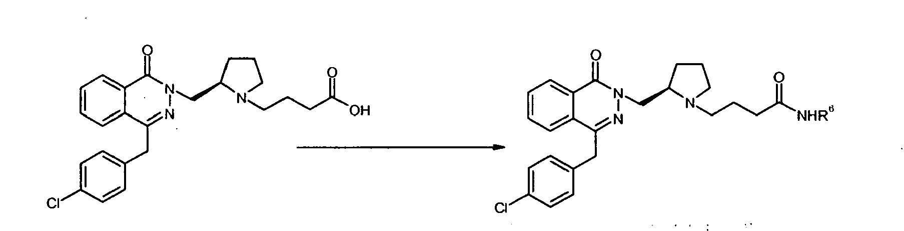

The following Examples were prepared by a generally similar method to that described herein in Example 1 using 4-((2*R*)-2-{[4-[(4-chlorophenyl)methyl]-1-oxo-2(1*H*)-phthalazinyl]methyl}-1-pyrrolidinyl)butanoic acid, formate salt (1:1) (for example, as prepared for Intermediate 10) and the appropriate amine.

The following amines were used
[2-(methyloxy)ethyl]amine and propylamine (all commercially available, for example, from Aldrich)

| Ex No | Compound Name | Structure | Amine | LCMS RT (min) | ES+ve *m*/*z* (M+H)⁺ |
|---|---|---|---|---|---|
| 24 | Formic acid - 4-((2*R*)-2-{[4-[(4-chlorophenyl)methyl]-1-oxo-2(1*H*)-phthalazinyl]methyl}-1-pyrrolidinyl)-*N*-[2-(methyloxy)ethyl]butanamide (1:1) | 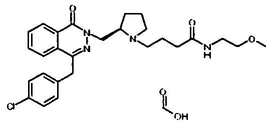 | 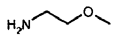 | 2.55 | 497 and 499 |
| 25 | 4-((2*R*)-2-{[4-[(4-Chlorophenyl)methyl]-1-oxo-2(1*H*)-phthalazinyl]methyl}-1-pyrrolidinyl)-*N*-[2-(methyloxy)ethyl]butanamide hydrochloride | 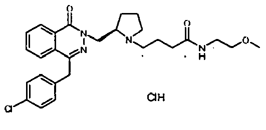 | 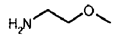 | 2.48 | 4.97 and 499 |
| 26 | 4-((2*R*)-2-{[4-[(4-Chlorophenyl)methyl]-1-oxo-2(1*H*)-phthalazinyl]methyl}-1-pyrrolidinyl)-*N*-propylbutanamide, hydrochloride | 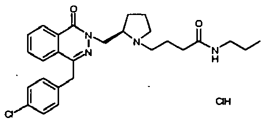 | 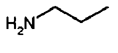 | 2.62 | 481 and 483 |

Example 25 was prepared from formic acid.- 4-((2*R*)-2-{[4-[(4-chlorophenyl)methyl]-1-oxo-2(1*H*)-phthalazinyl]methyl}-1-pyrrolidinyl)-*N*-[2-(methyloxy)ethyl]butanamide (1:1) (for example, as prepared for Example 24) using SCX-2 cartridge and eluting with 0.880 ammonia in MeOH and then adding excess HCl and drying.

### Example 27

### 2-((2R)-2-{[4-[(4-Chlorophenyl)methyl]-1-oxo-2(1H)-phthalazinyl]methyl}-1-pyrrolidinyl)-N-[3-(methyloxy)propyl]acetamide, trifluoroacetate

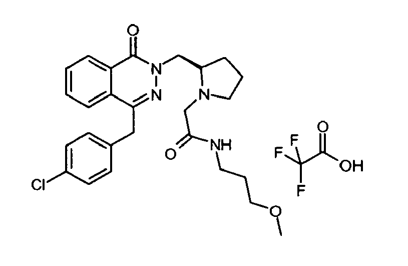

To a solution of ((2*R*)-2-{[4-[(4-chlorophenyl)methyl]-1-oxo-2(1*H*) phthalazinyl]methyl}-1-pyrrolidinyl)acetic acid trifluoroacetate, (for example, as prepared for Intermediate 12) (0.045 g, 0.086 mmol) in dry dimethylformamide (1 ml) was added *O*-(1*H*-benzotriazol-1-yl)-*N*,*N*,*N',N'*-tetramethyluronium tetrafluoroborate (TBTU) (commercially available, for example, from Novabiochem) (0.045 g, 0.14 mmol). The reaction mixture was stirred at room temperature for 10 min before adding 3-methoxypropylamine (commercially available, for example, from Aldrich) (0.010 ml, 0.11 mmol) followed with triethylamine (0.046 ml, 0.327 mmol). The resultant pale yellow solution was stirred at room temperature overnight and then applied onto a pre-conditioned (MeOH) SCX-2 cartridge (10 g), washed with MeOH (x 3) and then eluted with 10% 0.880 aqueous ammonia in MeOH (x 4). The solvents were removed *in vacuo* and the residue purified by MDAP HPLC and treated with trifluoroacetic acid to afford the *title compound* (21 mg). LCMS RT = 2.88 min ES+ve *m*/*z* 483 and 485 (M+H)⁺

### Examples 28 - 30

Examples 28 to 30 were prepared by a generally similar method to that described herein in Example 1 using ((2*R*)-2-{[4-[(4-Chlorophenyl)methyl]-1-oxo-2(1*H*)-phthalazinyl]methyl}-1-pyrrolidinyl)acetic acid trifluoroacetate (for example, as prepared for Intermediate 12) and the appropriate amine.

### The following amines were used

Butylamine, *tert*-butylamine and piperidine (all commercially available, for example, from Aldrich)

| Ex No | Compound Name | Structure | Amine | RT (min) | ES+ve *m*/*z* (M+H)⁺ |
|---|---|---|---|---|---|
| 28 | *N*-butyl-2-((2*R*)-2-{[4-[(4-chlorophenyl)methyl]-1-oxo-2(1*H*)-phthalazinyl]methyl}-1-pyrrolidinyl)acetamid e, trifluoroacetate | 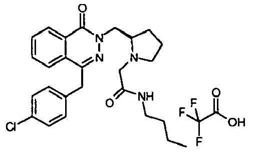 | 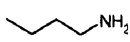 | 2.99 | 453 and 455 |
| 29 | 4-[(4-chlorophenyl)methyl]-2-({(2*R*)-1-[2-oxo-2-(1-piperidinyl)ethyl]-2-pyrrolidinyl}methyl)-1(2*H*)-phthalazinone, trifluoroacetate | 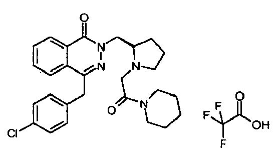 | 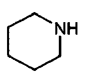 | 3.01 | 479 and 481 |
| 30 | 2-((2*R*)-2-{[4-[(4-chlorophenyl)methyl]-1-oxo-2(1*H*)-phthalazinyl]methyl}-1-pyrrolidinyl)-*N*-(1,1-dimethylethyl)acetam ide, trifluoroacetate | 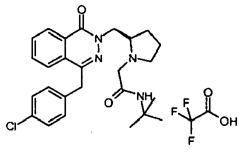 | 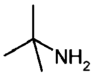 | 3.10 | 467 and 469 |

### Examples 31 - 33

The following examples were prepared by a generally similar method to that described herein in Example 1 using 3-{4-[4-[(4-Chlorophenyl)methyl]-1-oxo-2(1*H*)-phthalazinyl]-1-piperidinyl}propanoic acid -triethylamine (1:1) (for example, as prepared for Intermediate 16) and the appropriate amine.

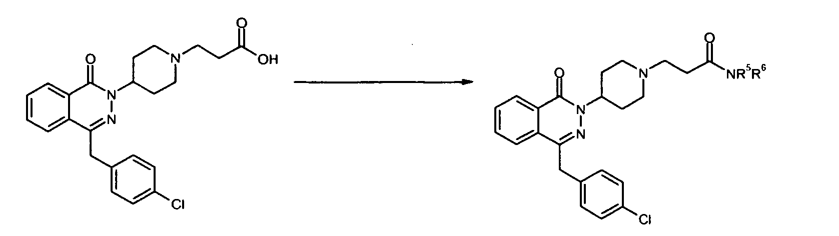

The following amines were used
[2-(methyloxy)ethyl]amine, [3-(methyloxy)propyl]amine and ethylamine (commercially available, for example, from Aldrich)

| Ex No | Compound Name | Structure | Amine | LCMS RT (min) | ES+ve *m*/*z* (M+H)⁺ |
|---|---|---|---|---|---|
| 31 | 3-{4-[4-[(4-Chlorophenyl)methyl]-1-oxo-2(1*H*)-phthalazinyl]-1-piperidinyl}-*N*-[2-(methyloxy)ethyl]propana mide, trifluoroacetate | 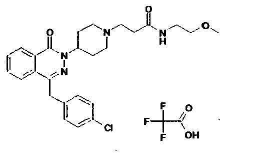 | 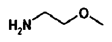 | 2.48 | 483 and 485 |
| 32 | 3-{4-[4-[(4-Chlorophenyl)methyl]-1-oxo-2(1*H*)-phthalazinyl]-1-piperidinyl}-*N*-[3-(methyloxy)propyl]propan amide, trifluoroacetate | 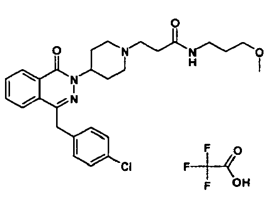 | 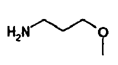 | 2.52 | 497 and 499 |
| 33 | 3-{4-[4-[(4-Chlorophenyl)methyl]-1-oxo-2(1*H*)-phthalazinyl]-1-piperidinyl}-*N-*ethylpropanamide, trifluoroacetate | 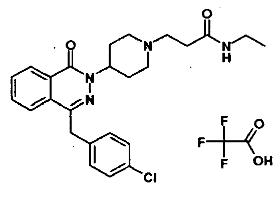 | 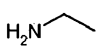 | 2.53 | 453 and 455 |

### Example 34

### N-[2-((2R)-2-{[4-[(4-Chlorophenyl)methyl]-1-oxo-2(1H)-phthalazinyl]methyl}-1-pyrrolidinyl)ethyl]-4-(methyloxy)butanamide

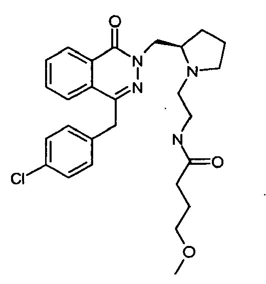

### Preparation (a)

4-(Methyloxy)butanoic acid (for example, as prepared for Intermediate 17) (155 mg, 1.31 mmol) was dissolved in DMF (3 ml) with stirring. Triethylamine (555 µl, 3.98 mmol) was added, followed by TBTU (422 mg, 1.31 mmol) in DMF (2 ml), and the mixture was stirred at room temperature. 2-{[(2*R*)-1-(2-Aminoethyl)-2-pyrrolidinyl]methyl}-4-[(4-chlorophenyl)methyl]-1(2*H*)-phthalazinone (for example, as prepared for intermediate 19) (401 mg, 1.01 mmol) in DMF (3 ml) was added and the mixture was stirred at room temperature for 2 h. The reaction mixture was applied to a silica cartridge (70 g, pre-conditioned with MeOH) and eluted with MeOH (3 column volumes) then 10% aqueous 0.88 s.g. ammonia in MeOH (3 column volumes). Appropriate fractions were combined and concentrated *in vacuo*. The residue was redissolved in MeOH and applied to a SCX cartridge (70 g, pre-conditioned with MeOH). The cartridge was washed with MeOH (2 column volumes) and then eluted with 10% 0.880 s.g. ammonia in MeOH (3 column volumes). The appropriate basic fractions were combined and the solvent removed *in vacuo.* The residue was purified by chromatography on silica (50 g, eluting with DCM-EtOH-aqueous ammonia, 200:8:1 (400 ml), then 150:8:1 (450 ml)). The appropriate fractions were combined and concentrated *in vacuo*. The sample was redissolved in EtOH and concentrated *in vacuo* to give the *title compound* (446 mg, 89%). LCMS RT = 2.86 min, ES+ve *m*/*z* 497/499 (M+H)⁺. ¹H NMR (400 MHz, MeOH-*d*₄) δ 8.43-8.36 (m, 1H), 7.96-7.90 (m, 1H), 7.89-7.81 (m, 2H), 7.34-7.27 (m, 4H), 4.36 (s, 2 H), 4.31 (dd, *J* = 13, 4 Hz, 1H), 4.24 (dd, *J* = 13, 7 Hz, 1H), 3.41-3.24 (m, 7H), 3.21-3.13 (m, 1H), 3.10-3.00 (m, 2H), 2.55-2.46 (m, 1H), 2.36-2.27 (m, 1H), 2.26 (t, *J* = 8 Hz, 2H), 1.94-1.68 (m, 6H).

### Preparation (b)

To the 4-methyloxybutanoic acid (1.49g 12.6mmol) in dry DMF (20ml) under nitrogen was added TBTU (4.38g 13.65mmol) followed by triethylamine (5.85ml 42mmol) and the mixture stirred for 10min at room temperature. To this mixture was added 2-{[(2*R*)-1-(2-aminoethyl)-2-pyrrolidinyl]methyl}-4-[(4-chlorophenyl)methyl]-1(2*H*)-phthalazinone (for example, as prepared for Intermediate 19) (4.17g 10.5mmol) suspended in dry DMF (40ml) and the mixture stirred for 60mins under nitrogen at room temperature. The mixture was then evaporated to a small volume and the residue applied to silica cartridge (120g). This was eluted using a gradient 0-10% DCM/MeOH in DCM. The product containing fractions were combined and evaporated to dryness. This contained an impurity, therefore, the material was taken up into DCM (300ml) and washed with 1 N NaOH (2 x 50ml), water (2 x 100ml) and the organic layer separated passed through phase separator. The filtrate was evaporated to dryness and purified on silica cartridge (100g) eluting with 7%MeOH in DCM isocratically. The product containing fractions were combined and evaporated to dryness. The residue obtained still contained a small amount of impurity, therefore, dissolved up into DCM (250ml) and washed with 2N NaOH (2 x 50ml), water (2 x 50ml) and passed through phase separator. The filtrate was evaporated to dryness to leave the product which was triturated with ether (200ml) for 10min. The solid was filtered off, washed with ether (50ml) and dried thoroughly in vacuo overnight to give the title compound (3.9g) as a solid. NMR consistent with the required product and LCMS RT = 2.85min ES+ve m/z 497/499 (M+H)⁺.

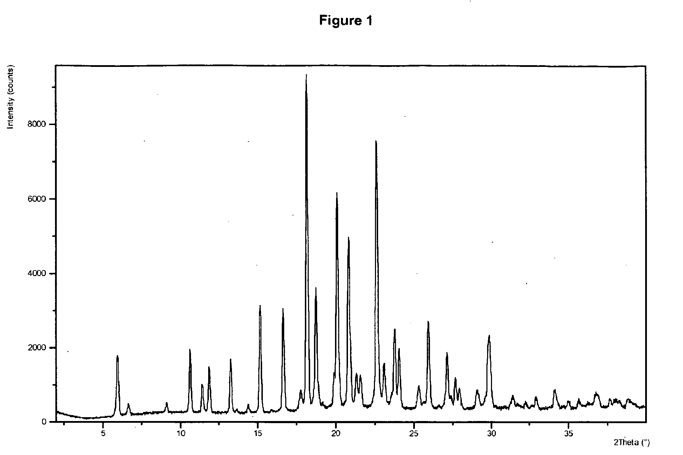

An XRPD pattern of *N*-[2-((2*R*)-2-{[4-[(4-Chlorophenyl)methyl]-1-oxo-2(1*H*)-phthalazinyl]methyl}-1-pyrrolidinyl)ethyl]-4-(methyloxy)butanamide (as prepared in Preparation (b)) is shown in Figure 1. The peak angles and d-spacings for this form are tabulated below.

| **Pos. [°2Th.]** | **d-spacing [Å]** |
|---|---|
| 6.0 | 14.8 |
| 6.7 | 13.3 |
| 9.1 | 9.7 |
| 10.6 | 8.4 |
| 11.4 | 7.8 |
| 11.8 | 7.5 |
| 13.2 | 6.7 |
| 15.2 | 5.8 |
| 16.6 | 5.3 |
| 18.2 | 4.9 |
| 18.7 | 4.7 |
| 20.1 | 4.4 |
| 20.8 | 4.3 |
| 22.6 | 3.9 |
| 23.1 | 3.9 |
| 23.7 | 3.7 |
| 24.0 | 3.7 |
| 25.9 | 3.4 |
| 27.1 | 3.3 |
| 29.8 | 3.0 |

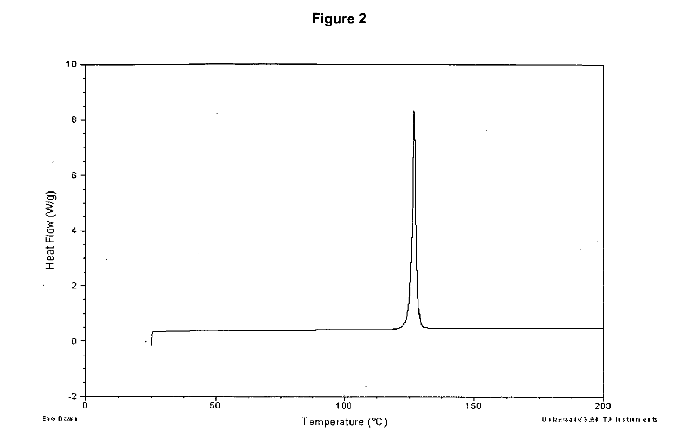

The DSC thermogram plots the differential rate of heating in watts per second against temperature. A DSC thermogram of *N*-[2-((2*R*)-2-{[4-[(4-clorophenyl)methyl]-1-oxo-2(1*H*)-phthalazinyl]methyl}-1-pyrrolidinyl)ethyl]-4-(methyloxy)butanamide (as prepared in Preparation (b)) is shown in Figure 2. A melting endotherm was observed at an onset temperature of 126 °C.

### Example 34a

### N-[2-((2R)-2-{[4-[(4-Chlorophenyl)methyl]-1-oxo-2(1H)-phthalazinyl]methyl}-1-pyrrolidinyl)ethyl]-4-(methyloxy)butanamide, hydrochloride salt

A sample of *N*-[2-((2*R*)-2-{[4-[(4-chlorophenyl)methyl]-1-oxo-2(1*H*)-phthalazinyl]methyl}-1-pyrrolidinyl)ethyl]-4-(methyloxy)butanamide (for example, as prepared for Example 34 Preparation (a)) (330 mg) was dissolved in MeOH (3 ml) and treated with a solution of HCl in MeOH (1.25 mmol, 3 ml). The solvent and excess acid were then removed using a stream of nitrogen, and the residue dried *in vacuo* at 45 °C to give the *title compound* (352 mg, 99%). LCMS RT = 2.85 min, ES+ve *m*/*z* 497/499 (M+H)⁺, ¹H NMR (400 MHz, MeOH-*d*₄) δ 8.45-8.41 (m, 1H), 8.02-7.98 (m, 1H), 7.95-7.86 (m, 2H), 7.36-7.29 (m, 4H), 4.74 (dd, *J* = 15, 4 Hz, 1H), 4.62 (dd, *J* = 15, 5.5 Hz, 1H), 4.41 (s, 2H), 4.11-4.03 (m, 1H), 3.92-3.84 (m, 1H), 3.72-3.63 (m, 2H), 3.56-3.46 (m, 1H), 3.37 (t, *J* = 6 Hz, 2H), 3.38-3.27 (obscured by MeOH) (m, 2H), 3.30 (s, 3H), 2.39-2.30 (m, 3H), 2.23-2.11 (m, 1H), 2.06-1.94 (m, 2H), 1.83-1.75 (m, 2H).

### Example 34b

### N-[2-((2R)-2-{[4-[(4-Chlorophenyl)methyl]-1-oxo-2(1H)-phthalazinyl]methyl}-1-pyrrolidinyl)ethyl]-4-(methyloxy)butanamide, formic acid salt

2-{[(2*R*)-1-(2-Aminoethyl)-2-pyrrolidinyl]methyl}-4-[(4-chlorophenyl)methyl]-1(2*H*)-phthalazinone (for example, as prepared for Intermediate 19) (35 mg, 0.088 mmol), TBTU (38 mg, 0.12 mmol), triethylamine (70µl, 0.50 mmol) and 4-(methyloxy)butanoic acid (for example, as prepared for Intermediate 17) (12 mg, 0.10 mmol) were stirred together in DMF (2 ml) at room temperature for 2 hours. The reaction mixture was applied to a SCX cartridge (10 g, pre-washed with MeOH). The cartridge was washed with MeOH and then eluted with 10% 0.880 ammonia in MeOH. The appropriate basic fractions were combined and the solvent removed *in vacuo.* The residue was purified by MDAP HPLC and the appropriate fractions combined. The solvent was removed *in vacuo* and the residue was re-dissolved in DCM and treated with excess formic acid to ensure isolation of the formate salt of the product. The solvent and excess formic acid were removed using a stream of nitrogen to give the *title compound* (27 mg, 56%). LCMS RT = 2.52 min, ES+ve *mlz* 497/499 (M+H)⁺, ¹H NMR (400 MHz, MeOH-*d*₄) δ 8.45-8.40 (m, 1H), 8.22 (br s, 1H), 8.02-7.97 (m, 1H), 7.95-7.85 (m, 2H), 7.36-7.28 (m, 4H), 4.65 (dd, *J* = 15, 4 Hz, 1H), 4.57 (dd, *J* = 14, 5 Hz, 1H), 4.40 (s, 2 H), 3.96-3.88 (m, 1H), 3.72-3.80 (m, 1H), 3.45-3.66 (m, 3H), 3.37 (t, *J* = 6 Hz, 2H), 3.30 (s, 3H), 3.25-3.14 (m, 2H), 2.32 (t, *J*=7.5 Hz, 2H), 2.33-2.20 (m, 1H), 2.15-1.91 (m, 3H), 1.84-1.75 (m, 2H).

### Example 34c

### N-[2-((2R)-2-{[4-[(4-Chlorophenyl)methyl]-1-oxo-2(1H)-phthalazinyl]methyl}-1-pyrrolidinyl)ethyl]-4-(methyloxy)butanamide, trifluoroacetate salt

4-(Methyloxy)butanoic acid (for example, as prepared for Intermediate 17) (30 mg, 0.25 mmol), TBTU (88 mg, 0.27 mmol) and triethylamine (119 µl, 0.85 mmol) were stirred together in DMF (4 ml) under nitrogen for 10 min. 2-{[(2*R*)-1-(2-aminoethyl)-2-pyrrolidinyl]methyl}-4-[(4-chlorophenyl)methyl]-1(2*H*)-phthalazinone (for example, as prepared for Intermediate 19) (85 mg, 0.21 mmol) was added and the mixture was stirred at room temperature under nitrogen for 2 h. The reaction mixture was applied to a SCX cartridge (70 g, pre-washed with MeOH). The cartridge was washed with MeOH and then eluted with 10% 0.880 ammonia in MeOH. The appropriate basic fractions were combined and the solvent removed *in vacuo.* The residue was purified by MDAP and the appropriate fractions combined. The solvent was removed *in vacuo* and the residue re-dissolved in DCM and treated with excess trifluoroacetic acid to convert the product to the trifluoroacetate salt. The solvent and excess acid were removed using a stream of nitrogen to give the *title compound* (97 mg, 74%). LCMS RT = 2.47 min, ES+ve *m*/*z* 497/499 (M+H)⁺, ¹H NMR (400 MHz, MeOH-*d*₄) δ 8.45-8.40 (m, 1H), 8.02-7.98 (m, 1H), 7.95-7.86 (m, 2H), 7.36-7.29 (m, 4H), 4.73 (dd, *J* = 15,4 Hz, 1H), 4.61 (dd, *J* = 16, 5.5 Hz, 1H), 4.41 (s, 2H), 4.10-4.02 (m, 1H), 3.92-3.83 (m, 1H), 3.72-3.62 (m, 2H), 3.55-3.45 (m, 1H), 3.37 (t, *J* = 6 Hz, 2H), 3.38-3.27 (obscured by MeOH) (m, 2H), 3.30 (s, 3H), 2.39-2.29 (m, 3H), 2.22-2.10 (m, 1H), 2.06-1.94 (m, 2H), 1.82-1.74 (m, 2H).

### Example 34d

### N-[2-((2R)-2-{[4-[(4-Chlorophenyl)methyl]-1-oxo-2(1H)-phthalazinyl]methyl}-1-pyrrolidinyl)ethyl]-4-(methyloxy)butanamide, tartrate salt

*N*-[2-((2*R*)-2-{[4-[(4-Chlorophenyl)methyl]-1-oxo-2(1*H*)-phthalazinyl]methyl}-1-pyrrolidinyl)ethyl]-4-(methyloxy)butanamide (for example, as prepared for Example 34 Preparation (b)) (400 mg) was dissolved in acetone (4 ml). To this was added (L)-tartaric acid (126.8 mg) dissolved in acetone (4 ml) and the reaction was left to temperature cycle (0 - 40 °C) over the weekend. The solid was isolated and washed with acetone (approximately 1 ml) and left to dry in air for approximately 2 h and then overnight *in vacuo* at room temperature to give the *title compound* (464.7 mg). LCMS RT = 2.73 min, ES+ve *m*/*z* 497/499 (M+H)⁺.

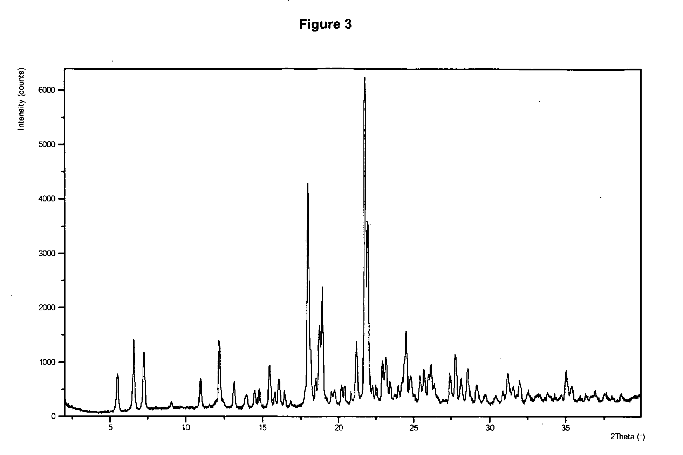

An XRPD pattern of *N*-[2-((2*R*)-2-{[4-[(4-Chlorophenyl)methyl]-1-oxo-2(1*H*)-phthalazinyl]methyl}-1-pyrrolidinyl)ethyl]-4-(methyloxy)butanamide tartrate salt (as prepared in Example 34d) is shown in Figure 3. The peak angles and d-spacings for this form are tabulated below.

| **Pos. [°2Th.]** | **d-spacing [Å]** |
|---|---|
| 5.5 | 16.0 |
| 6.6 | 13.4 |
| 7.3 | 12.2 |
| 11.0 | 8.1 |
| 12.2 | 7.3 |
| 15.5 | 5.7 |
| 18.0 | 4.9 |
| 18.8 | 4.7 |
| 18.9 | 4.7 |
| 21.2 | 4.2 |
| 21.7 | 4.1 |
| 21.9 | 4.0 |
| 22.9 | 3.9 |
| 23.2 | 3.8 |
| 24.5 | 3.6 |
| 27.7 | 3.2 |

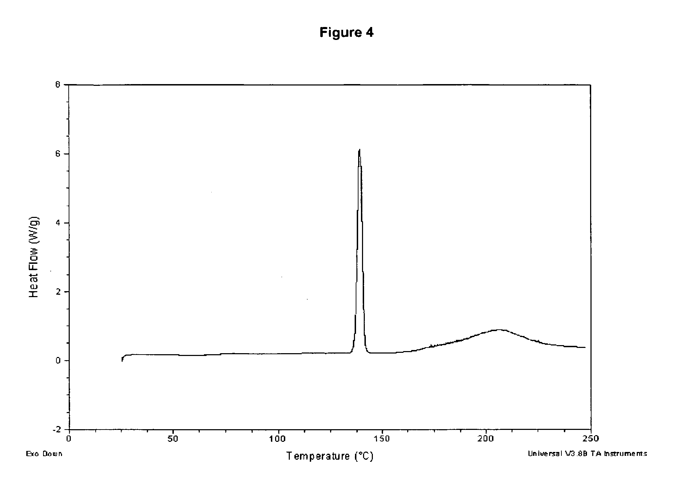

The DSC thermogram plots the differential rate of heating in watts per second against temperature. A DSC thermogram of *N*-[2-((2*R*)-2-{[4-[(4-Chlorophenyl)methyl]-1-oxo-2(1*H*)-phthalazinyl]methyl}-1-pyrrolidinyl)ethyl]-4-(methyloxy)butanamide tartrate salt (as prepared in Example 34d) is shown in Figure 4. A melting endotherm was observed at an onset temperature of 137 °C.

### Example 35

### N-[2-((2R)-2-{[4-[(4-Chlorophenyl)methyl]-1-oxo-2(1H)-phthalazinyl]methyl}-1-pyrrolidinyl)ethyl]-3-(methyloxy)propanamide, formate salt

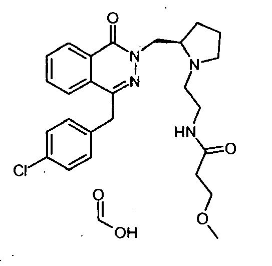

2-{[(2*R*)-1-(2-Aminoethyl)-2-pyrrolidinyl]methyl}-4-[(4-chlorophenyl)methyl]-1(2*H*)-phthalazinone (for example, as prepared for Intermediate 19) (35 mg, 0.088 mmol), TBTU (37 mg, 0.12 mmol), triethylamine (70 µl, 0.50 mmol) and 4-(methyloxy)propanoic acid (commercially available, for example, from Aldrich) (9 µl, 0.1 mmol) were stirred together in DMF (2 ml) at room temperature for 2 h. The reaction mixture was applied to a SCX cartridge (10 g, pre-washed with MeOH). The cartridge was washed with MeOH and then eluted with 10% 0.880 ammonia in MeOH. The appropriate basic fractions were combined and the solvent removed *in vacuo.* The residue was purified by MDAP HPLC and the appropriate fractions combined. The solvent was removed *in vacuo* and the residue was re-dissolved in DCM and treated with excess formic acid to ensure isolation of the formate salt of the product. The solvent and excess formic acid were removed using a stream of nitrogen to give the *title compound* (27 mg, 58%). LCMS RT = 2.48 min, ES+ve *m*/*z* 483/485 (M+H)⁺.

### Example 36

### N-[2-((2R)-2-{[4-[(4-Chlorophenyl)methyl]-1-oxo-2(1H)-phthalazinyl]methyl}-1-pyrrolidinyl)ethyl]-3,3,3-trifluoropropanamide

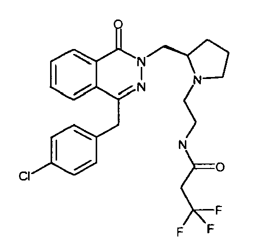

2-{[(2*R*)-1-(2-Aminoethyl)-2-pyrrolidinyl]methyl}-4-[(4-chlorophenyl)methyl]-1(2*H*)-phthalazinone (for example, as prepared for Intermediate 19) (398 mg, 1.00 mmol) was dissolved in DCM (3 ml) and treated with triethylamine (280 mg, 2.01 mmol), and the mixture was stirred at room temperature. 3,3,3-Trifluoropropionyl chloride (commercially available, for example, from Matrix Scientific) (237 µl, 1.62 mmol) was added as a solution in DCM (2 ml), and the mixture was stirred at room temperature for one hour. Further 3,3,3-trifluoropropionyl chloride (33 mg, 0.23 mmol) was added in DCM (1 ml) and stirring was continued at room temperature overnight. The reaction mixture was diluted with DCM (5 ml) and the solution was washed with water (10 ml). The layers were separated (hydrophobic frit), and the aqueous was washed with further DCM (2 x 10 ml). The combined DCM layers were concentrated *in vacuo.* The residue was purified by chromatography on silica (50 g, eluting with 2%-4% (10% saturated aqueous ammonia/MeOH) in DCM). The appropriate fractions were combined and concentrated *in vacuo* to give the *title compound* (276 mg, 54%). LCMS RT = 3.04 min, ES+ve *m*/*z* 507/509 (M+H)⁺, ¹H NMR (400 MHz, MeOH-*d*₄) δ 8.38-8.33 (m, 1H), 7.94-7.90 (m, 1H), 7.87-7.87 (m, 2H), 7.32-7.22 (m, 4H), 4.35 (s, 2 H), 4.29 (dd, *J* = 13, 4 Hz, 1H), 4.21 (dd, *J* = 13, 7 Hz, 1H), 3.44-3.35 (m, 1H), 3.32-3.25 (obscured by MeOH) (m, 1H), 3.25-3.12 (m, 3H), 3.10-2.98 (m, 2H), 2.57-2.49 (m, 1H), 2.36-2.27 (m, 1H), 1.93-1.65 (m, 4H).

### Example 36a

### N-[2-((2R)-2-{[4-[(4-Chlorophenyl)methyl]-1-oxo-2(1H)-phthalazinyl]methyl}-1-pyrrolidinyl)ethyl]-3,3,3-trifluoropropanamide, hydrochloride

*N*-[2-((2*R*)-2-{[4-[(4-Chlorophenyl)methyl]-1-oxo-2(1*H*)-phthalazinyl]methyl}-1-pyrrolidinyl)ethyl]-3,3,3-trifluoropropanamide (for example, as prepared for Intermediate Example 36) (276 mg) was dissolved in MeOH (5 ml) and treated with a solution of HCl in MeOH (1.25 mmol, 3 ml). The solvent and excess acid were then removed using a stream of nitrogen, and the residue was dried *in vacuo* to give the *title compound* as a yellow foam (281 mg, 95%). LCMS RT = 2.84 min, ES+ve *m*/*z* 507/509 (M+H)⁺, ¹H NMR (400 MHz, MeOH-*d*₄) δ 8.69 (br t, *J* = 5.0 Hz, 1H), 8.42-8.36 (m, 1H), 8.01-7.96 (m, 1H), 7.93-7.85 (m, 2H), 7.33-7.25 (m, 4H), 4.78 (dd, *J* = 15, 4 Hz, 1H), 4.58 (dd, *J* = 15, 5 Hz, 1H), 4.39 (s, 2H), 4.07-3.98 (m, 1H), 3.87-3.79 (m, 1H), 3.79-3.69 (m, 2H), 3.63-3.55 (m, 1H), 3.36-3.26 (obscured by MeOH) (m, 4H), 2.37-2.25 (m, 1H), 2.20-2.07 (m, 1H), 2.02-1.91 (m, 2H).

### Example 36b

### N-[2-((2R)-2-{[4-[(4-chlorophenyl)methyl]-1-oxo-2(1H)-phthalazinyl]methyl}-1-pyrrolidinyl)ethyl]-3,3,3-trifluoropropanamide, trifluoroacetate

2-{[(2*R*)-1-(2-Aminoethyl)-2-pyrrolidinyl]methyl}-4-[(4-chlorophenyl)methyl]-1(2*H*)-phthalazinone (for example, as prepared for Intermediate 19) (41 mg, 0.10 mmol), was dissolved in DCM (2 ml), and treated with triethylamine (43 µl, 0.31 mmol) and 3,3,3-trifluoropropionyl chloride (commercially available, for example, from Matrix Scientific) (21 µl, 0.16 mmol). The mixture was stirred at room temperature for 1 h. The reaction mixture was concentrated using a stream of nitrogen. The residue was purified by MDAP HPLC and the appropriate fractions combined. The solvent was removed *in vacuo* and the was residue re-dissolved in DCM and treated with excess trifluoroacetic acid. The solvent and excess acid were removed using a stream of nitrogen to give the *title compound* (30 mg, 48%). LCMS RT = 2.61 min, ES+ve *m*/*z* 507/509 (M+H)⁺, ¹H NMR (400 MHz, MeOH-*d*₄) δ 8.42-8.38 (m, 1H), 8.01-7.97 (m, 1H), 7.94-7.85 (m, 2H), 7.33-7.27 (m, 4H), 4.77 (dd, *J* = 15, 3 Hz, 1H), 4.57 (dd, *J* = 15, 5 Hz, 1H), 4.39 (s, 2 H), 4.06-3.98 (m, 1H), 3.88-3.78 (m, 1H), 3.78-3.67 (m, 2H), 3.61-3.54 (m, 1H), 3.36-3.24 (obscured by MeOH) (m, 4H), 2.37-2.27 (m, 1H), 2.19-2.07 (m, 1H), 2.01-1.89 (m, 2H).

### Examples 37 - 41

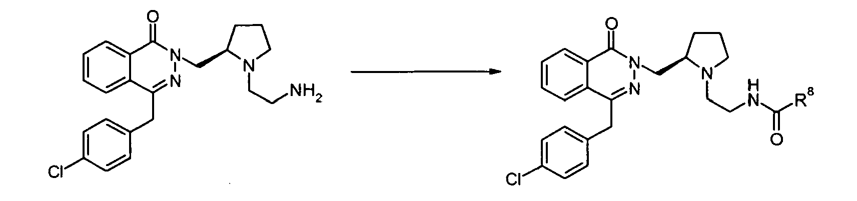

The following examples were prepared by a generally similar method to that described herein in Example 36 using 2-{[(2*R*)-1-(2-Aminoethyl)-2-pyrrolidinyl]methyl}-4-[(4-chlorophenyl)methyl]-1(2*H*)-phthalazinone (for example, as prepared for Intermediate 19) and the appropriate acid chloride.

The acid chlorides used were:
Butyryl chloride, pivaloyl chloride and isobutyryl chloride (commercially available, for example, from Aldrich)
2-Methoxyacetyl chloride (commercially available, for example, from Lancaster) 3,3,3-Trifluoropropionyl chloride (commercially available, for example, from Matrix Scientific)

| Ex No | Compound Name | Structure | Acid Chloride | LCMS RT (min) | ES+ve *(m*/*z)* (M+H)⁺ |
|---|---|---|---|---|---|
| 37 | *N*-[2-((2*R*)-2-{[4-[(4-chlorophenyl)methyl]-1-oxo-2(1*H*)-phthalazinyl]methyl}-1-pyrrolidinyl)ethyl]butanami de, trifluoroacetate | 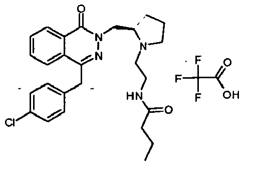 | 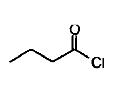 | 2.58 | 467 and 469 |
| 38 | *N*-[2-((2*R*)-2-{[4-[(4-chlorophenyl)methyl]-1-oxo-2(1*H*)-phthalazinyl]methyl}-1-pyrrolidinyl)ethyl]-2,2-dimethylpropanamide, trifluoroacetate | 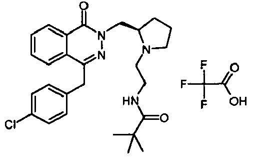 | 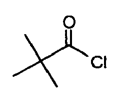 | 2.62 | 481 and 483 |
| 39 | *N*-[2-((2*R*)-2-{[4-[(4-chlorophenyl)methyl]-1-oxo-2(1*H*)-phthalazinyl]methyl}-1-pyrrolidinyl)ethyl]-2-(methyloxy)acetamide, trifluoroacetate | 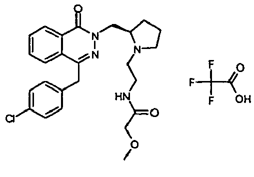 | 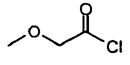 | 2.43 | 469 and 471 |
| 40 | *N*-[2-((2*R*)*-*2-{[4-[(4-Chlorophenyl)methyl]-1-oxo-2(1*H*)-phthalazinyl]methyl}-1-pyrrolidinyl)ethyl]propanam ide, trifluoroacetate salt | 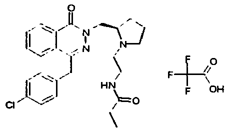 | 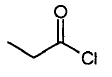 | 2.50 | 453 and 455 |
| 41 | *N*-[2-((2*R*)-2-{[4-[(4-chlorophenyl)methyl]-1-oxo-2(1*H*)-phthalazinyl]methyl}-1-pyrrolidinyl)ethyl]-2-methylpropanamide, trifluoroacetate | 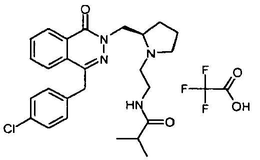 | 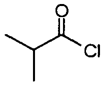 | 2.56 | 467 and 469 |

### Example 42

### N-[3-((2R)-2-{[4-[(4-Chlorophenyl)methyl]-1-oxo-2(1H)-phthalazinyl]methyl}-1-pyrrolidinyl)propyl]-3-(methyloxy)propanamide, trifluoroacetate

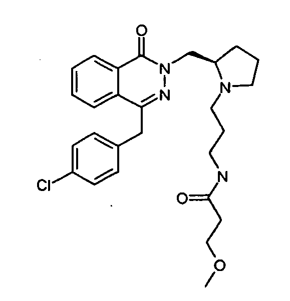

3-(Methyloxy)propanoic acid (commercially available, for example, from Aldrich) (10 µl, 0.11 mmol), TBTU (41 mg, 0.13 mmol), and triethylamine (78 µl, 0.56 mmol) were stirred together in DMF (2 ml) under nitrogen at room temperature for 10 min. A solution of 2-{[(2*R*)-1-(3-aminopropyl)-2-pyrrolidinyl]methyl}-4-[(4-chlorophenyl)methyl]-1(2*H*)-phthalazinone (for example, as prepared for Intermediate 21), (41 mg, 0.01 mmol) in DMF (1.5 ml) was added and the mixture was stirred at room temperature for 2 h. The reaction mixture was applied to a SCX cartridge (20 g, pre-washed with MeOH). The cartridge was washed with MeOH and then eluted with 10% 0.880 ammonia in MeOH. The appropriate basic fractions were combined and the solvent removed *in* vacuo. The residue was purified by MDAP HPLC and the appropriate fractions combined. The solvent was removed *in vacuo* and the residue re-dissolved in DCM and treated with excess TFA acid to ensure isolation of the salt of the product. The solvent and excess TFA were removed using a stream of nitrogen to give the *title compound* (39 mg, 79%). LCMS RT = 2.78 min, ES+ve *m*/*z* 497/499.

### Example 43

### N-[3-((2R)-2-{[4-[(4-Chlorophenyl)methyl]-1-oxo-2(1H)-phthalazinyl]methyl}-1-pyrrolidinyl)propyl]acetamide, trifluoroacetate

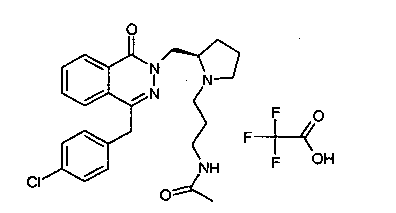

2-{[(2*R*)-1-(3-Aminopropyl)-2-pyrrolidinyl]methyl}-4-[(4-chlorophenyl)methyl]-1(2*H*)-phthalazinone (for example, as prepared for Intermediate 21) (42 mg, 0.10 mmol) was dissolved in DCM (2 ml) and treated with triethylamine (42 µl, 0.30 mmol) and acetyl chloride (commercially available, for example, from Fluka) (17 µl, 0.24 mmol). The mixture was stirred at room temperature overnight. The reaction mixture was concentrated and the residue was purified by MDAP HPLC. The appropriate fractions were combined and concentrated *in vacuo.* The residue was dissolved in MeOH and treated with excess TFA acid. The solvent and excess acid were removed using a stream of nitrogen to give the *title compound* (41 mg, 72%). LCMS RT = 2.44 min, ES+ve *mlz* 453/455 (M+H)⁺.

### Examples 44 - 46

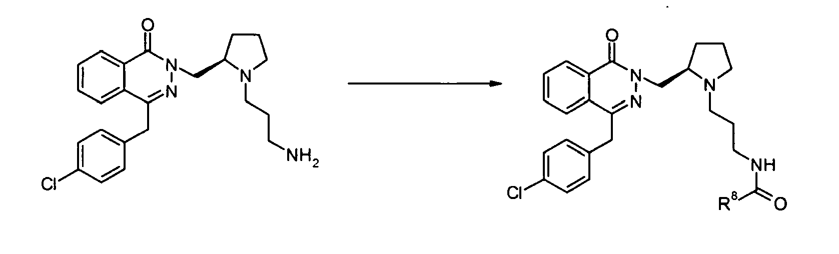

The following examples were prepared by a generally similar method to that described herein in Example 43 using 2-{[(2*R*)-1-(3-aminopropyl)-2-pyrrolidinyl]methyl}-4-[(4-chlorophenyl)methyl]-1(2*H*)-phthalazinone (for example, as prepared for Intermediate 21) and the appropriate acid chloride.

The acid chlorides used were:
Propionyl chloride, isobutyryl chloride and pivaloyl chloride( commercially available, for example, from Aldrich).

| Ex No | Compound Name | Structure | Acid Chloride | LCMS RT (min) | ES+ve *(m*/*z)* (M+H)⁺ |
|---|---|---|---|---|---|
| 44 | *N*-[3-((2*R*)-2-{[4-[(4-Chlorophenyl)methyl]-1-oxo-2(1*H*)-phthalazinyl]methyl}-1-pyrrolidinyl)propyl]propan amide, trifluoroacetate | 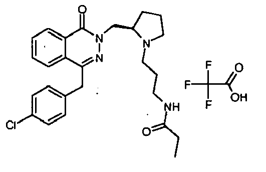 | 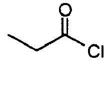 | 2.44 | 467 and 469 |
| 45 | *N*-[3-((2*R*)-2-{[4-[(4-Chlorophenyl)methyl]-1-oxo-2(1*H*)-phthalazinyl]methyl}-1-pyrrolidinyl)propyl]-2-methylpropanamide, trifluoroacetate | 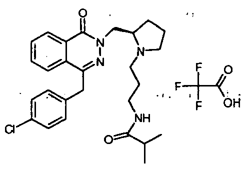 | 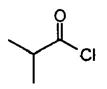 | 2.59 | 481 and 483 |
| 46 | *N*-[3-((2*R*)-2-{[4-[(4-Chlorophenyl)methyl]-1-oxo-2(1*H*)-phthalazinyl]methyl}-1-pyrrolidinyl)propyl]-2,2-dimethylpropanamide, trifluoroacetate | 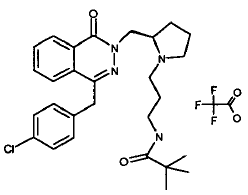 | 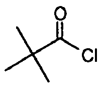 | 2.69 | 495 and 497 |

### Example 47

### N-[2-((2R)-2-{[4-[(4-Chlorophenyl)methyl]-1-oxo-2(1H)-phthalazinyl]methyl}-1-pyrrolidinyl)ethyl]-2,2,2-trifluoroacetamide

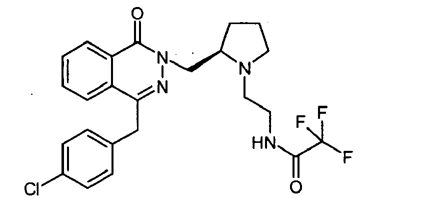

4-[(4-Chlorophenyl)methyl]-2-[(2*R*)-2-pyrrolidinylmethyl]-1(2*H*)-phthalazinone (for example, as prepared for Intermediate 2) (354 mg, 1.0 mmol) was dissolved in butanone (8 ml). 2,2,2-Trifluoro-*N*-(2-iodoethyl)acetamide (commercially available, for example, from Apollo) (323 mg, 1.2 mmol) was added as a solution in butanone (2 ml), followed by DIPEA (209 µl, 1.2 mmol). The mixture was heated to 80 °C and stirred under a nitrogen atmosphere overnight. Further DIPEA (87 µl, 0.5 mmol) and 2,2,2-trifluoro-*N*-(2-iodoethyl)acetamide (139 mg, 0.52 mmol) were added and the reaction stirred while heating at 80°C under nitrogen for 4.5 h. Further DIPEA (87 µl, 0.5 mmol) and 2,2,2-trifluoro-*N*-(2-iodoethyl)acetamide (132 mg, 0.49 mmol) were added and the reaction stirred while heating at 80 °C under nitrogen overnight. The reaction mixture was concentrated *in vacuo.* Purification on silica (50 g, eluting with 0-100% EtOAc-cyclohexane over 20 min) gave the *title compound* as a yellow gum (498 mg, 100%); LCMS RT = 3.05 min, ES+ve *m*/*z* 493/495 (M+H)⁺.

### Example 48

### N-[2-((2R)-2-{[4-[(4-Chlorophenyl)methyl]-1-oxo-2(1H)-phthalazinyl]methyl}-1-pyrrolidinyl)ethyl]-2,2,2-trifluoro-N-methylacetamide

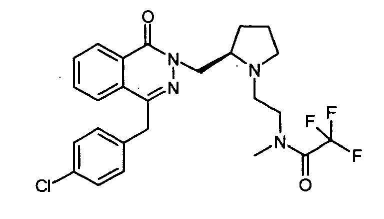

*N*-[2-((2*R*)-2-{[4-[(4-Chlorophenyl)methyl]-1-oxo-2(1*H*)-phthalazinyl]methyl}-1-pyrrolidinyl)ethyl]-2,2,2-trifluoroacetamide (for example, as prepared for Example 47) (155 mg, 0.31 mmol) was stirred in DMF (5 ml) with potassium carbonate (85 mg, 0.31 mg) for 5 min at room temperature. Methyl iodide (commercially available, for example, from Aldrich)(39 µl, 0.63 mmol) was added and the reaction was stirred at room temperature for 6 h and then left to stand. The mixture was applied to a C-18 cartridge (50 g) (pre-washed with MeOH followed by 5% MeOH-water). The cartridge was eluted with 5% MeOH-water followed by MeOH. The appropriate fractions were combined and concentrated *in vacuo* to give the *title compound* as a yellow gum (53 mg, impure); LCMS major component RT = 3.13 min, ES+ve *m*/*z* 507/509 (M+H)⁺.

### Example 49

### N-[2-((2R)-2-{[4-[(4-Chlorophenyl)methyl]-1-oxo-2(1H)-phthalazinyl]methyl}-1-pyrrolidinyl)ethyl]-N-methyl-4-(methyloxy)butanamide, trifluoroacetate

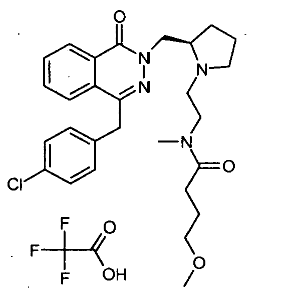

4-(Methyloxy)butanoic acid (for example, as prepared for Intermediate 17) (5 mg, 0.036 mmol) was stirred in DMF (0.5 ml) with TBTU (24 mg, 0.074 mmol) and triethylamine (17 µl, 0.12 mmol) for 15 min at room temperature. This mixture was added to a solution of 4-[(4-chlorophenyl)methyl]-2-({(2*R*)-1-[2-(methylamino)ethyl]-2-pyrrolidinyl}methyl)-1(2*H*)-phthalazinone (for example, as prepared for Intermediate 22) in DMF (0.5 ml). The reaction mixture was stirred at room temperature for 3 h, and then concentrated under a stream of nitrogen. The residue was applied to an SCX-2 cartridge (5 g), washing with MeOH and eluting with 10% aqueous 0.880 ammonia in MeOH. The appropriate basic fractions were combined and the solvent removed *in vacuo.* Purification by MDAP HPLC gave the *title compound* as the formate salt. This was re-dissolved in MeOH and treated with TFA (0.5 ml, excess). The solvent and excess acid were removed using a stream of nitrogen to give *the title compound* (11 mg, 59%): LCMS RT = 2.76 min, ES+ve *m*/*z* 511/513 (M+H)⁺, ¹H NMR (400 MHz, CD₃OD) δ 8.41 (1H, d, *J* = 8 Hz), 8.01-7.96 (1H, m), 7.94-7.84 (2H, m), 7.33-7.22 (4H, m), 4.78 (1H, dd, *J* = 15, 2 Hz), 4.57 (1H, dd, *J* = 15, 5 Hz), 4.38 (2H, s), 4.19-4.10 (1H, m), 4.05-3.96 (1H, m), 3.96-3.87 (1H, m), 3.82-3.72 (1H, m), 3.46-3.24 (m, partially obscured by CD₃OD), 3.12 (3H, s), 2.67-2.45 (2H, m), 2.36-2.27 (1H, m), 2.19-2.07 (1H, m), 2.00-1.88 (2H, m), 1.83-1.74 (2H, m).

### Example 50

### N-[2-((2R)-2-{[4-[(4-Chlorophenyl)methyl]-1-oxo-2(1H)-phthalazinyl]methyl}-1-pyrrolidinyl)ethyl]cyclopentanecarboxamide, trifluoroacetate

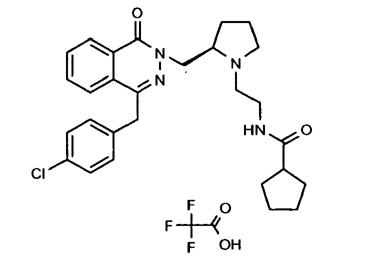

To a solution of 2-{[(2*R*)-1-(2-aminoethyl)-2-pyrrolidinyl]methyl}-4-[(4-chlorophenyl)methyl]-1(2*H*)-phthalazinone (for example, as prepared for Intermediate 19) (0.048 g, 0.12 mmol) in DMF (2 ml) was added cyclopentanecarboxylic acid (commercially available, for example, from Aldrich) (0.028 ml, 0.18 mmol). To the slight suspension was added triethylamine (0.1 ml, 0.7 mmol) and then TBTU (63 mg, 0.20mmol). The resulting solution was stirred at ambient temperature overnight. The crude was applied to a 10g SCX-2 cartridge (pre-washed with MeOH) and the cartridge washed with MeOH (2 column volumes). The cartridge was eluted with 10% aqueous 0.880 ammonia in MeOH (2 column volumes) and the basic fractions concentrated *in vacuo* to leave a yellow gum. The residue was purified by MDAP HPLC and the appropriate fractions combined. The combined fractions were treated with TFA (0.5 ml) and the solvent removed *in vacuo* to give the *title compound* (42 mg, 57%). LCMS RT = 2.93 min, ES+ve *mlz* 493/495 (M+H)⁺. ¹H NMR CD₃OD, (CD₃OD) δ 8.39 (1H, m), 7.98 (1H, m), 7.88 (2H, m), 7.30 (4H, m), 4.64 (2H, m), 4.49 (2H, s), 4.07 (1H, m), 3.85 (1H, m), 3.65-3.35 (2H, m), 3.52-3.44 (1H, m), 3.35-3.23 (2H, m, obscured by solvent), 2.65-2.59 (1H, m), 2.38-2.27 (1H, m), 2.20-2.10 (1H, m), 2.05-1.93 (2H, m), 1.85-1.70 (2H, m), 1.65-1.50 (6H, m).

### Examples 51 - 55

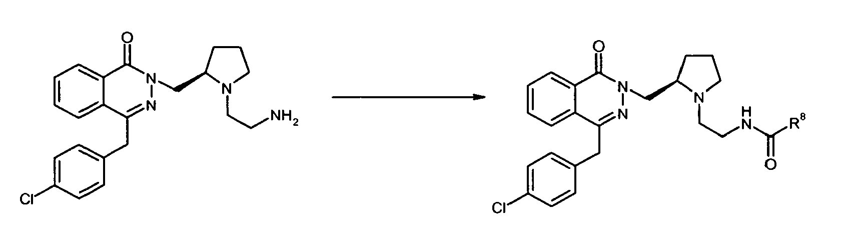

The following Examples were prepared by a generally similar method to that described herein in Example 50 using 2-{[(2*R*)-1-(2-aminoethyl)-2-pyrrolidinyl]methyl}-4-[(4-chlorophenyl)methyl]-1(2*H*)-phthalazinone (for example, as prepared for Intermediate 19) and the appropriate carboxylic acid.

The following carboxylic acids were used:
tetrahydro-2*H*-pyran-4-carboxylic acid (commercially available, for example, from Maybridge);
cyclopentanecarboxylic acid, cyclohexanecarboxylic acid and tetrahydro-3-furoic acid (commercially available, for example, from Aldrich);
3-hydroxy-2,2-dimethylpropanoic acid (commercially available, for example, from TCI-EP); and
Tetrahydro-pyran-3-carboxylic acid (commercially available, for example, from J & W Pharmalab)

| Ex No | Compound Name | Structure | Acid | LCMS RT (min) | ES+ve *m*/*z* (M+H)+ |
|---|---|---|---|---|---|
| 51 | *N*-[2-((2*R*)-2-{[4-[(4-chlorophenyl)methyl]-1-oxo-2(1*H*)-phthalazinyl]methyl}-1-pyrrolidinyl)ethyl]tetrahydro -2*H*-pyran-4-carboxamide, trifluoroacetate | 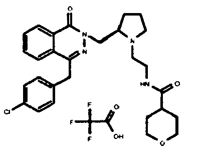 | 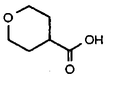 | 2.79 | 509 and 511 |
| 52 | *N*-[2-((2*R*)-2-{[4-[(4-chlorophenyl)methyl]-1-oxo-2(1*H*)-phthalazinyl]methyl}-1- | 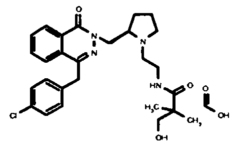 | 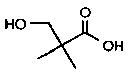 | 2.79 | 497 and 499 |
| | pyrrolidinyl)ethyl]-3-hydroxy-2,2-dimethylpropanamide, formic acid (1:1) | | | | |
| 53 | *N*-[2-((2*R*)-2-{[4-[(4-chlorophenyl)methyl]-1-oxo-2(1*H*)-phthalazinyl]methyl}-1-pyrrolidinyl)ethyl]tetrahydro -2*H*-pyran-3-carboxamide, hydrochloride | 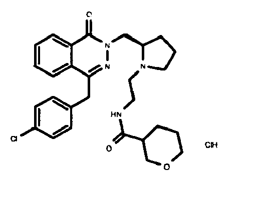 | 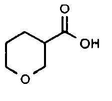 | 2.77 | 509 and 511 |
| 54 | *N*-[2-((2*R*)-2-{[4-[(4-chlorophenyl)methyl]-1-oxo-2(1*H*)-phthalazinyl]methyl}-1-pyrrolidinyl)ethyl]tetrahydro -3-furancarboxamide, hydrochloride | 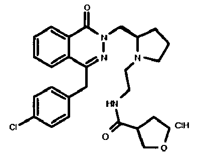 | 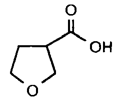 | 2.72 | 495 and 497 |
| 55 | *N*-[2-((2*R*)-2-{[4-[(4-chlorophenyl)methyl]-1-oxo-2(1*H*)-phthalazinyl]methyl}-1-pyrrolidinyl)ethyl]cyclohexa necarboxamide, hydrochloride | 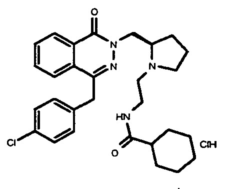 | 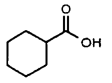 | 3.14 | 507 |

### Example 56

### 2-((2R)-2-{[4-[(4-Chlorophenyl)methyl]-1-oxo-2(1H)-phthalazinyl]methyl}-1-pyrrolidinyl)-N-(2,2-dimethylpropyl)acetamide, trifluoroacetate

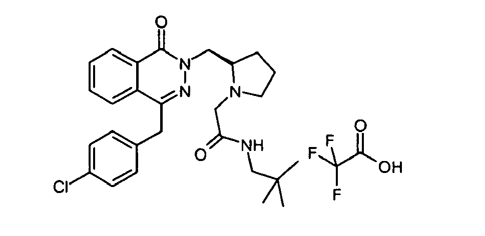

To a solution of ((2*R*)-2-{[4-[(4-chlorophenyl)methyl]-1-oxo-2(1*H*) phthalazinyl]methyl}-1-pyrrolidinyl)acetic acid, trifluoroacetate, (for example, as prepared for Intermediate 12) (0.040 g, 0.076 mmol) in dry DMF (1 ml) was added TBTU (0.045 g, 0.14 mmol) and triethylamine (0.042 ml, 0.30 mmol). The reaction mixture was stirred at room temperature for 15 min before adding neopenylamine (commercially available, for example, from Fluorochem) (0.010 ml, 0.08 mmol). The resultant dark yellow solution was allowed to stand at room temperature for 3 d and then applied onto a pre-conditioned (MeOH) SCX-2 cartridge (10 g), washed with MeOH (× 3) and then eluted with 10% aqueous 0.88 ammonia in MeOH (x 4). The solvents were removed *in vacuo* and the residue purified by MDAP HPLC and treated with TFA to afford the *title compound* (17 mg). LCMS RT = 3.14 min, ES+ve *m*/*z* 481 and 483 [M+H]⁺

### Example 57

### 2-((2R)-2-{[4-[(4-chlorophenyl)methyl]-1-oxo-2(1H)-phthalazinyl]methyl}-1-pyrrolidinyl)-N-(cyclohexylmethyl)acetamide, trifluoroacetate

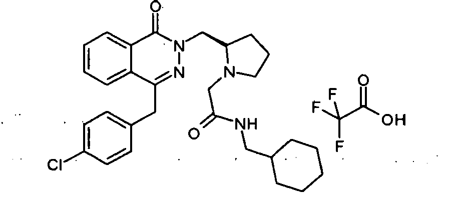

Prepared by a generally similar method to that described herein in Example 56 using ((2*R*)-2-{[4-[(4-chlorophenyl)methyl]-1-oxo-2(1*H*)-phthalazinyl]methyl}-1-pyrrolidinyl)acetic acid trifluoroacetate (for example, as prepared for Intermediate 12) and cyclohexylmethylamine (commercially available, for example, from Aldrich) to give the *title compound* LCMS RT = 3.34 min, ES+ve *mlz* 507/509 (M+H)⁺.

### Example 58

### 4-[(4-Chlorophenyl)methyl]-2-({(2R)-1-[(2-oxo-3-piperidinyl)methyl]-2-pyrrolidinyl}methyl)-1(2H)-phthalazinone

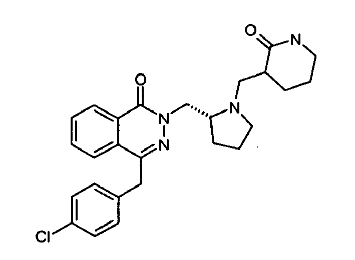

A mixture of (2-oxo-3-piperidinyl)methyl methanesulfonate (for example, as prepared for Intermediate 35)(35 mg, 0.17 mmol), 4-[(4-chlorophenyl)methyl]-2-[(2*R*)2-pyrrolidinylmethyl]-1(2*H*)-phthalazinone (for example, as prepared for Intermediate 2) (42 mg, 0.12 mmol), sodium iodide (27 mg, 0.18 mmol), sodium bicarbonate (72 mg, 0.86 mmol) in DMF (1 ml) was heated in a Smith Creator^{™} microwave oven at 150 °C for 15 min and then for a second 15 min period. The reaction did not progress any further and the elimination product 3-methylidene-2-piperidinone started to form. Further quantities of (2-oxo-3-piperidinyl)methyl methanesulfonate (14 mg) and DMF (0.5 ml) were added and the mixture was heated in a Smith Creator^{™} microwave oven at 150 °C for 15 min. A second additional portion of (2-oxo-3-piperidinyl)methyl methanesulfonate (14 mg) and DMF (0.5 ml) were added and the mixture was heated in a Smith Creator^{™} microwave oven at 150 °C for 15 min. LCMS indicated complete conversion of the (2-oxo-3-piperidinyl)methyl methanesulfonate to 3-methylidene-2-piperidinone. The reaction mixture was concentrated *in vacuo* and the residue transferred to a round-bottom flask, sodium bicarbonate (30 mg) added, followed by DMF (0.2 ml) and the reaction was heated conventionally at 150 °C overnight under nitrogen. The reaction mixture was applied on a 20 g SCX-2 cartridge (pre-conditioned with MeOH) and washed with MeOH. The product was eluted with 10% aqueous 0.880 ammonia in MeOH. The basic fractions were combined and evaporated under reduced pressure. The residue was purified by MDAP HPLC and was further purified by chromatography on a 5 g silica cartridge eluting with DCM-EtOH-aqueous 0.880 ammonia (80:8:1) to give the title compound (2.76 mg) LCMS RT = 2.80 min, ES+ve *m*/*z* 465/467 (M+H)⁺.

### Example 59

### 4-[(4-Chlorophenyl)methyl]-2-{[(2R)-1-(2-{3-[2-(methyloxy)ethyl]-1,2,4-oxadiazol-5-yl}ethyl)-2-pyrrolidinyl]methyl}-1(2H)-phthalazinone, trifluoroacetate

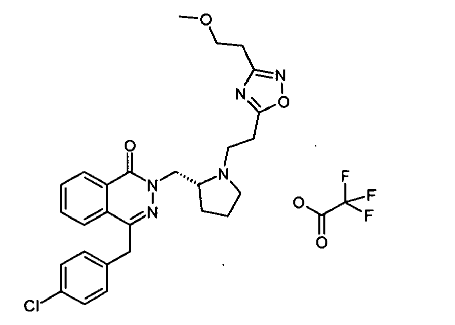

Oxalyl chloride (commercially available, for example, from Aldrich)(0.022 ml, 0.25 mmol) was added to a solution of dry DMF (0.030 ml, 0.38 mmol) in dry DCM (0.5 ml). After 5 min a solution of 3-((2*R*)-2-{[4-[(4-chlorophenyl)methyl]-1-oxo-2(1*H*)-phthalazinyl]methyl}-1-pyrrolidinyl)propanoic acid as the triethylamine salt (for example, as prepared for Intermediate 4) (103 mg, 0.242 mmol) in dry DCM (1 ml) was added and the mixture was stirred for a further 5 min. *N*-Hydroxy-3-(methyloxy)propanimidamide (commercially available, for example, from Fulcrum Scientific and/or Tyger Scientific) (0.053 g, 0.45 mmol) was added and the mixture heated to reflux. After 1.5 h toluene (3 ml) was added and refluxing was continued overnight. The reaction mixture was allowed to cool to room temperature and then poured onto a pre-conditioned (MeOH) amino-propyl SPE cartridge (20 g). The cartridge was washed with MeOH (× 4) and these fractions were combined and concentrated *in vacuo.* The resultant residue was purified by MDAP HPLC, treated with TFA (about 0.5 ml) and concentrated *in vacuo* to afford the *title compound* (0.016 g). LCMS RT = 2.57 min, ES+ve *m*/*z* 508/510 (M+H)⁺

### Example 60

### 4-[(4-Chlorophenyl)methyl]-2-({(2R)-1-[(3-propyl-1,2,4-oxadiazol-5-yl)methyl]-2-pyrrolidinyl}methyl)-1(2H)-phthalazinone, hydrochloride

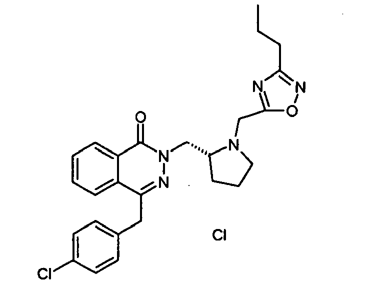

((2*R*)-2-{[4-[(4-Chlorophenyl)methyl]-1-oxo-2(1*H*)-phthalazinyl]methyl}-1-pyrrolidinyl)acetic acid, trifluoroacetate (for example, as prepared for Intermediate 12) (50 mg, 0.095 mmol), TBTU (49 mg, 0.15 mmol) and triethylamine (53 µl, 0.38 mmol) were stirred together in DMF (1 ml) at room temperature for 10 min. *N*-Hydroxybutanimidamide (commercially available, for example, from Alfa Aesar, Apollo, Maybridge, Fulcrum Scientific and/or Tyger Scientific) (14 mg, 0.14 mmol) was added as a solution in DMF (0.5 ml) and the mixture was stirred at room temperature for 30 min. The mixture was transferred to a microwave vial, adding further DMF (0.5 ml), and the sealed vial was heated to 80°C for 5 min in a Smith Creator^{™} microwave oven. Further triethylamine (66 µl, 0.47 mmol) was added and the re-sealed vial was heated to 80 °C for 5 min in a Smith Creator^{™} microwave oven, then left to stand at room temperature overnight. Further triethylamine (66 µl, 0.47 mmol) was added and the re-sealed vial was heated to 90°C for 10 min, then 10 min further, in a Smith Creator^{™} microwave oven, then left to stand at room temperature. The reaction mixture was applied to an SCX-2 cartridge (20 g, pre-washed with MeOH). The cartridge was washed with MeOH and then eluted with 10% aqueous 0.880 ammonia in MeOH. The appropriate basic fractions were combined and the solvent removed *in vacuo.* The residue was purified by MDAP HPLC and the appropriate fractions were combined and concentrated to give the *title compound* as the partial formate salt (3 mg). The residue was re-dissolved in MeOH and treated with a solution of HCl in MeOH (1.25M, 0.2 ml, excess). The solvent and excess acid were removed using a stream of nitrogen to give *the title compound* (3.5 mg, 7%). LCMS RT = 3.64 min, ES+ve *m*/*z* 478/480 (M+H)⁺.

### Example 61

### 4-[(4-Chlorophenyl)methyl]-2-[((2R)-1-{2-[3-(1-methylethyl)-1,2,4-oxadiazol-5-yl]ethyl}-2-pyrrolidinyl)methyl]-1(2H)-phthalazinone, hydrochloride

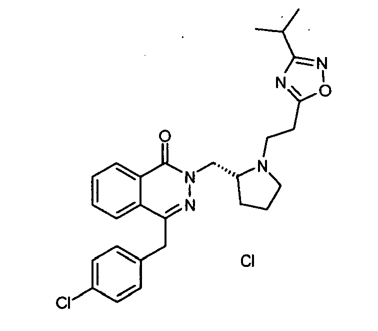

3-((2*R*)-2-{[4-[(4-Chlorophenyl)methyl]-1-oxo-2(1*H*)-phthalazinyl]methyl}-1-pyrrolidinyl)propanoic acid (as the triethylamine salt) (for example, as prepared for Intermediate 4) (45 mg, 0.085 mmol), TBTU (32 mg, 0.10 mmol) and triethylamine (28 µl, 0.20 mmol) were stirred together in DMF (1.5 ml) at room temperature for 20 min. *N-*Hydroxy-2-methylpropanimidamide (commercially available, for example, from Alfa Aesar) (10 mg, 0.10 mmol) was added and the mixture was stirred at room temperature for 30 min and left to stand at room temperature overnight. The mixture was transferred to a microwave vial, adding further DMF (0.5 ml) and pyridine (8 µl, 0.10 mmol), and the sealed vial was heated to 80 °C for 2 min in a Smith Creator^{™} microwave oven. Further pyridine (100 µl, excess), was added and the re-sealed vial was heated to 80 °C for 5 min, then 5 min further, then to 90 °C for 5 min in a Smith Creator^{™} microwave oven, then left to stand at room temperature. Triethylamine (66 µl, 0.47 mmol) was added and the re-sealed vial was heated to 80°C for 5 min in a Smith Creator^{™} microwave oven, then left to stand at room temperature overnight. Further triethylamine (35 µl, 0.25 mmol) was added and the re-sealed vial was heated to 85 °C for 1 h. Further triethylamine (35 µl, 0.25 mmol) was added and the re-sealed vial was heated to 85 °C for 2 h. Further triethylamine (35 µl, 0.25 mmol) was added and the re-sealed vial was heated to 85 °C for 2 h, then left to stand at room temperature. The reaction mixture was applied to an SCX-2 cartridge (20 g, pre-washed with MeOH). The cartridge was washed with MeOH and then eluted with 10% aqueous 0.880 ammonia in MeOH. The appropriate basic fractions were combined and the solvent removed *in vacuo.* The residue was purified by MDAP HPLC and the appropriate fractions were combined and concentrated to give the *title compound* as the free base (7 mg). The residue was re-dissolved in MeOH and treated with a solution of HCl in MeOH (1.25M, 0.1 ml, excess). The solvent and excess acid were removed using a stream of nitrogen to give the *title compound* (7 mg, 16%). LCMS RT = 3.08 min, ES+ve *m*/*z* 492/494 (M+H)⁺.

### Example 62

### 4-[(4-Chlorophenyl)methyl]-2-({(2R)-1-[2-(3-propyl-1,2,4-oxadiazol-5-yl)ethyl]-2-pyrrolidinyl}methyl)-1(2H)-phthalazinone, hydrochloride

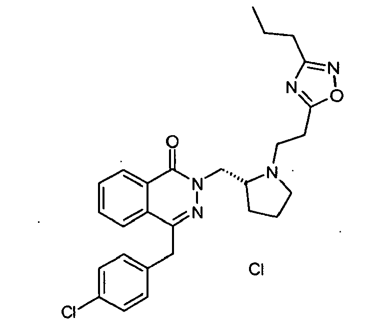

3-((2*R*)-2-{[4-[(4-Chlorophenyl)methyl]-1-oxo-2(1*H*)-phthalazinyl]methyl}-1-pyrrolidinyl)propanoic acid as the triethylamine salt (for example, as prepared for Intermediate 4) (53 mg, 0.10 mmol), TBTU (49 mg, 0.15 mmol) and triethylamine (42 µl, 0.30 mmol) were stirred together in DMF (1 ml) at room temperature for 5 min. *N-*Hydroxybutanimidamide (commercially available, for example, from Alfa Aesar, Apollo, Maybridge Fulcrum Scientific and/or Tyger Scientific) (10 mg, 0.10 mmol) was added as a solution in DMF (0.5 ml) and the mixture was stirred at room temperature for 2 h. The mixture was transferred to a microwave vial, adding further DMF (0.5 ml) and triethylamine (42 µl, 0.30 mmol), and the sealed vial was heated to 80 °C for 15 min in a Smith Creator^{™} microwave oven. Further triethylamine (42 µl, 0.30 mmol), was added and the re-sealed vial was heated to 80 °C for 30 min. This process of adding further triethylamine and heating at 80 °C or 85 °C in a Smith Creator^{™} microwave oven was repeated several times (30 min, 45 min, 30 min, 1 h, 1 h, 2 h, 1 hr, 1 h, totalling 8 h heating), with the reaction being left to stand at room temperature in between, and the progress being judged by LCMS analysis. The reaction mixture was applied to an SCX-2 cartridge (20 g, pre-washed with MeOH). The cartridge was washed with MeOH and then eluted with 10% aqueous 0.880 ammonia in MeOH. The appropriate basic fractions were combined and the solvent removed *in vacuo.* The residue was purified by MDAP HPLC and the appropriate fractions were combined and concentrated to give the *title compound* as the free base (12 mg). The residue was re-dissolved in MeOH and treated with a solution of HCl in MeOH (1.25 M, excess). The solvent and excess acid were removed using a stream of nitrogen to give the *title compound* (13 mg, 25%). LCMS RT = 3.19 min, ES+ve *m*/*z* 492/494 (M+H)⁺.

### Example 63

### 3-((2R)-2-{[4-[(4-Chlorophenyl)methyl]-1-oxopyrido[3,4-d]pyridazin-2(1H)-yl]methyl}-1-pyrrolidinyl)-N-[3-(methyloxy)propyl]propanamide, trifluoroacetate

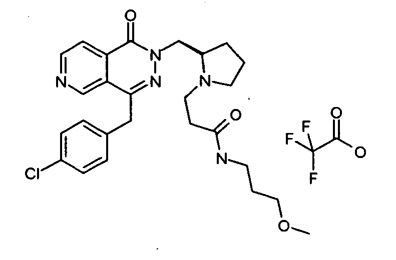

To a solution of 3-((2*R*)-2-{[4-[(4-chlorophenyl)methyl]-1-oxopyrido[3,4-*d*]pyridazin-2(1*H*)-yl]methyl}-1-pyrrolidinyl)propanoic acid, partial triethylamine salt (1:1) (for example, as prepared for Intermediate 30) (73 mg, 0.14 mmol) in dry dimethylformamide (2 ml) was added TBTU (66 mg, 0.20 mmol). The reaction mixture was stirred for 1.5 h before adding [3-(methyloxy)propyl]amine (commercially available, for example, from Aldrich) (0.013 ml, 0.17 mmol) and triethylamine (0.038 ml, 0.51 mmol). The resultant reaction mixture was stirred at room temperature overnight and then poured onto a pre-conditioned (MeOH) SCX-2 cartridge (20 g) and washed with MeOH (3 volumes). The product was eluted with 10% aqueous 0.880 ammonia in MeOH (× 4). The appropriate fraction was concentrated *in vacuo* and the resultant residue purified by MDAP HPLC and treated with TFA (approximately 0.5 ml) to afford the *title compound* (18 mg). LCMS RT = 2.64 min, ES+ve *m*/*z* 498/500 (M+H)⁺

### Example 64

### 3-((2R)-2-{[4-[(4-Chlorophenyl)methyl]-1-oxopyrido[3,4-d]pyridazin-2(1H)-yl]methyl}-1-pyrrolidinyl)-N-(2,2-difluoroethyl)propanamide, monoformate salt

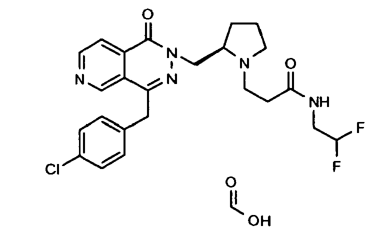

To a solution of 3-((2*R*)-2-{[4-[(4-chlorophenyl)methyl]-1-oxopyrido[3,4-*d*]pyridazin-2(1*H*)-yl]methyl}-1-pyrrolidinyl)propanoic acid, partial triethylamine salt (for example, as prepared for Intermediate 30) (0.11 g, 0.21 mmol) in DMF (1 ml) was added TBTU (0.11 g, 0.34 mmol) and then triethylamine (0.1 ml, 0.7 mmol). To the solution was added 2,2,-difluoroethylamine (commercially available, for example, from Apollo) (0.040 ml, 0.52 mmol). The solution was stirred at ambient temperature for 4 h. The reaction mixture was applied to a 20 g SCX-2 cartridge (pre-washed with MeOH) and the cartridge washed with MeOH (2 column volumes). The cartridge was eluted with 10% 0.880 ammonia in MeOH (2 column volumes) and the basic fractions concentrated *in vacuo* to leave a gum (0.178 g). The crude was further purified by MDAP HPLC and the appropriate fractions combined. The solvent was removed *in vacuo* to leave the *title compound* as a waxy solid (0.070 g, 62%). LCMS RT = 2.26 min, ES+ve *m*/*z* 490/492 (M+H)⁺.

### Examples 65 to 68

The following Examples were prepared by a generally similar method to that described herein in Example 64 using 3-((2*R*)-2-{[4-[(4-chlorophenyl)methyl]-1-oxopyrido[3,4-*d*]pyridazin-2(1*H*)-yl]methyl}-1-pyrrolidinyl)propanoic acid, partial triethylamine salt (for example, as prepared for Intermediate 30) and the appropriate amine:

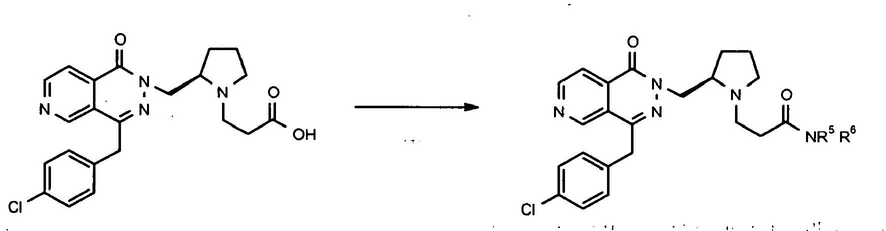

The following amines were used:
Propylamine, *N*-methyl,*N*-ethylamine, piperidine (all commercially available, for example, from Aldrich)
2,2,2-Trifluoroethylamine (commercially available, for example, from Acros)

| Ex No | Compound Name | Structure | LCMS RT (min) | ES +ve *m*/*z* (M+H)⁺ |
|---|---|---|---|---|
| 65 | 3-((2*R*)-2-{[4-[(4-Chlorophenyl)methyl] -1-oxopyrido[3,4-*d*]pyridazin-2(1*H*)-yl]methyl}-1-pyrrolidinyl)-*N*-propylpropanamide, trifluoroacetate | 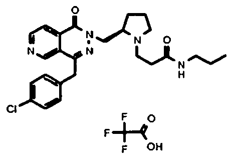 | 2.65 | 468/470 |
| 66 | 3-((2*R*)-2-{[4-[(4-Chlorophenyl)methyl] -1-oxopyrido[3,4-*d*]pyridazin-2(1*H*)-yl]methyl}-1-pyrrolidinyl)-*N*-ethyl-*N-*methylpropanamide, trifluoroacetate | 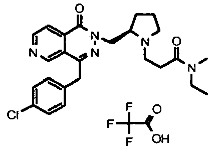 | 2.59 | 468/470 |
| 67 | 4-[(4-Chlorophenyl)methyl] -2-({(2*R*)-1-[3-oxo-3-(1-piperidinyl)propyl]-2-pyrrolidinyl}methyl)py rido[3,4-*d*]pyridazin-1(2*H*)-one, trifluoroacetate | 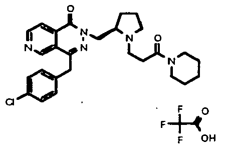 | 2.49 | 494/496 |
| 68 | 3-((2*R*)-2-{[4-[(4-Chlorophenyl)methyl] -1-oxopyrido[3,4-*d*]pyridazin-2(1*H*)-yl]methyl}-1-pyrrolidinyl)-*N*-(2,2,2-trifluoroethyl)propana mide, trifluoroacetate | 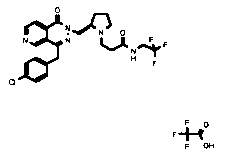 | 2.45 | 508/510 |

### Example 69

### N-[2-((2R)-2-{[4-[(4-Chlorophenyl)methyl]-1-oxopyrido[3,4-d]pyridazin-2(1H)-yl]methyl}-1-pyrrolidinyl)ethyl]-3-(methyloxy)propanamide, trifluoroacetate

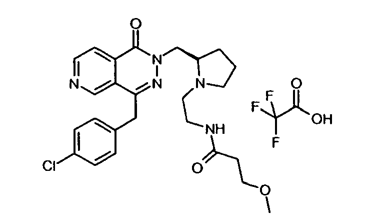

To a solution of 3-(methyloxy)propanoic acid (commercially available, for example, from Aldrich) (15.7 mg, 0.15 mmol) and TBTU (58 mg, 0.18 mmol) in dry DMF (0.5 ml), which had been stirred at room temperature for 10 min was added a solution of 2-{[(2*R*)-1-(2-aminoethyl)-2-pyrrolidinyl]methyl}-4-[(4-chlorophenyl)methyl]pyrido[3,4-*d*]pyridazin-1(2*H*)-one (for example, as prepared for Intermediate 32) (60 mg, 0.15 mmol) in dry DMF (1 ml) followed by triethylamine (63 µl, 0.45 mmol). The resultant solution was stirred at room temperature overnight. The reaction mixture was then poured onto a pre-conditioned (MeOH) SCX-2 cartridge (20 g). The cartridge was washed with MeOH (3 volumes) and eluted with 10 % aqueous 0.880 ammonia in MeOH (3 volumes). The appropriate fractions were combined and concentrated *in vacuo* and the residue purified by MDAP HPLC and treated with TFA (approximately 0.5 ml) to afford the *title compound* (51 mg). LCMS RT = 2.33 min, ES+ve *m*/*z* 484/486 (M+H)⁺.

### Example 70

### N-[2-((2R)-2-{[4-[(4-chlorophenyl)methyl]-1-oxopyrido[3,4-d]pyridazin-2(1H)-yl]methyl}-1-pyrrolidinyl)ethyl]-4-(methyloxy)butanamide, trifluoroacetate

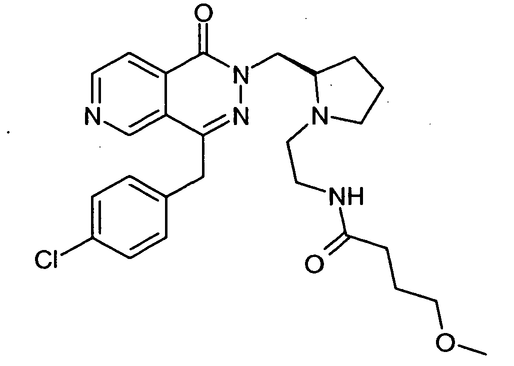

Prepared by a generally similar method to Example 69, using 2-{[(2*R*)-1-(2-aminoethyl)-2-pyrrolidinyl]methyl}-4-[(4-chlorophenyl)methyl]pyrido[3,4-*d*]pyridazin-1(2*H*)-one (for example, as prepared for Intermediate 32) and 4-(methyloxy)butanoic acid (for example, as prepared for Intermediate 17). LCMS RT = 2.62 min, ES+ve *m*/*z* 498/500 (M+H)⁺_{.}

### Examples 71 and 72

Prepared by a generally similar method to that described herein in Example 69, using 2-{[(2*R*)-1-(3-aminopropyl)-2-pyrrolidinyl]methyl}-4-[(4-chlorophenyl)methyl]pyrido[3,4-*d*]pyridazin-1(2*H*)-one (for example, as prepared for Intermediate 34) and the appropriate acids. The acids used were 3-(methyloxy)propanoic acid (commercially available, for example, from Aldrich) and 4-(methyloxy)butanoic acid (for example, as prepared for Intermediate 17).

| Ex No | Compound Name | Structure | Acid | LCM S RT (min) | ES+ve *m*/*z* (M+H)⁺ |
|---|---|---|---|---|---|
| 71 | *N*-[3-((2*R*)-2-{[4-[(4-chlorophenyl)methyl]-1-oxopyrido[3,4-*d*]pyridazin-2(1*H*)-yl]methyl}-1-pyrrolidinyl)propyl]-3-(methyloxy)propanamide, trifluoroacetate | 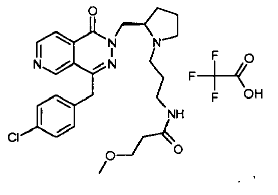 | 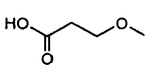 | 2.29 | 498 and 500 |
| 72 | ((2*R*)-2-{[4-[(4-chlorophenyl)methyl]-1oxopyrido[3,4-*d*]pyridazin-2(1*H*)-yl]methyl}-1-pyrrolidinyl)propyl]-4-(methyloxy)butanamide, trifluoroacetate | 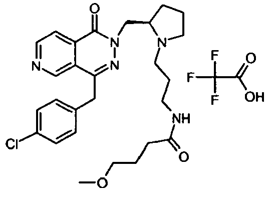 | 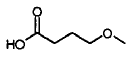 | 2.33 | 512 and 514 |

### Examples 73 to 76

The following examples were prepared by a generally similar method to that described herein in Example 36 using 2-{[(2*R*)-1-(2-aminoethyl)-2-pyrrolidinyl]methyl}-4-[(4-chlorophenyl)methyl]pyrido[3,4-*d*]pyridazin-1(2*H*)-one (for example as prepared for Intermediate 32) and the appropriate acid chloride.

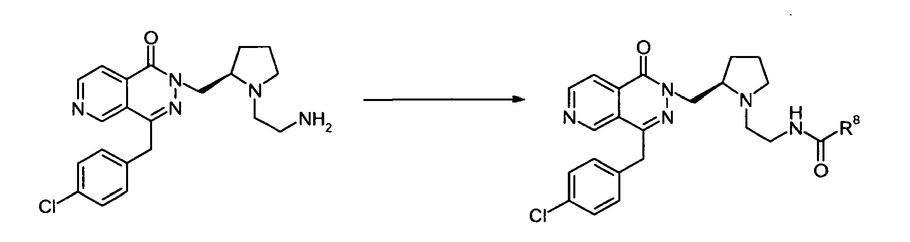

The following acid chlorides were used:
propanoyl chloride, butyryl chloride, pivaloyl chloride and isobutyryl chloride (commercially available, for example, from Aldrich).

| Ex No | Compound Name | Structure | Acid Chloride | LCMS RT (min) | ES +ve *m*/*z* (M+H)⁺ |
|---|---|---|---|---|---|
| 73 | *N*-[2-((2*R*)-2-{[4-[(4-chlorophenyl)methyl]-1-oxopyrido[3,4-*d*]pyridazin-2(1*H*)-yl]methyl}-1-pyrrolidinyl)ethyl]prop anamide, trifluoroacetate | 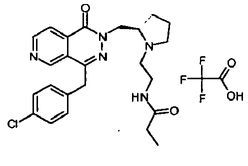 | 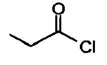 | 2.25 | 454 and 456 |
| 74 | *N*-[2-((2*R*)-2-{[4-[(4-chlorophenyl)methyl]-1-oxopyrido[3,4-*d*]pyridazin-2(1*H*)-yl]methyl}-1-pyrrolidinyl)ethyl]buta namide, trifluoroacetate | 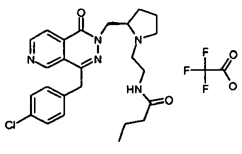 | 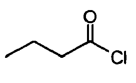 | 2.31 | 468 and 470 |
| 75 | *N*-[2-((2*R*)-2-{[4-[(4-chlorophenyl)methyl]-1-oxopyrido[3,4-*d*]pyridazin-2(1*H*)-yl]methyl}-1-pyrrolidinyl)ethyl]-2,2-dimethylpropanamide trifluoroacetate | 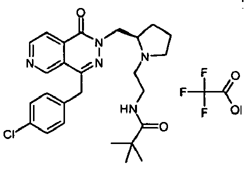 | 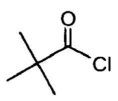 | 2.45 | 482 and 484 |
| 76 | *N*-[2-((2*R*)-2-{[4-[(4-chlorophenyl)methyl]-1-oxopyrido[3,4-*d*]pyridazin-2(1*H*)- yl]methyl}-1-pyrrolidinyl)ethyl]-2-methylpropanamide, trifluoroacetate | 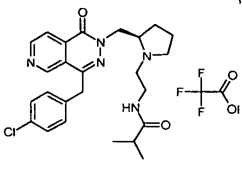 | 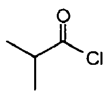 | 2.36 | 468 and 470 |

### Examples 77 to 80

The following examples were prepared by a generally similar method to that described herein in Example 36 using 2-{[(2*R*)-1-(3-aminopropyl)-2-pyrrolidinyl]methyl}-4-[(4-chlorophenyl)methyl]pyrido[3,4-*d*]pyridazin-1(2*H*)-one (for example, as prepared for Intermediate 34) and the appropriate acid chloride.

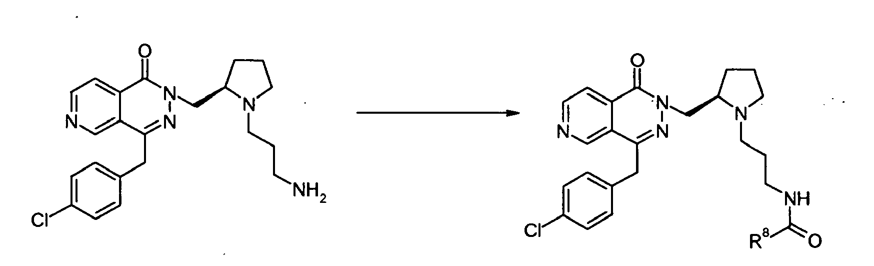

The following acid chlorides were used:
propanoyl chloride, butyryl chloride, pivaloyl chloride and isobutyryl chloride (commercially available, for example, from Aldrich)

| Ex No | Compound Name | Structure | Acid Chloride | LCMS RT (min) | ES+ve *m*/*z* (M+H)⁺ |
|---|---|---|---|---|---|
| 77 | *N*-[3-((2*R*)-2-{[4-[(4-chlorophenyl)methyl]-1-oxopyrido[3,4-*d*]pyridazin-2(1*H*)-yl]methyl}-1-pyrrolidinyl)propyl]propana mide, trifluoroacetate | 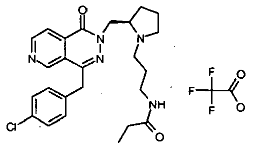 | 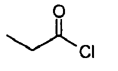 | 2.30 | 468 and 470 |
| 78 | *N*-[3-((2*R*)-2-{[4-[(4- chlorophenyl)methyl]-1-oxopyrido[3,4-*d*]pyridazin-2(1*H*)-yl]methyl}-1-pyrrolidinyl)propyl]propana mide, trifluoroacetate | 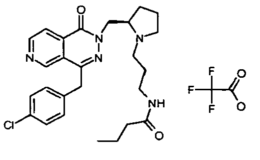 | 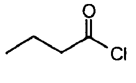 | 2.37 | 482 and 484 |
| 79 | *N*-[3-((2*R*)-2-{[4-[(4-chlorophenyl)methyl]-1-oxopyrido[3,4-*d*]pyridazin-2(1*H*)-yl]methyl}-1-pyrrolidinyl)propyl]-2-methylpropanamide, trifluoroacetate | 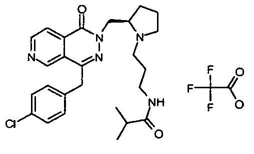 | 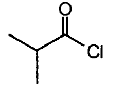 | 2.36 | 482 and 484 |
| 80 | *N*-[3-((2*R*)-2-{[4-[(4-chlorophenyl)methyl]-1-oxopyrido[3,4-*d*]pyridazin-2(1*H*)-yl]methyl}-1-pyrrolidinyl)propyl]-2,2-dimethylpropanamide, trifluoroacetate | 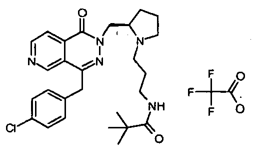 | 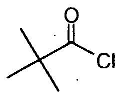 | 2.42 | 496 and 498 |

### Example 81

### 4-[(4-Chlorophenyl)methyl]-2-({(2R)-1-[2-(3-methyl-1,2,4-oxadiazol-5-yl)ethyl]-2-pyrrolidinyl}methyl)pyrido[3,4-d]pyridazin-1(2H)-one, hydrochloride

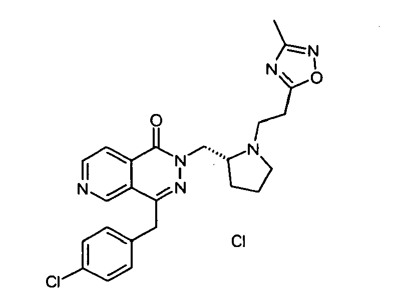

3-((2*R*)-2-{[4-[(4-Chlorophenyl)methyl]-1-oxopyrido[3,4-*d*]pyridazin-2(1*H*)-yl]methyl}-1-pyrrolidinyl)propanoic acid (partial triethylamine salt) (for example, as prepared for Intermediate 30) (20 mg, 0.041 mmol), PyBOP (21 mg, 0.041 mmol) and diisopropylethylamine (15 µl, 0.082 mmol) were mixed together in DMF (0.1 ml) at room temperature. *N*-hydroxyethanimidamide (commercially available, for example from ABCR Chemicals) (3 mg, 0.04 mmol) was added to the mixture instantly. The reaction mixture was shaken and stood at room temperature for 2 h. The mixture was transferred to a microwave vial, adding pyridine (0.1 ml), and the sealed vial was heated to 130°C for 2 min in a CEM^{™} microwave oven. This was reheated for a further 2 min at 120°C in a CEM^{™} microwave oven. The reaction mixture was purified by MDAP HPLC and the appropriate fractions were combined and concentrated. The residue was re-dissolved in chloroform and applied to an aminopropyl SPE cartridge (0.5 g), pre-conditioned with chloroform (2 ml). The product was eluted with chloroform and ethyl acetate-MeOH (10%), and the product was isolated after concentrating under a stream of nitrogen. The product was re-dissolved in a solution of HCl in MeOH (1.25M, 1.0 ml, excess). The solvent and excess acid was removed using a stream of nitrogen to give the *title compound* (4.5 mg, 24%). LCMS RT = 2.61 min, ES+ve *mlz* 465/467 (M+H)⁺.

### Example 82

### 4-[(4-Chlorophenyl)methyl]-2-({(2R)-1-[2-(3-ethyl-1,2,4-oxadiazol-5-yl)ethyl]-2-pyrrolidinyl}methyl)pyrido[3,4-d]pyridazin-1(2H)-one

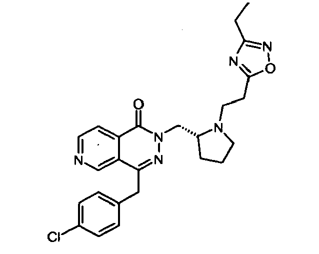

3-((2*R*)-2-{[4-[(4-chlorophenyl)methyl]-1-oxopyrido[3,4-*d*]pyridazin-2(1*H*)-yl]methyl}-1-pyrrolidinyl)propanoic acid (partial triethylamine salt) (for example, as prepared for Intermediate 30) (20 mg, 0.041 mmol), PyBOP (21 mg, 0.04 mmol) and DIPEA (7 µl, 0.08 mmol) were mixed together in DMF (0.2 ml) at room temperature. *N-*Hydroxypropanimidamide (commercially available, for example, from Alfar Aesar, Tyger Scientific and/or Fulcrum Scientific) (35 µl, 0.037 mmol) was added to the mixture and the reaction mixture was shaken and stood at room temperature for 2 h. The mixture was transferred to a microwave vial, adding pyridine (0.1 ml), and the sealed vial was heated to 80 °C for 1 min (100W) in a CEM^{™} microwave oven. Reheated thermally at 60 °C, and repeated heating at 80 °C for a further 2 min at 80°C (50W) in a CEM^{™} microwave oven. This reheating was repeated for a further 5 min at 80 °C in a CEM^{™} microwave oven, with the further addition of pyridine (0.1 ml). The reaction mixture was purified by MDAP and the appropriate fractions were combined and concentrated under a stream of nitrogen. The residue was re-dissolved in chloroform (0.5 ml) and applied to an aminopropyl SPE cartridge (0.5 g), pre-conditioned with chloroform (2 ml). The product was eluted with chloroform (1.5 ml) and the appropriate fraction was isolated after concentrating under a stream of nitrogen. The product was re-dissolved in a solution of HCl in MeOH (1.25 M, 1.0 ml, excess). The solvent and excess acid was removed using a stream of nitrogen to give the *title compound* (3.9 mg, 20%). LCMS RT = 2.76 min, ES+ve *m*/*z* 479/481 (M+H)⁺.

### Example 83

### 4-[(4-Chlorophenyl)methyl]-2-{[(2R)-1-(2-{3-[(methyloxy)methyl]-1,2,4-oxadiazol-5-yl}ethyl)-2-pyrrolidinyl]methyl}pyrido[3,4-d]pyridazin-1(2H)-one, hydrochloride

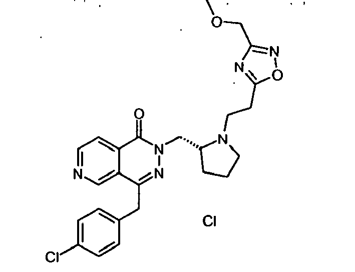

3-((2*R*)-2-{[4-[(4-Chlorophenyl)methyl]-1-oxopyrido[3,4-*d*]pyridazin-2(1*H*)-yl]methyl}-1-pyrrolidinyl)propanoic acid, partial triethylamine salt (for example, as prepared for Intermediate 30) (20 mg, 0.041 mmol), PyBOP (21 mg, 0.041 mmol) and diisopropylethylamine (7 µl, 0.08 mmol) were mixed together in DMF (0.2 ml) at room temperature. *N*-hydroxy-2-(methyloxy)ethanimidamide (commercially available, for example, from Tyger Scientific and/or Fulcrum Scientific) (40 µl, 0.037 mmol) was added to the mixture and the reaction mixture was shaken and stood at room temperature for 2 h. The mixture was transferred to a microwave vial, adding pyridine (0.1 ml), and the sealed vial was heated to 80 °C for 1 min (100W) in a CEM^{™} microwave oven. Reheated thermally at 60 °C, and repeated heating at 80°C for a further 2 min at 80 °C (50W) in a CEM^{™} microwave oven. This reheating was repeated for a further 5 min at 80 °C in a CEM^{™} microwave oven, with the further addition of pyridine (0.1 ml). The reaction mixture was purified by MDAP HPLC and the appropriate fractions were combined and concentrated to dryness under a stream of nitrogen. The residue was re-dissolved in chloroform (0.5 ml) and applied to an aminopropyl SPE cartridge (0.5 g), pre-conditioned with chloroform (2 ml). The product was eluted with chloroform (1.5 ml) and the appropriate fraction was concentrated under a stream of nitrogen. The product was re-dissolved in a solution of HCl in MeOH (1.25M, 1.0 ml, excess) and the solvent and excess acid was removed using a stream of nitrogen to give the *title compound* (3.4 mg, 17%). LCMS RT = 2.68 min, ES+ve *m*/*z* 495/497 (M+H)⁺.

### Example 84

### 4-[(4-Chlorophenyl)methyl]-2-({(2R)-1-[2-(3-propyl-1,2,4-oxadiazol-5-yl)ethyl]-2-pyrrolidinyl}methyl)pyrido[3,4-d]pyridazin-1(2H)-one, hydrochloride

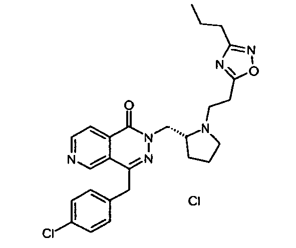

3-((2*R*)-2-{[4-[(4-Chlorophenyl)methyl]-1-oxopyrido[3,4-*d*]pyridazin-2(1*H*)-yl]methyl}-1-pyrrolidinyl)propanoic acid, partial triethylamine salt (for example, as prepared for Intermediate 30) (20 mg, 0.041 mmol), PyBOP (21 mg, 0.041 mmol) and diisopropylethylamine (7 µl, 0.082 mmol) were mixed together in DMF (0.2 ml) at room temperature. *N*-hydroxybutanimidamide (commercially available, for example, from Alfa Aesar, Apollo, Maybridge, Tyger Scientific and/or Fulcrum Scientific) (3.82 mg, 0.037 mmol) was added to the mixture and the reaction mixture was shaken and stood at room temperature for 2 h. The mixture was transferred to a microwave vial, adding pyridine (0.1 ml), and the sealed vial was heated to 80 °C for 2 min (50 W) in a CEM^{™} microwave oven. Repeated heating at 80 °C for a total of 10 min at 80 °C (50 W) in a CEM^{™} microwave oven. The reaction mixture was purified by MDAP and the appropriate fractions were combined and concentrated to dryness under a stream of nitrogen. The residue was re-dissolved in acetonitrile (0.5 ml) and applied to an aminopropyl SPE cartridge (0.5 g), pre-conditioned with chloroform (2 ml). The product was eluted with acetonitrile (1 ml) and the fraction was concentrated under a stream of nitrogen. The product was re-dissolved in a solution of HCl in MeOH (1.25M, 1.0 ml, excess). The solvent and excess acid was removed using a stream of nitrogen to give the *title compound* (2.3 mg, 12%). LCMS RT = 2.83 min, ES+ve *mlz* 493/495 (M+H)⁺.

### Biological Data

The compounds of the invention may be tested for *in vitro* and/or *in vivo* biological activity in accordance with the following or similar assays.

### H1 receptor cell line generation and FLIPR assay protocol

### 1. Generation of histamine H1 cell line

The human H1 receptor is cloned using known procedures described in the literature [Biochem. Biophys. Res. Commun., 201(2):894 (1994)]. Chinese hamster ovary (CHO) cells stably expressing the human H1 receptor are generated according to known procedures described in the literature [Br. J. Pharmacol., 117(6):1071 (1996)].

### Histamine H1 functional antagonist assay: Determination of functional pKi values

The histamine H1 cell line is seeded into non-coated black-walled clear bottom 384-well tissue culture plates in alpha minimum essential medium (Gibco/Invitrogen, cat no. 22561-021), supplemented with 10% dialysed foetal calf serum (Gibco/Invitrogen cat no. 12480-021) and 2 mM L-glutamine (Gibco/Invitrogen cat no 25030-024) and is maintained overnight at 5% CO₂, 37 °C.

Excess medium is removed from each well to leave 10 µl. 30 µl loading dye (250 µM Brilliant Black, 2 µM Fluo-4 diluted in Tyrodes buffer + probenecid (145 mM NaCl, 2.5 mM KCI, 10 mM HEPES, 10 mM D-glucose, 1.2 mM MgCl₂, 1.5 mM CaCl₂, 2.5 mM probenecid, pH adjusted to 7.40 with NaOH 1.0 M)) is added to each well and the plates are incubated for 60 min at 5% CO₂, 37 °C.

10 µl of test compound, diluted to the required concentration in Tyrodes buffer + probenecid (or 10 µl Tyrodes buffer + probenecid as a control) is added to each well and the plate is incubated for 30 min at 37 °C, 5% CO₂. The plates are then placed into a FLIPR™ (Molecular Devices, UK) to monitor cell fluorescence (λₑₓ = 488 nm, λ_{EM} = 540 nm) in the manner described in Sullivan et al., (In: Lambert DG (ed.), Calcium Signaling Protocols, New Jersey: Humana Press, 1999, 125-136) before and after the addition of 10 µl histamine at a concentration that results in the final assay concentration of histamine being EC₈₀.

Functional antagonism is indicated by a suppression of histamine induced increase in fluorescence, as measured by the FLIPR™ system (Molecular Devices). By means of concentration effect curves, functional affinities are determined using standard pharmacological mathematical analysis.

### Histamine H1 functional antagonist assay: Determination of antagonist pA2 and duration

The histamine H1 receptor expressing CHO cells are seeded into non-coated black-walled clear bottom 96-well tissue culture plates as described above.

Following overnight culture, growth medium is removed from each well, washed with 200 µl PBS and is replaced with 50 µl loading dye (250 µM Brilliant Black, 1 µM Fluo-4 diluted in Tyrodes buffer + probenecid (145 mM NaCl, 2.5 mM KCI, 10mM HEPES, 10mM D-glucose, 1.2 mM MgCl₂, 1.5 mM CaCl₂, 2.5 mM probenecid, pH adjusted to 7.40 with NaOH 1.0 M)). Cells are incubated for 45 min at 37 °C. The loading buffer is removed and the cells are washed as above, and 90 µl of Tyrodes buffer + probenecid is added to each well. 10 µl of test compound, diluted to the required concentration in Tyrodes buffer + probenecid (or 10 µl Tyrodes buffer + probenecid as a control) is added to each well and the plate is incubated for 30 min at 37 °C, 5% CO₂.

The plates are then placed into a FLIPR™ (Molecular Devices, UK) to monitor cell fluorescence (λₑₓ = 488 nm, λ_{EM} = 540 nm) in the manner described in Sullivan et al., (In: Lambert DG (ed.), Calcium Signaling Protocols, New Jersey: Humana Press, 1999, 125-136) before and after the addition of 50 µl histamine over a concentration range of 1 mM-0.1 nM. The resultant concentration response curves are analysed by non-linear regression using a standard four parameter logistic equation to determine the histamine EC₅₀, the concentration of histamine required to produce a response of 50% of the maximum response to histamine. The antagonist pA2 is calculated using the following standard equation: pA2 = log(DR-1)-log[B] where DR = dose ratio, defined as EC₅₀antagonist-treated/EC₅₀control and [B] = concentration of antagonist.

To determine the antagonist duration, cells are cultured overnight in non-coated black-walled clear bottom 96-well tissue culture plates, are washed with PBS and are incubated with a concentration of antagonist chosen to give an approximate DR in the range 30 - 300. Following the 30 min antagonist incubation period, the cells are washed two or three times with 200 µl of PBS and then 100 µl Tyrodes buffer is added to each well to initiate antagonist dissociation. Following incubation for predetermined times, typically 30 - 270 min at 37 °C, the cells are then washed again with 200 µl PBS and are incubated with 100 µl Tyrodes buffer containing Brilliant Black, probenecid and Fluo-4 for 45 min at 37 °C, as described above. After this period, the cells are challenged with histamine in the FLIPR™ as described above. The dose ratio at each time point is used to determine the fractional H1 receptor occupancy by the following equation: fractional receptor occupancy = (DR-1)/DR. The decrease in receptor occupancy over time approximates to a straight line and is analysed by linear regression. The slope of this straight line fit is used as an index of the dissociation rate of the antagonist. The dose ratios for antagonist treated cells and for antagonist treated and washed cells at each time point are used to calculate a relative dose ratio (rel DR) which is also used as an index of antagonist duration. Antagonists with long duration of action produce rel DR values close to 1, and antagonists with short duration of action produce rel DR values that approaches the dose ratio value obtained for antagonist treatment alone.

### 2. H3 receptor cell line generation, membrane preparation and functional GTPγS assay protocols

### Generation of histamine H3 cell line

The histamine H3 cDNA is isolated from its holding vector, pCDNA3.1 TOPO (InVitrogen), by restriction digestion of plasmid DNA with the enzymes BamH1 and Not-1 and is ligated into the inducible expression vector pGene (InVitrogen) digested with the same enzymes. The GeneSwitch™ system (a system where in transgene expression is switched off in the absence of an inducer and switched on in the presence of an inducer) is performed as described in US Patents: 5,364,791; 5,874,534; and 5,935,934. Ligated DNA is transformed into competent DH5α *E. coli* host bacterial cells and is plated onto Luria Broth (LB) agar containing Zeocin™ (an antibiotic which allows the selection of cells expressing the *sh ble* gene which is present on pGene and pSwitch) at 50 µgml⁻¹. Colonies containing the re-ligated plasmid are identified by restriction analysis. DNA for transfection into mammalian cells is prepared from 250 ml cultures of the host bacterium containing the pGeneH3 plasmid and is isolated using a DNA preparation kit (Qiagen Midi-Prep) as per manufacturers guidelines (Qiagen).

CHO K1 cells previously transfected with the pSwitch regulatory plasmid (InVitrogen) are seeded at 2×10⁶ cells per T75 flask in Complete Medium, containing Hams F12 (GIBCOBRL, Life Technologies) medium supplemented with 10% v/v dialysed foetal bovine serum, L-glutamine, and hygromycin (100 µgml⁻¹), 24 h prior to use. Plasmid DNA is transfected into the cells using Lipofectamine plus according to the manufacturer's guidelines (InVitrogen). 48 h post transfection, cells are placed into complete medium supplemented with 500 µgml⁻¹ Zeocin™.

10-14 days post selection, 10 nM Mifepristone (InVitrogen) is added to the culture medium to induce the expression of the receptor. 18 h post induction, cells are detached from the flask using ethylenediamine tetra-acetic acid (EDTA; 1:5000; InVitrogen), following several washes with PBS, pH 7.4 and are resuspended in Sorting Medium containing Minimum Essential Medium (MEM), without phenol red, and are supplemented with Earles salts and 3% Foetal Clone II (Hyclone). Approximately 1×10⁷ cells are examined for receptor expression by staining with a rabbit polyclonal antibody, 4a, raised against the *N*-terminal domain of the histamine H3 receptor, are incubated on ice for 60 min, followed by two washes in sorting medium. Receptor bound antibody is detected by incubation of the cells for 60 min on ice with a goat anti rabbit antibody, conjugated with Alexa 488 fluorescence marker (Molecular Probes). Following two further washes with Sorting Medium, cells are filtered through a 50 µm Filcon™ (BD Biosciences) and then are analysed on a FACS Vantage SE Flow Cytometer fitted with an Automatic Cell Deposition Unit. Control cells are non-induced cells treated in an analogous manner. Positively stained cells are sorted as single cells into 96-well plates, containing Complete Medium containing 500 µgml⁻¹ Zeocin™ and are allowed to expand before reanalysis for receptor expression via antibody and ligand binding studies. One clone, 3H3, is selected for membrane preparation.

### Membrane preparation from cultured cells

All steps of the protocol are carried out at 4 °C and with pre-cooled reagents. The cell pellet is resuspended in 10 volumes of homogenisation buffer (50 mM *N*-2-hydroxyethylpiperazine-*N*'-2-ethanesulfonic acid (HEPES), 1 mM ethylenediamine tetra-acetic acid (EDTA), pH 7.4 with KOH, supplemented with 10⁻⁶ M leupeptin (acetyl-leucyl-leucyl-arginal; Sigma L2884), 25 µgml⁻¹ bacitracin (Sigma B0125), 1 mM phenylmethylsulfonyl fluoride (PMSF) and 2×10⁻⁶ M pepstain A (Sigma)). The cells are then homogenised by 2 × 15 second bursts in a 1 litre glass Waring blender, followed by centrifugation at 500 g for 20 min. The supernatant is then spun at 48,000 g for 30 min. The pellet is resuspended in homogenisation buffer (4x the volume of the original cell pellet) by vortexing for 5 sec, followed by homogenisation in a Dounce homogeniser (10-15 strokes). At this point the preparation is aliquoted into polypropylene tubes and stored at -80 °C.

### Histamine H3 functional antagonist assay

For each compound being assayed, in a solid white 384 well plate, is added:-
(a) 0.5 µl of test compound diluted to the required concentration in DMSO (or 0.5 µl DMSO as a control);
(b) 30 µl bead/membrane/GDP mix which is prepared by mixing Wheat Germ Agglutinin Polystyrene LeadSeeker® (WGA PS LS) scintillation proximity assay (SPA) beads with membrane (prepared in accordance with the methodology described above) and diluting in assay buffer (20 mM *N*-2-hydroxyethylpiperazine-*N*'-2-ethanesulfonic acid (HEPES) + 100 mM NaCl + 10 mM MgCl₂, pH 7.4 NaOH) to give a final volume of 30 µl which contains 5 µg protein, 0.25 mg bead per well and 10 µM final assay concentration of guanosine 5' diphosphate (GDP) (Sigma, diluted in assay buffer) incubating at room temperature for 60 min on a roller;
(c) 15 µl 0.38 nM [³⁵S]-GTPγS (Amersham; Radioactivity concentration = 37 MBqml⁻¹; Specific activity = 1160 Cimmol⁻¹), histamine (at a concentration that results in the final assay concentration of histamine being EC₈₀).

After 2-6 h, the plate is centrifuged for 5 min at 1500 rpm and counted on a Viewlux counter using a 613/55 filter for 5 minplate⁻¹. Data is analysed using a 4-parameter logistic equation. Basal activity is used as minimum, i.e. histamine not added to well.

### Intranasal Challenge Method: Whole Body Plethysmography

### (a)Sensitisation

Female Dunkin-Hartley guinea pigs 150-250g are sensitised twice daily for 5 days (week 1) with ovalbumin (OVA) and aluminium hydroxide (Al(OH)₃ or Alum) in physiological saline, 25µl/nostril. Solution is made up at 20µg/ml OVA, 180mg/ml Alum. During weeks 2 and 3 animals receive 25µl/nostril of OVA (5mg/ml) once daily. During Week 4 guinea pigs will be entered into study but are continually sensitized as per weeks 2 and 3 until the day before dosing with compound or vehicle.

### (b) Compound/Vehicle Pretreatment

Pretreatment with test compound is performed at various times prior to histamine challenge. Efficacy dose-response curves are determined 1 hr and/or 3hr after dosing whereas duration of action may be studied up to 7 days post dose. Test compounds are formulated as solutions in 0.9% sterile saline or suspensions in 0.9% sterile saline/tween80.

Guinea pigs were anaesthetised with isoflurane (5%, 2-3l/min O₂), placed in a supine position, and 25µl of test compound or vehicle dosed into each nostril using a Gilson pipette. After dosing, animals remain supine for at least 30 seconds during recovery from anaesthesia.

### (c) Histamine Challenge Protocol

At 30 minutes before the time of histamine challenge, guinea pigs are dosed with atropine sulphate (Sigma A0257, dissolved in saline), 1mg/kg i.p. Animals are then placed into whole body plethysmograph systems (Buxco® Electronics) where the parameter PenH area under curve (AUC) is recorded as outlined in HAMELMANN, E., SCHWARZE, J., TAKEDA, K., OSHIBA, A., LARSEN, L., IRVIN, C.G. & GELFAND, E.W. (1997) Noninvasive measurement of airway responsiveness in allergic mice using barometric plethysmography. Am. J. Respir. Crit. Care Med. 156, 766-775.
Hamelmann et al. A 10 minute baseline AUC is recorded and if this value is over 1000, the animals are excluded.

After the stipulated pre-dose time has been reached, guinea pigs are re-anaesthetised with isoflurane and dosed with either 10 mM or 15 mM histamine or phosphate-buffered saline (PBS), (25µl per nostril). On recovery from anaesthesia animals are returned to the individual plethysmograph chambers and 4x10 min consecutive PenH AUC recordings are made. These recordings are summed to give a cumulative AUC over 40 mins post histamine challenge for each animal. Data are analysed using ANOVA with post-hoc Fishers LSD test (general linear models, Statistica®) and finally Hochberg adjustment. Inhibition of histamine-induced congestion is determined by statistically significant differences between the mean responses of compound pre-treated groups compared to the vehicle pre-treated, histamine-challenged group.

### CNS penetration

### (i) CNS penetration by bolus administration

Compounds are dosed intravenously at a nominal dose level of 1 mgkg⁻¹ to male CD Sprague Dawley rats. Compounds are formulated in 5% DMSO/45% PEG200/50% water. Blood samples are taken under terminal anaesthesia with isoflurane at 5 min post-dose and the brains are also removed for assessment of brain penetration. Blood samples are taken directly into heparinised tubes. Blood samples are prepared for analysis using protein precipitation and brain samples are prepared using extraction of drug from brain by homogenisation and subsequent protein precipitation. The concentration of parent drug in blood and brain extracts is determined by quantitative LC-MS/MS analysis using compound-specific mass transitions.

### (ii) CNS penetration following intravenous infusion at steady state

A loading dose of the compounds is given to male CD Sprague Dawley rats at a nominal dose level of 0.4 mgkg⁻¹. The compounds are then infused intravenously for 4 h at a nominal dose level of 0.1 mgkg⁻¹h⁻¹. Compounds are formulated in 2% DMSO/30% PEG200/68% water. Serial or terminal blood samples are taken at 0.5, 1.5, 2.5, 3, 3.5 and 4 h post dose. The final blood sample is collected under terminal anaesthesia with isoflurane and the brains are also removed for assessment of brain penetration. Blood samples are taken directly into heparinised tubes. Blood samples are prepared for analysis using protein precipitation and brain samples are prepared using extraction of drug from brain by homogenisation and subsequent protein precipitation. The concentration of parent drug in blood and brain extracts is determined by quantitative LC-MS/MS analysis using compound-specific mass transitions.

### Rat pharmacokinetics

Compounds are dosed to male CD Sprague Dawley rats by single intravenous or oral administration at a nominal dose level of 1 mgkg⁻¹ and 3 mgkg⁻¹ respectively. Compounds are formulated in 5% DMSO/45% PEG200/50% water. An intravenous profile is obtained by taking serial or terminal blood samples at 0.083, 0.25, 0.5, 1, 2, 4, and 7 h post dose (for some studies 12 and 24 h samples may be taken). An oral profile is obtained by taking serial or terminal blood samples at 0.25, 0.5, 1, 2, 4, 7 and 12 h post dose (for some studies 24 and 30 h samples may be taken). Blood samples are taken directly into heparinised tubes. Blood samples are prepared by protein precipitation and subjected to quantitative analysis by LC-MS/MS using compound-specific mass transitions. Drug concentration-time profiles are generated and non-compartmental PK analysis used to generate estimates of half-life, clearance, volume of distribution and oral bioavailability.

### Dog pharmacokinetics

Compounds are dosed to male Beagle dogs by single intravenous or oral administration at a nominal dose level of 1 mgkg⁻¹ and 2 mgkg⁻¹ respectively. The study is carried out according to a crossover design such that the same dog is used for both dosing events and the dosing events occurred 1 week apart. Compounds are formulated in 5% DMSO/45% Peg200/50% water. An intravenous profile is obtained by taking serial blood samples at 0.083, 0.25, 0.5, 0.75, 1, 2, 4, 6 and 12 h post dose (for some studies 24 h samples may be taken). An oral profile is obtained by taking serial blood samples at 0.25, 0.5, 0.75, 1, 2, 4, 6, 12 and 24 h post dose. Blood samples are taken directly into heparinised tubes. Blood samples are prepared by protein precipitation and subjected to quantitative analysis by LC-MS/MS using compound-specific mass transition. Drug concentration-time profiles are generated and non-compartmental PK analysis used to generate estimates of half-life, clearance, volume of distribution and oral bioavailability.

### Results

In these or similar biological assays, the following data were obtained:
(i) Compound of Examples 1-34c, 35, 36a, 36b, 37-46, 49-80 and 82-84 had an average pKi (pKb) at H1 of greater than approximately 6.5. Compound of Examples 1-29, 31-35, 36a, 36b, 37-46, 49-80 and 82-84 had an average pKi (pKb) at H1 of greater than approximately 7.5. Compound of Examples 1-34c, 35, 36a, 36b, 37-46, 49-80 and 82-84 had average pA2 values of greater than approximately 8.
(ii) Compound of Examples 1-34c, 35, 36a, 36b, 37-46, 49-80 and 82-84 had an average pKi (pKb) at H3 of less than approximately 6.5.
(iii) Compound of Examples 1, 4, 6,12,14,17-20, 22, 24, 26, 30, 34, 35 ,36a, 36b, 38, 39, 41, 42, 49-56, 61, 67-69 70, and 80 exhibited at one or more time points a longer duration of action than azelastine in the histamine H1 functional antagonist assay. Other compounds were either not tested or were tested and did not exhibit a longer duration of action.
(iv) In the intranasal challenge model compound of Examples 34, 34a, 34c and 36b dosed intranasally at 1mg/ml either 1 hr or 24hr before histamine challenge significantly (p<0.05) inhibited the response at both timepoints. In the same model, azelastine failed to show a similar duration of action when administered at the same concentration. In another experiment compound of Example 34 dosed intranasally at 1mg/ml and at 10mg/ml either 3hr or 24hr before histamine challenge significantly (p<0.05) inhibited the response at the 3hr timepoint and not the 24 hr timepoint. Other compounds were either not tested or were tested and significantly (p<0.05) inhibited the response at the 1 hr timepoint and not the 24 hr timepoint.

## Claims

1. A compound of formula (I) 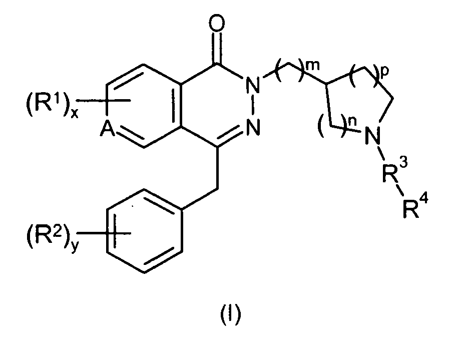 in which:
A represents N or CH;
R¹ and R² each independently represent halogen, C₁₋₃alkyl, C₁₋₃alkoxy, hydroxy or trifluoromethyl;
x and y each independently represent 0, 1 or 2;
m represents 0, n represents 2 and p represents 1; or
m represents 1, n represents 0 and p represents 2;
R³ represents a straight chain C₁₋₆alkylene optionally substituted by one or two C₁₋₃alkyl; and
R⁴ represents a group of formula (i), (ii), (iii) or (iv)
(i) -C(O)N(R⁵)R⁶ in which
R⁵ represents hydrogen or -C₁₋₆alkyl;
R⁶ represents hydrogen; -C₁₋₆alkyl (optionally substituted by up to three substituents independently selected from halogen and hydroxy); -C₁₋₆alkyl-O-C₁₋₆alkyl (optionally substituted by up to three substituents independently selected from halogen and hydroxy); C₃₋₇cycloalkyl (optionally substituted by up to three substituents independently selected selected from halogen, hydroxy and C₁₋₃alkyl); -C₁₋₃alkylC₃₋₇cycloalkyl (in which the C₁₋₃alkyl is optionally substituted by up to three substituents independently selected from halogen and hydroxy, and the C₃₋₇cycloalkyl is optionally substituted by up to three substituents independently selected from halogen, hydroxy and C₁₋₃alkyl); aryl (optionally substituted by up to three substituents independently selected from halogen, C₁₋₃alkyl, trifluoromethyl, and cyano); -C₁₋₆alkylaryl (in which the C₁₋₆alkyl is optionally substituted by up to three substituents independently selected from C₁₋₃alkyl, halogen and hydroxy, and the aryl is optionally substituted by up to three substituents independently selected from halogen, C₁₋₃alkyl, trifluoromethyl, and cyano); or -(C₁₋₃alkyl)_{z}Het, in which z represents 0 or 1 and Het represents a 5 to 7 membered saturated heterocyclic ring containing one or two heteroatoms independently selected from O and S;
or R⁵ and R⁶ together with the N atom to which they are attached represent a 5 to 7 membered saturated heterocyclic ring optionally containing one or two further heteroatoms independently selected from O and S;
(ii) -N(R⁷)C(O)R⁸ in which
R⁷ represents hydrogen or -C₁₋₆alkyl; and
R⁸ represents -C₁₋₆alkyl (optionally substituted up to three substituents independently selected from halogen and hydroxy); -C₁₋₆alkyl-O-C₁₋₆alkyl (optionally substituted up to three substituents independently selected from halogen and hydroxy); C₃₋₇cycloalkyl (optionally substituted by up to three substituents independently selected selected from halogen, hydroxy and C₁₋₃alkyl); -C₁₋₃alkylC₃₋₇cycloalkyl (in which the C₁₋₃alkyl is optionally substituted by up to three substituents independently selected from halogen and hydroxy, and the C₃₋₇cycloalkyl is optionally substituted by up to three substituents independently selected from halogen, hydroxy and C₁₋₃alkyl); aryl (optionally substituted by up to three substituents independently selected from halogen, C₁₋₃alkyl, trifluoromethyl, and cyano); - C₁₋₆alkylaryl (in which the C₁₋₆alkyl is optionally substituted by up to three substituents independently selected from C₁₋₃alkyl, halogen and hydroxy, and the aryl is optionally substituted by up to three substituents independently selected from by halogen, C₁₋₃alkyl, trifluoromethyl, and cyano); or -(C₁₋₃alkyl)_{z}Het, in which z represents 0 or 1 and Het represents a 5 to 7 membered saturated heterocyclic ring containing one or two heteroatoms independently selected from O and S; 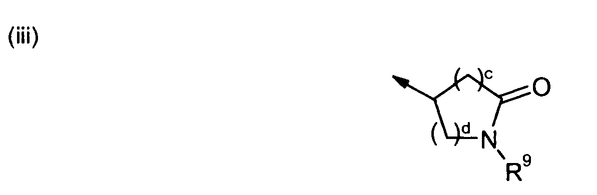 in which c represents 0 or 1 and d represents 2 or 3, or c represents 2 or 3 and d represents 0 or 1; and
R⁹ represents hydrogen or C₁₋₆alkyl; 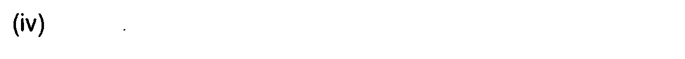 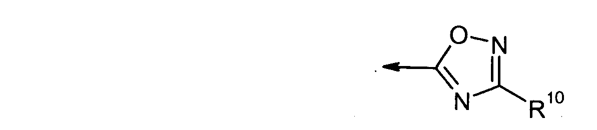 in which R¹⁰ represents hydrogen, or -C₁₋₆alkyl (optionally substituted by up to three substituents independently selected from halogen and hydroxy); -C₁₋₆alkyl-O-C₁₋₆alkyl (optionally substituted by up to three substituents independently selected from halogen and hydroxy); aryl (optionally substituted by up to three substituents independently selected from halogen, C₁₋₃alkyl, trifluoromethyl, and cyano); or -C₁₋₆alkylaryl (in which the C₁₋₆alkyl is optionally substituted by up to three substituents independently selected from C₁₋₃alkyl, halogen and hydroxy, and the aryl is optionally substituted by up to three substituents independently selected from halogen, C₁₋₃alkyl, trifluoromethyl, and cyano);
or a salt thereof.

2. A compound according to claim 1 wherein A represents CH.

3. A compound according to claim 1 or claim 2 wherein m represents 1, n represents 0 and p represents 2.

4. A compound according to any of claims 1 to 3 wherein x is 0.

5. A compound according to any of claims 1 to 4 wherein R² is halogen and y is 1.

6. A compound according to any of claims 1 to 5 wherein R⁴ represents -C(O)N(R⁵)R⁶.

7. A compound according to any of claims 1 to 5 wherein R⁴ represents -N(R⁷)C(Q)R⁸.

8. A compound according to any of claims 1 to 5 wherein R⁴ represents

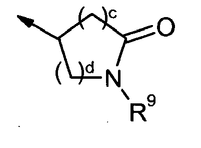

9. A compound which is
3-((2*R*)-2-{[4-[(4-Chlorophenyl)methyl]-1-oxo-2(1*H*)-phthalazinyl]methyl}-1-pyrrolidinyl)-*N-*(2-fluoroethyl)propanamide;
3-((2*R*)-2-{[4-[(4-chlorophenyl)methyl]-1-oxo-2(1*H*)-phthalazinyl]methyl}-1-pyrrolidinyl)-2-methyl-N-[2-(methyloxy)ethyl]propanamide;
3-((2*R*)-2-{[4-[(4-chlorophenyl)methyl]-1-oxo-2(1*H*)-phthalazinyl]methyl}-1-pyrrolidinyl)-*N-*(phenylmethyl)propanamide;
3-((2*R*)-2-{[4-[(4-chlorophenyl)methyl]-1-oxo-2(1*H*)-phthalazinyl]methyl}-1-pyrrolidinyl)-*N-*(2,2,2-trifluoroethyl)propanamide;
3-((2*R*)-2-{[4-[(4-chlorophenyl)methyl]-1-oxo-2(1*H*)-phthalazinyl]methyl}-1-pyrrolidinyl)-*N-*(2,2,2-trifluoroethyl)propanamide;
3-((2*R*)-2-{[4-[(4-chlorophenyl)methyl]-1-oxo-2(1*H*)-phthalazinyl]methyl}-1-pyrrolidinyl)-*N-*(2,2-difluoroethyl)propanamide;
3-((2*R*)-2-{[4-[(4-chlorophenyl)methyl]-1-oxo-2(1*H*)-phthalazinyl]methyl}-1-pyrrolidinyl)-*N-*(2,2-difluoroethyl)propanamide;
3-((2*R*)-2-{[4-[(4-chlorophenyl)methyl]-1-oxo-2(1*H*)-phthalazinyl]methyl}-1-pyrrolidinyl)-*N-*(2-fluoroethyl)propanamide;
3-((2*R*)-2-{[4-[(4-chlorophenyl)methyl]-1-oxo-2(1*H*)-phthalazinyl]methyl}-1-pyrrolidinyl)-*N-*methylpropanamide;
3-((2*R*)-2-{[4-[(4-chlorophenyl)methyl]-1-oxo-2(1*H*)-phthalazinyl]methyl}-1-pyrrolidinyl) propanamide;
3-((2*R*)-2-{[4-[(4-chlorophenyl)methyl]-1-oxo-2(1*H*)-phthalazinyl]methyl}-1-pyrrolidinyl)-*N-*ethyl-N-methylpropanamide; 4-[(4-chlorophenyl)methyl]-2-({(2*R*)-1-[3-oxo-3-(1-piperidinyl)propyl]-2-pyrrolidinyl}methyl)-1 (2H)-phthalazinone;
4-[(4-chlorophenyl)methyl]-2-({(2*R*)-1-[3-(4-morpholinyl)-3-oxopropyl]-2-pyrrolidinyl} methyl)-1(2*H*)-phthalazinone;
3-((2*R*)-2-{[4-[(4-chlorophenyl)methyl]-1-oxo-2(1*H*)-phthalazinyl]methyl}-1-pyrrolidinyl)-*N-*methyl-*N*-[2-(methyloxy)ethyl]propanamide;
3-((2*R*)-2-{[4-[(4-chlorophenyl)methyl]-1-oxo-2(1*H*)-phthalazinyl]methyl}-1-pyrrolidinyl)-*N-*{2-[(2,2,2-trifluoroethyl)oxy]ethyl}propanamide;
3-((2*R*)-2-{[4-[(4-chlorophenyl)methyl]-1-oxo-2(1*H*)-phthalazinyl]methyl}-1-pyrrolidinyl)-*N-*ethylpropanamide;
3-((2*R*)-2-{[4-[(4-chlorophenyl)methyl]-1-oxo-2(1*H*)-phthalazinyl]methyl}-1-pyrrolidinyl)-*N-*ethylpropanamide;
3-((2*R*)-2-{[4-[(4-chlorophenyl)methyl]-1-oxo-2(1*H*)-phthalazinyl]methyl}-1-pyrrolidinyl)-*N-*(1-methylethyl)propanamide;
3-((2*R*)2-{[4-[(4-chlorophenyl)methyl]-1-oxo-2(1*H*)-phthalazinyl]methyl}-1-pyrrolidinyl)-*N-*propylpropanamide;
3-((2*R*)-2-{[4-[(4-chlorophenyl)methyl]-1-oxo-2(1*H*)-phthalazinyl]methyl}-1-pyrrolidinyl)-*N-*(1,1-dimethylethyl)propanamide;
3-((2*R*)-2-{[4-[(4-chlorophenyl)methyl]-1-oxo-2(1*H*)-phthalazinyl]methyl}-1-pyrrolidinyl)-*N-*[2-(methyloxy)ethyl]propanamide;
3-((2*R*)-2-{[4-[(4-chlorophenyl)methyl]-1-oxo-2(1*H*)-phthalazinyl]methyl}-1-pyrrolidinyl)-*N-*[2-(ethyloxy)ethyl]propanamide;
3-((2*R*)-2-{[4-[(4-chlorophenyl)methyl]-1-oxo-2(1*H*)-phthalazinyl]methyl}-1-pyrrolidinyl)-*N-*[3-(methyloxy)propyl]propanamide;
4-((2*R*)-2-{[4-[(4-chlorophenyl)methyl]-1-oxo-2(1*H*)-phthalazinyl]methyl}-1-pyrrolidinyl)-*N-*[2-(methyloxy)ethyl]butanamide;
4-((2*R*)-2-{[4-[(4-Chlorophenyl)methyl]-1-oxo-2(1*H*)-phthalazinyl]methyl}-1-pyrrolidinyl)-*N-*[2-(methyloxy)ethyl]butanamide;
4-((2*R*)-2-{[4-[(4-Chlorophenyl)methyl]-1-oxo-2(1*H*)-phthalazinyl]methyl}-1-pyrrolidinyl)-*N-*propylbutanamide;
2-((2*R*)-2-{[4-[(4-Chlorophenyl)methyl]-1-oxo-2(1*H*)-phthalazinyl]methyl}-1-pyrrolidinyl)-*N-*[3-(methyloxy)propyl]acetamide;
*N*-butyl-2-((2*R*)-2-{[4-[(4-chlorophenyl)methyl]-1-oxo-2(1*H*)-phthalazinyl]methyl}-1-pyrrolidinyl)acetamide;
4-[(4-chlorophenyl)methyl]-2-({(2*R*)-1-[2-oxo-2-(1-piperidinyl)ethyl]-2-pyrrolidinyl}methyl)-1(2*H*)-phthalazinone;
2-((2*R*)2-{[4-[(4-chlorophenyl)methyl]-1-oxo-2(1*H*)-phthalazinyl]methyl}-1-pyrrolidinyl)-*N-*(1,1-dimethylethyl)acetamide;
3-{4-[4-[(4-Chlorophenyl)methyl]-1-oxo-2(1*H*)-phthalazinyl]-1-piperidinyl}-*N*-[2-(methyloxy) ethyl]propanamide;
3-{4-[4-[(4-Chlorophenyl)methyl]-1-oxo-2(1*H*)-phthalazinyl]-1-piperidinyl}-*N*-[3-(methyloxy) propyl]propanamide;
3-{4-[4-[(4-Chlorophenyl)methyl]-1-oxo-2(1*H*)-phthalazinyl]-1-piperidinyl}-*N-*ethylpropanamide;
*N*-[2-((2*R*)-2-{[4-[(4-Chlorophenyl)methyl]-1-oxo-2(1*H*)-phthalazinyl]methyl}-1-pyrrolidinyl) ethyl]-4-(methyloxy)butanamide;
*N*-[2-((2*R*)-2-{[4-[(4-Chlorophenyl)methyl]-1-oxo-2(1*H*)-phthalazinyl]methyl}-1-pyrrolidinyl) ethyl]-3-(methyloxy)propanamide;
*N*-[2-((2*R*)-2-{[4-[(4-Chlorophenyl)methyl]-1-oxo-2(1*H*)-phthalazinyl]methyl}-1-pyrrolidinyl) ethyl]-3,3,3-trifluoropropanamide;
*N*-[2-((2*R*)-2-{[4-[(4-chlorophenyl)methyl]-1-oxo-2(1*H*)-phthalazinyl]methyl}-1-pyrrolidinyl) ethyl]butanamide;
*N*-[2-((2*R*)-2-{[4-[(4-chlorophenyl)methyl]-1-oxo-2(1*H*)-phthalazinyl]methyl}-1-pyrrolidinyl) ethyl]-2,2-dimethylpropanamide;
*N*-[2-((2*R*)-2-{[4-[(4-chlorophenyl)methyl]-1-oxo-2(1*H*)-phthalazinyl]methyl}-1-pyrrolidinyl) ethyl]-2-(methyloxy)acetamide;
*N*-[2-((2*R*)-2-{[4-[(4-Chlorophenyl)methyl]-1-oxo-2(1*H*)-phthalazinyl]methyl}-1-pyrrolidinyl) ethyl]propanamide;
*N*-[2-((2*R*)-2-{[4-[(4-chlorophenyl)methyl]-1-oxo-2(1*H*)-phthalazinyl]methyl}-1-pyrrolidinyl) ethyl]-2-methylpropanamide;
*N*-[3-((2*R*)-2-{[4-[(4-Chlorophenyl)methyl]-1-oxo-2(1*H*)-phthalazinyl]methyl}-1-pyrrolidinyl) propyl]-3-(methyloxy)propanam ide;
*N*-[3-((2*R*)-2-{[4-[(4-Chlorophenyl)methyl]-1-oxo-2(1*H*)-phthalazinyl]methyl}-1-pyrrolidinyl) propyl]acetamide;
*N*-[3-((2*R*)-2-{[4-[(4-Chlorophenyl)methyl]-1-oxo-2(1*H*)-phthalazinyl]methyl}-1-pyrrolidinyl) propyl]propanamide;
*N*-[3-((2*R*)-2-{[4-[(4-Chlorophenyl)methyl]-1-oxo-2(1*H*)-phthalazinyl]methyl}-1-pyrrolidinyl) propyl]-2-methylpropanamide;
*N*-[3-((2*R*)-2-{[4-[(4-Chlorophenyl)methyl]-1-oxo-2(1*H*)-phthalazinyl]methyl}-1-pyrrolidinyl) propyl]-2,2-dimethylpropanamide;
*N*-[2-((2*R*)-2-{[4-[(4-Chlorophenyl)methyl]-1-oxo-2(1*H*)-phthalazinyl]methyl}-1-pyrrolidinyl) ethyl]-2,2,2-trifluoroacetamide;
*N*-[2-((2*R*)-2-{[4-[(4-Chlorophenyl)methyl]-1-oxo-2(1*H*)-phthalazinyl]methyl}-1-pyrrolidinyl) ethyl]-2,2,2-trifluoro-N-methylacetamide;
*N*-[2-((2*R*)-2-{[4-[(4-Chlorophenyl)methyl]-1-oxo-2(1*H*)-phthalazinyl]methyl}-1-pyrrolidinyl) ethyl]-*N*-methyl-4-(methyloxy)butanamide;
*N*-[2-((2*R*)-2-{[4-[(4-Chlorophenyl)methyl]-1-oxo-2(1*H*)-phthalazinyl]methyl}-1-pyrrolidinyl) ethyl]cyclopentanecarboxamide;
*N*-[2-((2*R*)-2-{[4-[(4-chlorophenyl)methyl]-1-oxo-2(1*H*)-phthalazinyl]methyl}-1-pyrrolidinyl) ethyl]tetrahydro-2*H*-pyran-4-carboxamide;
*N*-[2-((2*R*)-2-{[4-[(4-chlorophenyl)methyl]-1-oxo-2(1*H*)-phthalazinyl]methyl}-1-pyrrolidinyl) ethyl]-3-hydroxy-2,2-dimethylpropanamide;
*N*-[2-((2*R*)-2-{[4-[(4-chlorophenyl)methyl]-1-oxo-2(1*H*)-phthalazinyl]methyl}-1-pyrrolidinyl) ethyl]tetrahydro-2*H*-pyran-3-carboxamide;
*N*-[2-((2*R*)-2-{[4-[(4-chlorophenyl)methyl]-1-oxo-2(1*H*)-phthalazinyl]methyl}-1-pyrrolidinyl) ethyl]tetrahydro-3-furancarboxamide;
*N*-[2-((2*R*)-2-{[4-[(4-chlorophenyl)methyl]-1-oxo-2(1*H*)-phthalazinyl]methyl}-1-pyrrolidinyl) ethyl]cyclohexanecarboxamide;
2-((2*R*)-2-{[4-[(4-Chlorophenyl)methyl]-1-oxo-2(1*H*)-phthalazinyl]methyl}-1-pyrrolidinyl)-*N-*(2,2-dimethylpropyl)acetamide;
2-((2*R*)-2-{[4-[(4-chlorophenyl)methyl]-1-oxo-2(1*H*)-phthalazinyl]methyl}-1-pyrrolidinyl)-*N-*(cyclohexylmethyl)acetamide;
4-[(4-Chlorophenyl)methyl]-2-({(2*R*)-1-[(2-oxo-3-piperidinyl)methyl]-2-pyrrolidinyl}methyl)-1(2*H*)-phthalazinone;
4-[(4-Chlorophenyl)methyl]-2-{[(2*R*)1-(2-{3-[2-(methyloxy)ethyl]-1,2,4-oxadiazol-5-yl}ethyl) -2-pyrrolidinyl]methyl}-1(2*H*)-phthalazinone;
4-[(4-Chlorophenyl)methyl]-2-({(2*R*)-1-[(3-propyl-1,2,4-oxadiazol-5-yl)methyl]-2-pyrrolidinyl}methyl)-1(2*H*)-phthalazinone;
4-[(4-Chlorophenyl)methyl]-2-[((2*R*)-1-{2-[3-(1-methylethyl)-1,2,4-oxadiazol-5-yl]ethyl}-2-pyrrolidinyl)methyl]-1(2*H*)-phthalazinone;
4-[(4-Chlorophenyl)methyl]-2-({(2*R*)-1-[2-(3-propyl-1,2,4-oxadiazol-5-yl)ethyl]-2-pyrrolidinyl}methyl)-1(2*H*)-phthalazinone;
3-((2*R*)-2-{[4-[(4-Chlorophenyl)methyl]-1-oxopyrido[3,4-*d*]pyridazin-2(1*H*)-yl]methyl}-1-pyrrolidinyl)-N-[3-(methyloxy)propyl]propanamide;
3-((2*R*)-2-{[4-[(4-Chlorophenyl)methyl]-1-oxopyrido[3,4-*d*]pyridazin-2(1*H*)-yl]methyl}-1-pyrrolidinyl)-N-(2,2-difluoroethyl)propanamide;
3-((2*R*)-2-{[4-[(4-Chlorophenyl)methyl]-1-oxopyrido[3,4-*d*]pyridazin-2(1*H*)-yl]methyl}-1-pyrrolidinyl)-N-propylpropanamide;
3-((2*R*)-2-{[4-[(4-Chlorophenyl)methyl]-1-oxopyrido[3,4-*d*]pyridazin-2(1*H*)-yl]methyl}-1-pyrrolidinyl)-*N*-ethyl-*N*-methylpropanamide;
4-[(4-Chlorophenyl)methyl]-2-({(2*R*)-1-[3-oxo-3-(1-piperidinyl)propyl]-2-pyrrolidinyl}methyl) pyrido[3,4-*d*]pyridazin-1(2*H*)-one;
3-((2*R*)-2-{[4-[(4-Chlorophenyl)methyl]-1-oxopyrido[3,4-*d*]pyridazin-2(1*H*)-yl]methyl}-1-pyrrolidinyl)-*N*-(2,2,2-trifluoroethyl)propanamide;
*N*-[2-((2*R*)-2-{[4-[(4-Chlorophenyl)methyl]-1-oxopyrido[3,4-*d*]pyridazin-2(1*H*)-yl]methyl}-1-pyrrolidinyl)ethyl]-3-(methyloxy)propanamide;
*N*-[2-((2*R*)-2-{[4-[(4-chlorophenyl)methyl]-1-oxopyrido[3,4-*d*]pyridazin-2(1*H*)-yl]methyl}-1-pyrrolidinyl)ethyl]-4-(methyloxy)butanamide;
*N*-[3-((2*R*)-2-{[4-[(4-chlorophenyl)methyl]-1-oxopyrido[3,4-*d*]pyridazin-2(1*H*)-yl]methyl}-1-pyrrolidinyl)propyl]-3-(methyloxy)propanamide;
((2*R*)-2-{[4-[(4-chlorophenyl)methyl]-1-oxopyrido[3,4-*d*]pyridazin-2(1*H*)-yl]methyl}-1-pyrrolidinyl)propyl]-4-(methyloxy)butanamide;
*N*-[2-((2*R*)-2-{[4-[(4-chlorophenyl)methyl]-1-oxopyrido[3,4-*d*]pyridazin-2(1*H*)-yl]methyl}-1-pyrrolidinyl)ethyl]propanamide;
*N*-[2-((2*R*)-2-{[4-[(4-chlorophenyl)methyl]-1-oxopyrido[3,4-*d*]pyridazin-2(1*H*)-yl]methyl}-1-pyrrolidinyl)ethyl]butanamide;
*N*-[2-((2*R*)-2-{[4-[(4-chlorophenyl)methyl]-1-oxopyrido[3,4-*d*]pyridazin-2(1*H*)-yl]methyl}-1-pyrrolidinyl)ethyl]-2,2-dimethylpropanamide;
*N*-[2-((2*R*)2-{[4-[(4-chlorophenyl)methyl]-1-oxopyrido[3,4-*d*]pyridazin-2(1*H*)-yl]methyl}-1-pyrrolidinyl)ethyl]-2-methylpropanamide;
N-[3-((2*R*)-2-{[4-[(4-chlorophenyl)methyl]-1-oxopyrido[3,4-*d*]pyridazin-2(1*H*)-yl]methyl}-1-pyrrolidinyl)propyl]propanamide;
*N*-[3-((2*R*)2-{[4-[(4-chlorophenyl)methyl]-1-oxopyrido[3,4-*d*]pyridazin-2(1*H*)-yl]methyl}-1-pyrrolidinyl)propyl]propanamide;
*N*-[3-((2*R*)-2-{[4-[(4-chlorophenyl)methyl]-1-oxopyrido[3,4-*d*]pyridazin-2(1*H*)-yl]methyl}-1-pyrrolidinyl)propyl]-2-methylpropanamide;
*N*-[3-((2*R*)-2-{[4-[(4-chlorophenyl)methyl]-1-oxopyrido[3,4-*d*]pyridazin-2(1*H*)-yl]methyl}-1-pyrrolidinyl)propyl]-2,2-dimethylpropanamide;
4-[(4-Chlorophenyl)methyl]-2-({(2*R*)-1-[2-(3-methyl-1,2,4-oxadiazol-5-yl)ethyl]-2-pyrrolidinyl}methyl)pyrido[3,4-*d*]pyridazin-1(2*H*)-one;
4-[(4-Chlorophenyl)methyl]-2-({(2*R*)-1-[2-(3-ethyl-1,2,4-oxadiazol-5-yl)ethyl]-2-pyrrolidinyl} methyl)pyrido[3,4-*d*]pyridazin-1(2*H*)-one;
4-[(4-Chlorophenyl)methyl]-2-{[(2*R*)-1-(2-{3-[(methyloxy)methyl]-1,2,4-oxadiazol-5-yl}ethyl) -2-pyrrolidinyl]methyl}pyrido[3,4-*d*]pyridazin-1(2*H*)-one;
4-[(4-Chlorophenyl)methyl]-2-({(2*R*)-1-[2-(3-propyyl-1,2,4-oxadiazol-5-yl)ethyl]-2-pyrrolidinyl}methyl)pyrido[3,4-*d*]pyridazin-1(2*H*)-one;
or a salt thereof.

10. A compound according to any of claims 1 to 9 or a pharmaceutically acceptable salt thereof.

11. A compound according to any of claims 1 to 9 or a pharmaceutically acceptable salt thereof for use in therapy.

12. A compound according to any of claims 1 to 9 or a pharmaceutically acceptable salt thereof for use in the treatment of inflammatory and/or allergic disease.

13. A compound according to any of claims 1 to 9 or a pharmaceutically acceptable salt thereof for use in the treatment of allergic rhinitis.

14. A pharmaceutical composition which comprises a compound according to any of claims 1 to 9 or a pharmaceutically acceptable salt thereof, optionally with one or more pharmaceutically acceptable carriers and/or excipients.

15. A combination comprising a compound according to any of claims 1 to 9 or a pharmaceutically acceptable salt thereof and one or more other therapeutic compounds.

## Patentansprüche

1. Verbindung der Formel (I): 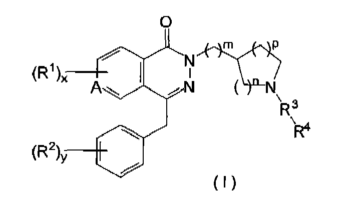 worin:
A N oder CH darstellt;
R¹ und R² jeweils unabhängig voneinander Halogen, C₁₋₃-Alkyl, C₁₋₃-Alkoxy, Hydroxy oder Trifluormethyl darstellen;
x und y jeweils unabhängig voneinander 0, 1 oder 2 darstellen;
m 0 darstellt, n 2 darstellt und p 1 darstellt; oder
m 1 darstellt, n 0 darstellt und p 2 darstellt;
R³ geradkettiges C₁₋₆-Alkylen darstellt, das gegebenenfalls mit ein oder zwei C₁₋₃-Alkylgruppen substituiert ist; und
R⁴ eine Gruppe der Formel (i), (ii), (iii) oder (iv) darstellt:
(i) -C(O)N(R⁵)R⁶, worin
R⁵ wasserstoff oder -C₁₋₆-Alkyl darstellt;
R⁶ Wasserstoff; C₁₋₆-Alkyl (gegebenenfalls substituiert mit bis zu drei Substituenten, unabhängig voneinander ausgewählt aus Halogen und Hydroxy); -C₁₋₆-Alkyl-O-C₁₋₆-alkyl (gegebenenfalls substituiert mit bis zu drei Substituenten, unabhängig voneinander ausgewählt aus Halogen und Hydroxy); C₃₋₇-Cycloalkyl (gegebenenfalls substituiert mit bis zu drei Substituenten, unabhängig voneinander ausgewählt aus Halogen, Hydroxy und C₁₋₃-Alkyl); -C₁₋₃-Alkyl-C₃₋₇-cycloalkyl (worin das C₁₋₃-Alkyl gegebenenfalls mit bis zu drei Substituenten, unabhängig voneinander ausgewählt aus Halogen und Hydroxy, substituiert ist und das C₃₋₇-Cycloalkyl gegebenenfalls mit bis zu drei Substituenten, unabhängig voneinander ausgewählt aus Halogen, Hydroxy und C₁₋₃-Alkyl, substituiert ist); Aryl (gegebenenfalls substituiert mit bis zu drei Substituenten, unabhängig voneinander ausgewählt aus Halogen, C₁₋₃-Alkyl, Trifluormethyl und Cyano); -C₁₋₆-Alkylaryl (worin das C₁₋₆-Alkyl gegebenenfalls mit bis zu drei Substituenten, unabhängig voneinander ausgewählt aus C₁₋₃-Alkyl, Halogen und Hydroxy, substituiert ist und das Aryl gegebenenfalls mit bis zu drei Substituenten, unabhängig voneinander ausgewählt aus Halogen, C₁₋₃-Alkyl, Trifluormethyl und Cyano, substituiert ist); oder -(C₁₋₃-Alkyl)_{z}Het darstellt, worin z 0 oder 1 darstellt und Het einen 5- bis 7-gliedrigen, gesättigten, heterocyclischen Ring mit ein oder zwei Heteroatomen, unabhängig voneinander ausgewählt aus O und S, darstellt;
oder R⁵ und R⁶ zusammen mit dem N-Atom, an das sie gebunden sind, einen 5- bis 7-gliedrigen, gesättigten, heterocyclischen Ring darstellen, der gegebenenfalls ein oder zwei weitere Heteroatome, unabhängig voneinander ausgewählt aus O und S, enthält;
(ii) -N(R⁷)C(O)R⁸, worin
R⁷ Wasserstoff oder -C₁₋₆-Alkyl darstellt; und
R⁸ -C₁₋₆-Alkyl (gegebenenfalls substituiert mit bis zu drei Substituenten, unabhängig voneinander ausgewählt aus Halogen und Hydroxy); -C₁₋₆-Alkyl-O-C₁₋₆-alkyl (gegebenenfalls substituiert mit bis zu drei Substituenten, unabhängig voneinander ausgewählt aus Halogen und Hydroxy); C₃₋₇-Cycloalkyl (gegebenenfalls substituiert mit bis zu drei Substituenten, unabhängig voneinander ausgewählt aus Halogen, Hydroxy und C₁₋₃-Alkyl); -C₁₋₃-Alkyl-C₃₋₇-cycloalkyl (worin das C₁₋₃-Alkyl gegebenenfalls mit bis zu drei Substituenten, unabhängig voneinander ausgewählt aus Halogen und Hydroxy, substituiert ist und das C₃₋₇-Cycloalkyl gegebenenfalls mit bis zu drei Substituenten, unabhängig voneinander ausgewählt aus Halogen, Hydroxy und C₁₋₃-Alkyl, substituiert ist); Aryl (gegebenenfalls substituiert mit bis zu drei Substituenten, unabhängig voneinander ausgewählt aus Halogen, C₁₋₃-Alkyl, Trifluormethyl und Cyano); -C₁₋₆-Alkylaryl (worin das C₁₋₆-Alkyl gegebenenfalls mit bis zu drei Substituenten, unabhängig voneinander ausgewählt aus C₁₋₃-Alkyl, Halogen und Hydroxy, substituiert ist und das Aryl gegebenenfalls mit bis zu drei Substituenten, unabhängig voneinander ausgewählt aus Halogen, C₁₋₃-Alkyl, Trifluormethyl und Cyano, substituiert ist); oder -(C₁₋₃-Alkyl)_{z}Het darstellt, worin z 0 oder 1 darstellt und Het einen 5- bis 7-gliedrigen heterocyclischen Ring mit ein oder zwei Heteroatomen, unabhängig voneinander ausgewählt aus O und S, darstellt; 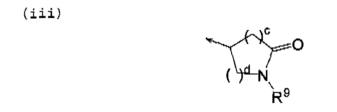 worin c 0 oder 1 darstellt und d 2 oder 3 darstellt, oder c 2 oder 3 darstellt und d 0 oder 1 darstellt; und
R⁹ Wasserstoff oder C₁₋₆-Alkyl darstellt; 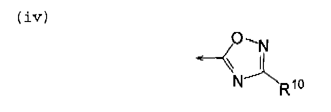 worin R¹⁰ Wasserstoff oder -C₁₋₆-Alkyl (gegebenenfalls substituiert mit bis zu drei Substituenten, unabhängig voneinander ausgewählt aus Halogen und Hydroxy); -C₁₋₆-Alkyl-O-C₁₋₆-alkyl (gegebenenfalls substituiert mit bis zu drei Substituenten, unabhängig voneinander ausgewählt aus Halogen und Hydroxy); Aryl (gegebenenfalls substituiert mit bis zu drei Substituenten, unabhängig voneinander ausgewählt aus Halogen, C₁₋₃-Alkyl, Trifluormethyl und Cyano); oder -C₁₋₆-Alkylaryl (worin das C₁₋₆-Alkyl gegebenenfalls mit bis zu drei Substituenten, unabhängig voneinander ausgewählt aus C₁₋₃-Alkyl, Halogen und Hydroxy,
substituiert ist und das Aryl gegebenenfalls mit bis zu drei Substituenten, unabhängig voneinander ausgewählt aus Halogen, C₁₋₃-Alkyl, Trifluormethyl und Cyano, substituiert ist) darstellt;
oder ein Salz davon.

2. Verbindung gemäss Anspruch 1, worin A CH darstellt.

3. Verbindung gemäss Anspruch 1 oder Anspruch 2, worin m 1 darstellt, n 0 darstellt und p 2 darstellt.

4. Verbindung gemäss irgendeinem der Ansprüche 1 bis 3, worin x 0 ist.

5. Verbindung gemäss irgendeinem der Ansprüche 1 bis 4, worin R² Halogen ist und y 1 ist.

6. Verbindung gemäss irgendeinem der Ansprüche 1 bis 5, worin R⁴ -C(O)N(R⁵)R⁶ darstellt.

7. Verbindung gemäss irgendeinem der Ansprüche 1 bis 5, worin R⁴ -N(R⁷)C(O)R⁸ darstellt.

8. Verbindung gemäss irgendeinem der Ansprüche 1 bis 5, worin R⁴ 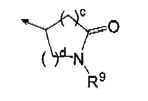 darstellt.

9. Verbindung, die ist:
3-((2R)-2-{[4-[(4-Chlorphenyl)methyl]-1-oxo-2(1H)-phthalazinyl]methyl}-1-pyrrolidinyl)-N-(2-fluorethyl)propanamid;
3-((2R)-2-{[4-[(4-Chlorphenyl)methyl]-1-oxo-2(1H)-phthalazinyl]methyl}-1-pyrrolidinyl)-2-methyl-N-[2-(methyloxy)ethyl]propanamid;
3-((2R)-2-{[4-[(4-Chlorphenyl)methyl]-1-oxo-2(1H)-phthalazinyl]methyl}-1-pyrrolidinyl)-N-(phenyl-methyl)propanamid;
3-((2R)-2-{[4-[(4-Chlorphenyl)methyl]-1-oxo-2(1H)-phthalazinyl]methyl}-1-pyrrolldinyl)-N-(2,2,2-trifluorethyl)propanamid;
3-((2R)-2-{[4-[(4-Chlorphenyl)methyl]-1-oxo-2(1H)-phthalazinyl]methyl}-1-pyrrolidinyl)-N-(2,2,2-trifluorethyl)propanamid;
3-((2R)-2-{[4-[(4-Chlorphenyl)methyl]-1-oxo-2(1H)-phthalazinyl]methyl}-1-pyrrolidinyl)-N-(2,2-difluorethyl)propanamid;
3-((2R)-2-{[4-[(4-Chlorphenyl)methyl]-1-oxo-2(1H)-phthalazinyl]methyl}-1-pyrrolidinyl)-N-(2,2-difluorethyl)propanamid;
3-((2R)-2-{[4-[(4-Chlorphenyl)methyl]-1-oxo-2(1H)-phthalazinyl]methyl}-1-pyrrolidinyl)-N-(2-fluorethyl)propanamid;
3-((2R)-2-{[4-[(4-Chlorphenyl)methyl]-1-oxo-2(1H)-phthalazinyl]methyl}-1-pyrrolidinyl)-N-methylpropanamid;
3-((2R)-2-{[4-[(4-Chlorphenyl)methyl]-1-oxo-2(1H)-phthalazinyl]methyl}-1-pyrrolidinyl)-propanamid;
3-((2R)-2-{[4-[(4-Chlorphenyl)methyl]-1-oxo-2(1H)-phthalazinyl]methyl}-1-pyrrolidinyl)-N-ethyl-N-methylpropanamid;
4-[(4-Chlorphenyl)methyl]-2-({(2R)-1-[3-oxo-3-(1-piperidinyl)propyl]-2-pyrrolidinyl}methyl)-1(2H)-phthalazinon;
4-[(4-Chlorphenyl)methyl]-2-({(2R)-1-[3-(4-morpholinyl)-3-oxopropyl]-2-pyrrolidinyl}methyl)-1(2H)-phthalazinon;
3-((2R)-2-{[4-[(4-Chlorphenyl)methyl]-1-oxo-2(1H)-phthalazinyl]methyl}-1-pyrrolidinyl)-N-methyl-N-[2-(methyloxy)ethyl]propanamid;
3-((2R)-2-{[4-[(4-Chlorphenyl)methyl]-1-oxo-2(1H)-phthalazinyl]methyl}-1-pyrrolidinyl)-N-{2-[(2,2,2-trifluorethyl)oxy]ethyl}propanamid;
3-((2R)-2-{[4-[(4-Chlorphenyl)methyl]-1-oxo-2(1H)-phthalazinyl]methyl}-1-pyrrolidinyl)-N-ethylpropanamid;
3-((2R)-2-{[4-[(4-Chlorphenyl)methyl]-1-oxo-2(1H)-phthalazinyl]methyl}-1-pyrrolidinyl)-N-ethylpropanamid;
3-((2R)-2-{[4-[(4-Chlorphenyl)methyl]-1-oxo-2(1H)-phthalazinyl]methyl}-1-pyrrolidinyl)-N-(1-methylethyl)propanamid;
3-((2R)-2-{[4-[(4-Chlorphenyl)methyl]-1-oxo-2(1H)-phthalazinyl]methyl}-1-pyrrolidinyl)-N-propylpropanamid;
3-((2R)-2-{[4-[(4-Chlorphenyl)methyl]-1-oxo-2(1H)-phthalazinyl]methyl}-1-pyrrolidinyl)-N-(1,1-dimethylethyl)propanamid;
3-((2R)-2-{[4-[(4-Chlorphenyl)methyl]-1-oxo-2(1H)-phthalazinyl]methyl}-1-pyrrolidinyl)-N-[2-(methyl-oxy)ethyl]propanamid;
3-((2R)-2-{[4-[(4-Chlorphenyl)methyl]-1-oxo-2(1H)-phthalazinyl]methyl}-1-pyrrolidinyl)-N-[2-(ethyl-oxy)ethyl]propanamid;
3-((2R)-2-{[4-[(4-Chlorphenyl)methyl]-1-oxo-2(1H)-phthalazinyl]methyl}-1-pyrrolidinyl)-N-[3-(methyloxy)propyl]propanamid;
4-((2R)-2-{[4-[(4-Chlorphenyl)methyl]-1-oxo-2(1H)-phthalazinyl]methyl}-1-pyrrolidinyl)-N-[2-methyl-oxy)ethyl]butanamid;
4-((2R)-2-{[4-[(4-Chlorphenyl)methyl]-1-oxo-2(1H)-phthalazinyl]methyl}-1-pyrrolidinyl)-N-[2-(methyl-oxy)ethyl]butanamid;
4-((2R)-2-{[4-[(4-Chlorphenyl)methyl]-1-oxo-2(1H)-phthalazinyl]methyl}-1-pyrrolidinyl)-N-propylbutan-amid;
2-((2R)-2-{[4-[(4-Chlorphenyl)methyl]-1-oxo-2(1H)-phthalazinyl]methyl}-1-pyrrolidinyl)-N-[3-(methyl-oxy)propyl]acetamid;
N-Butyl-2-((2R)-2-{[4-[(4-chlorphenyl)methyl]-1-oxo-2(1H)-phthalazinyl]methyl}-1-pyrrolidinyl)acetamid;
4-[(4-Chlorphenyl)methyl]-2-({(2R)-1-[2-oxo-2-(1-piperidinyl)ethyl]-2-pyrrolidinyl}methyl)-1(2H)-phthalazinon;
2-((2R)-2-{[4-[(4-Chlorphenyl)methyl]-1-oxo-2(1H)-phthalazinyl]methyl}-1-pyrrolidinyl)-N-(1,1-dimethylethyl)acetamid;
3-{4-[4-[(4-Chlorphenyl)methyl]-1-oxo-2(1H)-phthalazinyl]-1-piperidinyl}-N-[2-(methyloxy)ethyl]-propanamid;
3-{4-[4-[(4-Chlorphenyl)methyl]-l-oxo-2(1H) - phthalazinyl]-1-piperidinyl}-N-[3-(methyloxy)propyl]-propanamid;
3-{4-[4-[(4-Chlorphenyl)methyl]-1-oxo-2(1H)-phthalazinyl]-1-piperidinyl}-N-ethylpropanamid;
N-[2-((2R)-2-{[4-[(4-Chlorphenyl)methyl]-1-oxo-2(1H)-phthalazinyl]methyl}-1-pyrrolidinyl)-ethyl]-4-(methyloxy)butanamid;
N-[2-((2R)-2-{[4-[(4-Chlorphenyl)methyl]-1-oxo-2(1H)-phthalazinyl]methyl}-1-pyrrolidinyl)-ethyl]-3-(methyloxy)propanamid;
N-[2-((2R)-2-{[4-[(4-Chlorphenyl)methyl]-1-oxo-2(1H)-phthalazinyl]methyl}-1-pyrrolidinyl)-ethyl]-3,3,3-trifluorpropanamid;
N-[2-((2R)-2-{[4-[(4-Chlorphenyl)methyl]-1-oxo-2(1H)-phthalazinyl]methyl}-1-pyrrolidinyl)-ethyl]butanamid;
N-[2-((2R)-2-{[4-[(4-Chlorphenyl)methyl]-1-oxo-2(1H)-phthalazinyl]methyl}-1-pyrrolidinyl)-ethyl]-2,2-dimethylpropanamid;
N-[2-((2R)-2-{[4-[(4-Chlorphenyl)methyl]-1-oxo-2(1H)-phthalazinyl]methyl}-1-pyrrolidinyl)-ethyl]-2-(methyloxy)acetamid;
N-[2-((2R)-2-{[4-[(4-Chlorphenyl)methyl]-1-oxo-2(1H)-phthalazinyl]methyl}-1-pyrrolidinyl)-ethyl]propanamid;
N-[2-((2R)-2-{[4-[(4-Chlorphenyl)methyl]-1-oxo-2(1H)-phthalazinyl]methyl}-1-pyrrolidinyl)-ethyl]-2-methylpropanamid;
N-[3-((2R)-2-{[4-[(4-Chlorphenyl)methyl]-1-oxo-2(1H)-phthalazinyl]methyl}-1-pyrrolidinyl)propyl]-3-(methyloxy)propanamid;
N-[3-((2R)-2-{[4-[(4-Chlorphenyl)methyl]-1-oxo-2(1H)-phthalazinyl]methyl}-1-pyrrolidinyl)propyl]acetamid;
N-[3-((2R)-2-{[4-[(4-Chlorphenyl)methyl]-1-oxo-2(1H)-phthalazinyl]methyl}-1-pyrralidinyl)propyl]propanamid;
N-[3-((2R)-2-{[4-[(4-Chlorphenyl)methyl]-1-oxo-2(1H)-phthalazinyl]methyl}-1-pyrrolidinyl)propyl]-2-methylpropanamid;
N-[3-((2R)-2-{[4-[(4-Chlorphenyl)methyl]-1-oxo-2(1H)-phthalazinyl]methyl}-1-pyrrolidinyl)propyl]-2,2-dimethylpropanamid;
N-[2-((2R)-2-{[4-[(4-Chlorphenyl)methyl]-1-oxo-2(1H)-phthalazinyl]methyl}-1-pyrrolidinyl)ethyl]-2,2,2-trifluoracetamid;
N-[2-((2R)-2-{[4-[(4-Chlorphenyl)methyl]-1-oxo-2(1H)-phthalazinyl]methyl}-1-pyrrolidinyl)ethyl]-2,2,2-trifluor-N-methylacetamid;
N-[2-((2R)-2-{[4-[(4-Chlorphenyl)methyl]-1-oxo-2(1H)-phthalazinyl]methyl}-1-pyrrolidinyl)ethyl]-N-methyl-4-(methyloxy)butanamid;
N-[2-((2R)-2-{[4-[(4-Chlorphenyl)methyl]-1-oxo-2(1H)-phthalazinyl]methyl}-1-pyrrolidinyl)ethyl]cyclo-pentancarboxamid;
N-[2-((2R)-2-{[4-[(4-Chlorphenyl)methyl]-1-oxo-2(1H)-phthalazinyl]methyl}-1-pyrrolidinyl)ethyl]tetrahydro-2H-pyran-4-carboxamid;
N-[2-((2R)-2-{[4-[(4-Chlorphenyl)methyl]-1-oxo-2(1H)-phthalazinyl]methyl}-1-pyrrolidinyl)ethyl]-3-hydroxy-2,2-dimethylpropanamid;
N-[2-((2R)-2-{[4-[(4-Chlorphenyl)methyl]-1-oxo-2(1H)-phthalazinyl]methyl}-1-pyrrolidinyl)ethyl]tetrahydro-2H-pyran-3-carboxamid;
N-[2-((2R)-2-{[4-[(4-Chlorphenyl)methyl]-1-oxo-2(1H)-phthalazinyl]methyl}-1-pyrrolidinyl)ethyl]tetrahydro-3-furancarboxamid;
N-[2-((2R)-2-{[4-[(4-Chlorphenyl)methyl]-1-oxo-2(1H)-phthalazinyl]methyl}-1-pyrrolidinyl)ethyl]cyclohexan-carboxamid;
2-((2R)-2-{[4-[(4-Chlorphenyl)methyl]-1-oxo-2(1H)-phthalazinyl]methyl}-1-pyrrolidinyl)-N-(2,2-dimethylpropyl)acetamid;
2-((2R)-2-{[4-[(4-Chlorphenyl)methyl]-1-oxo-2(1H)-phthalazinyl]methyl}-1-pyrrolidinyl)-N-(cyclohexylmethyl)acetamid;
4-[(4-Chlorphenyl)methyl]-2-({(2R)-1-[(2-oxo-3-piperidinyl)methyl]-2-pyrrolidinyl}methyl)-1(2H)-phthalazinon;
4-[(4-Chlorphenyl)methyl]-2-({(2R)-1-(2-{3-[2-(methyloxy)ethyl]-1,2,4-oxadiazol-5-yl}ethyl)-2-pyrrolidinyl]methyl}-1(2H)-phthalazinon;
4-[(4-Chlorphenyl)methyl]-2-({(2R)-1-[(3-propyl-1,2,4-oxadiazol-5-yl)methyl]-2-pyrrolidinyl}methyl)-1(2H)-phthalazinon;
4-[(4-Chlorphenyl)methyl]-2-({(2R)-1-{2-[3-(1-methylethyl)-1,2,4-oxadiazol-5-yl]ethyl}-2-pyrrolidinyl)methyl]-1(2H)-phthalazinon;
4-[(4-Chlorphenyl)methyl]-2-({(2R)-1-[2-(3-propyl-1,2,4-oxadiazol-5-yl)ethyl]-2-pyrrolidinyl}methyl)-1(2H)-phthalazinon;
3-((2R)-2-{[4-[(4-Chlorphenyl)methyl]-1-oxopyrido-[3,4-d]pyridazin-2(1H)-yl]methyl}-1-pyrrolidinyl)-N-[3-(methyloxy)propyl]propanamid;
3-((2R)-2-{[4-[(4-Chlorphenyl)methyl]-1-oxopyrido-[3,4-d]pyridazin-2(1H)-yl]methyl}-1-pyrrolidinyl)-N-(2,2-difluorethyl)propanamid;
3-((2R)-2-{[4-[(4-Chlorphenyl)methyl]-1-oxopyrido-[3,4-d]pyridazin-2(1H)-yl]methyl}-1-pyrrolidinyl)-N-propylpropanamid;
3-((2R)-2-{[4-[(4-Chlorphenyl)methyl]-1-oxopyrido-[3,4-d]pyridazin-2(1H)-yl]methyl}-1-pyrrolidinyl)-N-ethyl-N-methylpropanamid;
4-[(4-Chlorphenyl)methyl]-2-({(2R)-1-[3-oxo-3-(1-piperidinyl)propyl]-2-pyrrolidinyl}methyl)pyrido-[3,4-d]pyridazin-1(2H)-on;
3-((2R)-2-{[4-[(4-Chlorphenyl)methyl]-1-oxopyrido-[3,4-d]pyridazin-2(1H)-yl]methyl}-1-pyrrolidinyl)-N-(2,2,2-trifluorethyl)propanamid;
N-[2-((2R)-2-{[4-[(4-Chlorphenyl)methyl]-1-oxopyrido-[3,4-d]pyridazin-2(1H)-yl]methyl}-1-pyrrolidinyl)-ethyl]-3-(methyloxy)propanamid;
N-[2-((2R)-2-{[4-[(4-Chlorphenyl)methyl]-1-oxopyrido-[3,4-d]pyridazin-2(1H)-yl]methyl}-1-pyrrolidinyl)-ethyl]-4-(methyloxy)butanamid;
N-[3-((2R)-2-{[4-[(4-Chlorphenyl)methyl]-1-oxopyrido-[3,4-d]pyridazin-2(1H)-yl]methyl}-1-pyrrolidinyl)-propyl]-3-(methyloxy)propanamid;
((2R)-2-{[4-[(4-Chlorphenyl)methyl]-1-oxopyrido-[3,4-d]pyridazin-2(1H)-yl]methyl}-1-pyrrolidinyl)-propyl]-4-(methyloxy)butanamid;
N-[2-((2R)-2-{[4-[(4-Chlorphenyl)methyl]-1-oxopyrido-[3,4-d]pyridazin-2(1H)-yl]methyl}-1-pyrrolidinyl)-ethyl]propanamid;
N-[2-((2R)-2-{[4-[(4-Chlorphenyl)methyl]-1-oxopyrido-[3,4-d]pyridazin-2(1H)-yl]methyl}-1-pyrrolidinyl)-ethyl]butanamid;
N-[2-((2R)-2-{[4-[(4-Chlorphenyl)methyl]-1-oxopyrido-[3,4-d]pyridazin-2(1H)-yl]methyl}-1-pyrrolidinyl)-ethyl]-2,2-dimethylpropanamid;
N-[2-((2R)-2-{[4-[(4-Chlorphenyl)methyl]-1-oxopyrido-[3,4-d]pyridazin-2(1H)-yl]methyl-1-pyrrolidinyl)-ethyl]-2-methylpropanamid;
N-[3-((2R)-2-{[4-[(4-Chlorphenyl)methyl]-1-oxopyrido-[3,4-d]pyridazin-2(1H)-yl]methyl}-1-pyrrolidinyl)-propyl]-propanamid;
N-[3-((2R)-2-{[4-[(4-Chlorphenyl)methyl]-1-oxopyrido-[3,4-d]pyridazin-2(1H)-yl]methyl}-1-pyrrolidinyl)-propyl]-propanamid;
N-[3-((2R)-2-{[4-[(4-Chlorphenyl)methyl]-1-oxopyrido-[3,4-d]pyridazin-2(1H)-yl]methyl}-1-pyrrolidinyl)-propyl]-2-methylpropanamid;
N-[3-((2R)-2-{[4-[(4-Chlorphenyl)methyl]-1-oxopyrido-[3,4-d]pyridazin-2(1H)-yl]methyl}-1-pyrrolidinyl)-propyl]-2,2-dimethylpropanamid;
4-[(4-Chlorphenyl)methyl]-2-({(2R)-1-[2-(3-methyl-1,2,4-oxadiazol-5-yl)ethyl]-2-pyrrolidinyl}methyl)-pyrido[3,4-d]pyridazin-1(2H)-on;
4-[(4-Chlorphenyl)methyl]-2-({(2R)-1-[2-(3-ethyl-1,2,4-oxadiazol-5-yl)ethyl]-2-pyrrolidinyl}methyl)-pyrido[3,4-d]pyridazin-1(2H)-on;
4-[(4-Chlorphenyl)methyl]-2-({(2R)-1-(2-{3-[(methyloxy)methyl]-1,2,4-oxadiazol-5-yl}ethyl)-2-pyrrolidinyl]methyl}pyrido[3,4-d]pyridazin-1(2H)-on;
4-[(4-Chlorphenyl)methyl]-2-({(2R)-1-[2-(3-pxopyl-1,2,4-oxadiazol-5-yl)ethyl]-2-pyrrolidinyl}methyl)-pyrido[3,4-d]pyridazin-1(2H)-on;
oder ein Salz davon.

10. Verbindung gemäss irgendeinem der Ansprüche 1 bis 9 oder ein pharmazeutisch annehmbares Salz davon.

11. Verbindung gemäss irgendeinem der Ansprüche 1 bis 9 oder ein pharmazeutisch annehmbares Salz davon zur Verwendung in der Therapie.

12. Verbindung gemäss irgendeinem der Ansprüche 1 bis 9 oder ein pharmazeutisch annehmbares Salz davon zur Verwendung bei der Behandlung von entzündlichen und/oder allergischen Erkrankungen.

13. Verbindung gemäss irgendeinem der Ansprüche 1 bis 9 oder ein pharmazeutisch annehmbares Salz davon zur Verwendung bei der Behandlung von allergischer Rhinitis.

14. Pharmazeutische Zusammensetzung, umfassend eine Verbindung gemäss irgendeinem der Ansprüche 1 bis 9 oder ein pharmazeutisch annehmbares Salz davon, gegebenenfalls mit ein oder mehreren pharmazeutisch annehmbaren Trägern und/oder Exzipienten.

15. Kombination, umfassend eine Verbindung gemäss irgendeinem der Ansprüche 1 bis 9 oder ein pharmazeutisch annehmbares Salz davon und ein oder mehrere andere therapeutische Verbindungen.

## Revendications

1. Composé de formule (I) 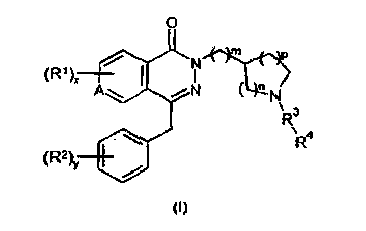 dans laquelle :
A représente N ou CH ;
R¹ et R² représentent chacun indépendamment un atome d'halogène, un groupe alkyle en C₁ à C₃, alcoxy en C₁ à C₃, hydroxy ou trifluorométhyle ;
x et y représentent chacun indépendamment 0, 1 ou 2 ;
m représente 0, n représente 2 et p représente 1 ; ou
m représente 1, n représente 0 et p représente 2 ;
R³ représente un groupe alkylène en C₁ à C₆ à chaîne linéaire éventuellement substitué par un ou deux groupes alkyle en C₁ à C₃ ; et
R⁴ représente un groupe de formule (i), (ii), (iii) ou (iv)
(i) -C(O)N(R⁵)R⁶ dans laquelle
R⁵ représente un atome d'hydrogène ou un groupe alkyle en C₁ à C₆ ;
R⁶ représente un atome d'hydrogène ; un groupe alkyle en C₁ à C₆ (éventuellement substitué par jusqu'à trois substituants choisis indépendamment parmi un atome d'halogène et un groupe hydroxy) ; -alkyl en C₁ à C₆-O-alkyle en C₁ à C₆ (éventuellement substitué par jusqu'à trois substituants choisis indépendamment parmi un atome d'halogène et un groupe hydroxy) ; cycloalkyle en C₃ à C₇ (éventuellement substitué par jusqu'à trois substituants choisis indépendamment parmi un atome d'halogène, un groupe hydroxy et alkyle en C₁ à C₃) ; -alkyl en C₁ à C₃-cycloalkyle en C₃ à C₇ (dans lequel le groupe alkyle en C₁ à C₃ est éventuellement substitué par jusqu'à trois substituants choisis indépendamment parmi un atome d'halogène et un groupe hydroxy, et le groupe cycloalkyle en C₃ à C₇ est éventuellement substitué par jusqu'à trois substituants choisis indépendamment parmi un atome d'halogène, un groupe hydroxy et alkyle en C₁ à C₃) ; aryle (éventuellement substitué par jusqu'à trois substituants choisis indépendamment parmi un atome d'halogène, un groupe alkyle en C₁ à C₃, trifluorométhyle et cyano) ; -alkyle en C₁ à C₆-aryle (dans lequel le groupe alkyle en C₁ à C₆ est éventuellement substitué par jusqu'à trois substituants choisis indépendamment parmi un groupe alkyle en C₁ à C₃, un atome d'halogène et un groupe hydroxy, et le groupe aryle est éventuellement substitué par jusqu'à trois substituants choisis indépendamment parmi un atome d'halogène, un groupe alkyle en C₁ à C₃, trifluorométhyle et cyano) ; ou -(alkyl en C₁ à C₃)_{z}Het, dans lequel z représente 0 ou 1 et Het représente un cycle hétérocyclique saturé de 5 à 7 chaînons contenant un ou deux hétéroatomes choisis indépendamment parmi O et S ;
ou R⁵ et R⁶ conjointement avec l'atome N auquel ils sont fixés représentent un cycle hétérocyclique saturé de 5 à 7 chaînons contenant éventuellement un ou deux autres hétéroatomes choisis indépendamment parmi O et S ;
(ii) -N(R⁷)C(O)R⁸ dans laquelle
R⁷ représente un atome d'hydrogène ou un groupe -alkyle en C₁ à C₆ ; et
R⁸ représente un groupe -alkyle en C₁ à C₆ (éventuellement substitué par jusqu'à trois substituants choisis indépendamment parmi un atome d'halogène et un groupe hydroxy) ; -alkyl en C₁ à C₆-O-alkyle en C₁ à C₆ (éventuellement substitué par jusqu'à trois substituants choisis indépendamment parmi un atome d'halogène et un groupe hydroxy) ; cycloalkyle en C₃ à C₇ (éventuellement substitué par jusqu'à trois substituants choisis indépendamment parmi un atome d'halogène, un groupe hydroxy et alkyle en C₁ à C₃) ; -alkyl en C₁ à C₃-cycloalkyle en C₃ à C₇ (dans lequel le groupe alkyle en C₁ à C₃ est éventuellement substitué par jusqu'à trois substituants choisis indépendamment parmi un atome d'halogène et un groupe hydroxy, et le groupe cycloalkyle en C₃ à C₇ est éventuellement substitué par jusqu'à trois substituants choisis indépendamment parmi un atome d'halogène, un groupe hydroxy et alkyle en C₁ à C₃) ; aryle (éventuellement substitué par jusqu'à trois substituants choisis indépendamment parmi un atome d'halogène, un groupe alkyle en C₁ à C₃, trifluorométhyle et cyano) ; -alkyl en C₁ à C₆-aryle (dans -lequel le groupe alkyle en C₁ à C₆ est éventuellement substitué par jusqu'à trois substituants choisis indépendamment parmi un groupe alkyle en C₁ à C₃, un atome d'halogène et un groupe hydroxy, et le groupe aryle est éventuellement substitué par jusqu'à trois substituants choisis indépendamment parmi un atome d'halogène, un groupe alkyle en C₁ à C₃, trifluorométhyle et cyano) ; ou -(alkyl en C₁ à C₃)_{z}Het, dans lequel z représente 0 ou 1 et Het représente un cycle hétérocyclique saturé de 5 à 7 chaînons contenant un ou deux hétéroatomes choisis indépendamment parmi O et S ; 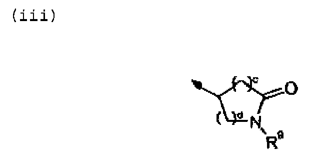 dans laquelle c représente 0 ou 1 et d représente 2 ou 3 ou c représente 2 ou 3 et d représente 0 ou 1 ; et
R⁹ représente un atome d'hydrogène ou un groupe alkyle en C₁ à C₆ ; 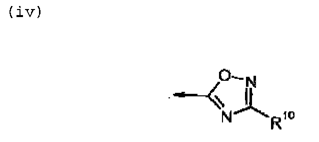
dans laquelle R¹⁰ représente un atome d'hydrogène ou un groupe -alkyle en C₁ à C₆ (éventuellement substitué par jusqu'à trois substituants choisis indépendamment parmi un atome d'halogène et un groupe hydroxy) ; -alkyl en C₁ à C₆-O-alkyle en C₁ à C₆ (éventuellement substitué par jusqu'à trois substituants choisis indépendamment parmi un atome d'halogène et un groupe hydroxy) ; aryle (éventuellement substitué par jusqu'à trois substituants choisis indépendamment parmi un atome d'halogène, un groupe alkyle en C₁ à C₃, trifluorométhyle et cyano) ; -alkyl en C₁ à C₆-aryle (dans lequel le groupe alkyle en C₁ à C₆ est éventuellement substitué par jusqu'à trois substituants choisis indépendamment parmi un groupe alkyle en C₁ à C₃, un atome d'halogène et un groupe hydroxy, et le groupe aryle est éventuellement substitué par jusqu'à trois substituants choisis indépendamment parmi un atome d'halogène, un groupe alkyle en C₁ à C₃, trifluorométhyle et cyano) ;
ou un sel de celui-ci.

2. Composé selon la revendication 1, dans lequel A représente CH.

3. Composé selon la revendication 1 ou la revendication 2, dans lequel m représente 1, n représente 0 et p représente 2.

4. Composé selon l'une quelconque des revendications 1 à 3, dans lequel x vaut 0.

5. Composé selon l'une quelconque des revendications 1 à 4, dans lequel R² représente un atome d'halogène et y vaut 1.

6. Composé selon l'une quelconque des revendications 1 à 5, dans lequel R⁴ représente -C(O)N(R⁵)R⁶.

7. Composé selon l'une quelconque des revendications 1 à 5, dans lequel R⁴ représente -N(R⁷)C(O)R⁸.

8. Composé selon l'une quelconque des revendications 1 à 5, dans lequel R⁴ représente

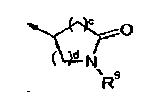

9. Composé qui est
le 3-((2*R*)-2-{[4-[(4-chlorophênyl)méthyl]-1-oxo-2(1*H*)-phtalazinyl]méthyl}-1-pyrrolidinyl)-*N*-(2-fluoro-éthyl)propanamide ;
le 3-((2-*R*)-2-{[4-[(4-chlorophényl)méthyl]-1-oxo-2(1*H*)-phtalazinyl]méthyl}-1-pyrrolidinyl)-2-méthyl-*N-*[2-(méthyloxy)éthyl]propanamide ;
le 3-((2*R*)-2-{[4-[(4-chlorophényl)méthyl]-1-oxo-2(1*H*)-phtalazinyl]méthyl}-1-pyrrolidinyl)-*N*-(phényl-méthyl)propanamide ;
le 3-((2*R*)-2-{[4-[(4-chlorophényl)méthyl]-1-oxo-2(1*H*)-phtalazinyl]méthyl}-1-pyrrolidinyl)-*N*-(2,2,2-trifluoroéthyl)propanamide ;
le 3-((2*R*)-2-{[4-[(4-chlorophényl)méthyl]-1-oxo-2(1*H*)-phtalazinyl]méthyl}-1-pyrrolidinyl)-*N*-(2,2,2-trifluoroéthyl)propanamide ;
le 3-((2*R*)-2-{[4-[(4-chlorophényl)méthyl]-1-oxo-2(1*H*)-phtalazinyl]méthyl}-1-pyrrolidinyl)-*N*-(2,2-difluoroéthyl)propanamide ;
le 3-((2*R*)-2-{[4-[(4-chlorophényl)méthyl]-1-oxo-2(1*H*)-phtalazinyl]méthyl}-1-pyrrolidinyl)-*N*-(2,2-difluoroéthyl)propanamide ;
le 3-((2*R*)-2-{[4-[(4-chlorophényl)méthyl]-1-oxo-2(1*H*)-phtalazinyl]méthyl}-1-pyrrolidinyl)-*N*-(2-fluoro-éthyl)propanamide ;
le 3-((2*R*)-2-{[4-[(4-chlorophênyl)méthyl]-1-oxo-2(1*H*)-phtalazinyl]méthyl}-1-pyrrolidinyl)-*N*-méthylpropanamide ;
le 3-((2*R*)-2-{[4-[(4-chlorophényl)méthyl]-1-oxo-2(1*H*)-phtalazinyl]méthyl}-1-pyrrolidinyl)propanamide ;
le 3-((2*R*)-2-{[4-[(4-chlorophényl)méthyl]-1-oxo-2(1*H*)-phtalazinyl]méthyl}-1-pyrrolidinyl)-*N*-éthyl-*N-*méthylpropanamide ;
la 4-[(4-chlorophényl)méthyl]-2-({(2*R*)-1-[3-oxo-3-(1-pipéridinyl)propyl]-2-pyrrolidinyl}mëthyl)-1(2*H*)-phtalazinone ;
la 4-[(4-chlorophényl)méthyl]-2-({(2*R*)-1-[3-(4-morpholinyl)-3-oxopropyl]-2-pyrrolidinyl}méthyl)-1(2*H*)-phtalazinone ;
le 3-((2*R*)-2-{[4-[(4-chlorophényl)méthyl]-1-oxo-2(1*H*)-phtalazinyl]méthyl}-1-pyrrolidinyl)-*N*-méthyl-*N-*[2-(méthyloxy)éthyl]propanamide ;
le 3-((2*R*)-2-{[4-[(4-chlorophényl)méthyl]-1-oxo-2(1*H*)-phtalazinyl]méthyl}-1-pyrrolidinyl)-*N*-{2-[(2,2,2-trifluoroéthyl)oxy]éthyl}propanamide ;
le 3-((2*R*)-2-{[4-[(4-chlorophényl)méthyl]-1-oxo-2(1*H*)-phtalazinyl]méthyl}-1-pyrrolidinyl)-*N*-éthyl-propanamide ;
le 3-((2*R*)-2-{[4-[(4-chlorophényl)méthyl]-1-oxo-2(1*H*)-phtalazinyl]méthyl}-1-pyrrolidinyl)-*N*-éthyl-propanamide ;
le 3-((2*R*)-2-{[4-[(4-chlorophényl)méthyl]-1-oxo-2(1*H*)-phtalazinyl]méthyl}-1-pyrrolidinyl)-*N*-(1-méthyl-éthyl)propanamide ;
le 3-((2*R*)-2-{[4-[(4-chlorophényl)méthyl]-1-oxo-2(1*H*)-phtaiazinyl]méthyl}-1-pyrroiidinyl)-*N*-propylpropanamide ;
le 3-((2*R*)-2-{[4-[(4-chlorophényl)méthyl]-1-oxo-2(1*H*)-phtalazinyl]méthyl}-1-pyrrolidinyl)-*N*-(1,1-diméthyléthyl)propanamide ;
le 3-((2*R*)-2-{[4-[(4-chlorophényl)méthyl]-1-oxo-2(1*H*)-phtalazinyl]méthyl}-1-pyrrolidinyl)-*N*-[2-(méthyloxy)éthyl]propanamide ;
le 3-((2*R*)-2-{[4-[(4-chlorophényl)méthyl]-1-oxo-2(1*H*)-phtalazinyl]méthyl}-1-pyrrolidinyl)-*N*-[2-(éthyl-oxy)éthyl]propanamide ;
le 3-((2*R*)-2-{[4-[(4-chlorophényl)méthyl]-1-oxo-2(1*H*)-phtalazinyl]méthyl}-1-pyrrolidinyl)-*N*-[3-(méthyl-oxy)propyl]propanamide ;
le 4-((2*R*)-2-{[4-[(4-chlorophényl)méthyl]-1-oxo-2(1*H*)-phtalazinyl]méthyl}-1-pyrrolidinyl)-*N*-[2-(méthyl-oxy)éthyl]butanamide ;
le 4-((2*R*)-2-{[4-{(4-chlorophényl)méthyl)-1-oxo-2(1*H*)-phtalazinyl]méthyl}-1-pyrrolidinyl)-*N*-[2-(méthyl-oxy)éthyl]butanamide ;
le 4-((2*R*)-2-{[4-[(4-chlorophényl)méthyl]-1-oxo-2(1*H*)-phtalazinyl]méthyl}-1-pyrrolidinyl)-*N*-propylbutanamide ;
le 2-((2*R*)-2-{[4-[(4-chlorophényl)méthyl]-1-oxo-2(1*H*)-phtalazinyl]méthyl}-1-pyrrolidinyl)-*N*-[3-(méthyl-oxy)propyl]acétamide ;
le *N*-butyl-2-((2*R*)-2-{[4-[(4-chlorophényl)méthyl]-1-oxo-2(1*H*)-phtalazinyl]méthyl}-1-pyrrolidinyl)-acétamide ;
la 4-[(4-chlorophényl)méthyl]-2-({(2*R*)-1-[2-oxo-2-(1-pipéridinyl)éthyl]-2-pyrrolidinyl}méthyl)-1(2*H*)-phtalazinone ;
le 2-((2*R*)-2-{[4-[(4-chlorophényl)méthyl]-1-oxo-2(1*H*)-phtalazinyl]méthyl}-1-pyrrolidinyl)-*N*-(1,1-diméthyléthyl)acétamide ;
le 3-{4-[4-[(4-chlorophényl)méthyl]-1-oxo-2(1*H*)-phtalazinyl]-1-pipéridinyl}-*N*-[2-(méthyloxy)éthyl]-propanamide ;
le 3-{4-[4-[(4-chlorophényl)méthyl]-1-oxo-2(1*H*)-phtalazinyl]-1-pipéridinyl}-*N*-[3-(méthyloxy)propyl]-propanamide ;
le 3-{4-[4-[(4-chlorophényl)méthyl]-1-oxo-2(1*H*)-phtalazinyl]-1-pipéridinyl}-*N*-éthylpropanamide ;
le *N*-[2-((2*R*)-2-{[4-[(4-chlorophényl)méthyl]-1-oxo-2(1*H*)-phtalazinyl]méthyl}-1-pyrrolidinyl)éthyl]-4-(méthyloxy)butanamide ;
le *N*-[2-((2*R*)-2-{[4-[(4-chlorophényl)méthyl]-1-oxo-2(1*H*)-phtalazinyl]méthyl}-1-pyrrolidinyl)éthyl]-3-(méthyloxy)propanamide ;
le N-[2-((2*R*)-2-{[4-[(4-chlorophényl)méthyl]-1-oxo-2(1*H*)-phtalazinyl]méthyl}-1-pyrrolidinyl)éthyl]-3,3,3-trifluoropropanamide ;
le *N*-[2-((2*R*)-2-{[4-[(4-chlorophényl)méthyl]-1-oxo-2(1*H*)-phtaiazinyl]méthyl}-1-pyrrolidinyl)éthyl]-butanamide ;
le *N*-[2-((2*R*)-2-{[4-[(4-chlorophényl)méthyl]-1-oxo-2(1*H*)-phtalazinyl]méthyl}-1-pyrrolidinyl)éthyl]-2,2-diméthylpropanamide ;
le *N*-[2-((2*R*)-2-{[4-[(4-chlorophényl)méthyl]-1-oxo-2(1*H*)-phtalazinyl]méthyl}-1-pyrrolidinyl)éthyl]-2-(méthyloxy)acétamide ;
le *N*-[2-((2*R*)-2-{[4-[(4-chlorophényl)méthyl]-1-oxo-2(1*H*)-phtalazinyl]méthyl}-1-pyrrolidinyl)éthyl]-propanamide ;
le *N*-[2-((2*R*)-2-{[4-[(4-chlorophényl)méthyl]-1-oxv-2(1*H*)-phtalazinyl]méthyl}-1-pyrrolidinyl)éthyl]-2-méthylpropanamide ;
le *N*-[3-((2*R*)-z-{[4-[(4-chlorophényl)méthyl]-1-oxo-2(1*H*)-phtalazinyl]méthyl}-1-pyrrolidinyl)propyl]-3-(méthyloxy)propanamide ;
le *N*-[3-((2*R*)-2-{[4-[(4-chlorophényl)méthyl]-1-oxo-2(1*H*)-phtalazinyl]méthyl}-1-pyrrolidinyl)propyl]-acétamide ;
le *N*-[3-((2*R*)-2-{[4-[(4-chlorophényl)méthyl]-1-oxo-2(1*H*)-phtalazinyl]méthyl}-1-pyrrolidinyl)propyl]-propanamide ;
le *N*-[3-((2*R*)-2-{[4-[(4-chlorophényl)méthyl]-1-oxo-2(1*H*)-phtalazinyl]méthyl}-1-pyrrolidinyl)propyl]-2-méthylpropanamide ;
le *N*-[3-((2*R*)-2-{[4-[(4-chlorophényl)méthyl]-1-oxo-2(1*H*)-phtalazinyl]méthyl}-1-pyrrolidinyl)propyl]-2,2-diméthylpropanamide ;
le *N*-[2-((2*R*)-2-{[4-[(4-chlorophényl)méthyl]-1-oxo-2(1*H*)-phtalazinyl]méthyl}-1-pyrrolidinyl)éthyl]-2,2,2-trifluoroacétamide ;
le *N*-[2-((2*R*)-2-{[4-[(4-chloraphényl)méthyl]-1-oxo-2(1*H*)-phtalazinyl]méthyl}-1-pyrrolidinyl)éthyl]-2,2,2-trifluoro-N-méthylacétamide ;
le *N*-[2-((2*R*)-2-{[4-[(4-chlorophényl)méthyl]-1-oxo-2(1*H*)-phtalazinyl]méthyl}-1-pyrrolidinyl)éthyl]-N-méthyl-4-(méthyloxy)butanamide ;
le *N*-[2-((2*R*)-2-{[4-[(4-chlorophényl)méthyl]-1-oxo-2(1*H*)-phtalazinyl]méthyl}-1-pyrrolidinyl)éthyl]-cyclopentanecarboxamide ;
le *N*-[2-((2*R*)-2-{[4-[(4-chlorophényl)méthyl]-1-oxo-2(1*H*)-phtalazinyl]méthyl}-1-pyrrolidinyl)éthyl]-tétrahydro-2*H*-pyrane-4-carboxamide ;
le *N*-[2-((2*R*)-2-{[4-[(4-chlorophényl)méthyl]-1-oxo-2(1*H*)-phtalazinyl]méthyl}-1-pyrrolidinyl)éthyl]-3-hydroxy-2,2-diméthylpropanamide ;
le *N*-[2-((2*R*)-2-{[4-[(4-chlorophényl)méthyl]-1-oxo-2(1*H*)-phtalazinyl]méthyl}-1-pyrrolidinyl)éthyl]-tétrahydro-2*H*-pyrane-3-carboxamide ;
le *N*-[2-((2*R*)-2-{[4-[(4-chlorophényl)méthyl]-1-oxo-2(1*H*)-phtalazinyl]méthyl}-1-pyrrolidinyl)éthyl]-tétrahydro-3-furanecarboxamide ;
le *N*-[2-((2*R*)-2-{[4-[(4-chlorophényl)méthyl]-1-oxo-2(1*H*)-phtalazinyl]méthyl}-1-pyrrolidinyl)éthyl]-cyclohexanecarboxamide ;
le 2-((2*R*)-2-{[4-[(4-chlorophényl)méthyl]-1-oxo-2(1*H*)-phtalazinyl]méthyl}-1-pyrrolidinyl)-*N*-(2,2-diméthylpropyl)acétamide ;
le 2-((2*R*)-2-{[4-[(4-chlorophényl)méthyl]-1-oxo-2(1*H*)-phtalazinyl]méthyl}-1-pyrrolidinyl)-*N-*(cyclohexylméthyl)acétamide ;
la 4-[(4-chlorophényl)méthyl]-2-({(2*R*)-1-[(2-oxo-3-pipéridinyl)méthyl]-2-pyrrolidinyl}méthyl)-1(2*H*)-phtalazinone ;
la 4-[(4-chlorophényl)méthyl]-2-{[(2*R*)-1-(2-{3-[2-(méthyloxy)éthyl]-1,2,4-oxadiazol-5-yl}éthyl)-2-pyrrolidinyl]méthyl}-1(2*H*)-phtalazinone ;
la 4-[(4-chlorophényl)méthyl]-2-({(2*R*)-1-[(3-propyl-1,2,4-oxasiazol-5-yl)méthyl]-2-pyrrolidinyl}-méthyl)-1(2*H*)-phtalazinone ;
la 4-[(4-chlorophényl)méthyl]-2-[((2*R*)-1-{2-[3-(1-méthyléthyl)-1,2,4-oxadiazol-5-yl]éthyl}-2-pyrrolidinyl)méthyl]-1(2*H*)-phtalazinone ;
la 4-[(4-chlorophényl)méthyl]-2-({(2*R*)-1-[2-(3-propyl-1,2,4-oxadiazol-5-yl)éthyl]-2-pyrrolidinyl}-méthyl)-1(2*H*)-phtalazinone ;
le 3-((2*R*)-2-{[4-[(4-chlorophényl)méthyl]-1-oxopyrido[3,4-*d*]pyridazin-2(1*H*)-yl]méthyl}-1-pyrrolidinyl)-*N*-[3-(méthyloxy)propyl]propanamide ;
le 3-((2*R*)-2-{[4-[(4-chlorophényl)méthyl]-1-oxopyrido[3,4-*d*]pyridazin-2(1*H*)-yl]méthyl}-1-pyrrolidinyl)-*N*-(2,2-difluoroéthyl)propanamide ;
le 3-((2*R*)-2-{[4-[(4-chlorophényl)méthyl]-1-oxopyrido[3,4-*d*]pyridazin-2(1*H*)-yl]méthyl}-1-pyrrolidinyl)-*N*-propylpropanamide ;
le 3-((2*R*)-2-{[4-[(4-chlorophényl)méthyl]-1-oxopyrido[3,4-*d*]pyridazin-2(1*H*)-yl]méthyl}-1-pyrrolidinyl)-*N*-éthyl-*N*-méthylpropanamide ;
la 4-[(4-chlorophényl)méthyl]-2-({(2*R*)-1-[3-oxo-3-(1-pipéridinyl)propyl]-2-pyrrolidinyl}méthyl)pyrido-[3,4-*d*]pyridazin-1(2*H*)-one ;
le 3-((2*R*)-2-{[4-[(4-chlorophényl)méthyl]-1-oxopyrido[3,4-*d*]pyridazin-2(1*H*)-yl]méthyl}-1-pyrrolidinyl)-*N*-(2,2,2-trifluoroéthyl)propanamide ;
le *N*-[2-((2*R*)-2-{[4-[(4-chlorophényl)méthyl]-1-oxopyrido[3,4-*d*]pyridazin-2(1*H*)-yl]méthyl}-1-pyrrolidinyl)éthyl]-3-(méthyloxy)propanamide ;
le *N*-[2-((2*R*)-2-{[4-[(4-chlorophényl)méthyl]-1-oxopyrido[3,4-*d*]pyridazin-2(1*H*)-yl]méthyl}-1-pyrrolidinyl)éthyl]-4-(méthyloxy)butanamide ;
le *N*-[3-((2*R*)-2-{[4-[(4-chlorophényl)méthyl]-1-oxopyrido[3,4-*d*]pyridazin-2(1*H*)-yl]méthyl}-1-pyrrolidinyl)propyl]-3-(méthyloxy)propanamide ;
le ((2*R*)-2-{[4-[(4-chlorophényl)méthyl]-1-oxopyrido[3,4-*d*]pyridazin-2(1*H*)-yl]méthyl}-1-pyrrolidinyl)propyl]-4-(méthyloxy)butanamide ;
le *N*-[2-((2*R*)-2-{[4-[(4-chlorophényl)méthyl]-1-oxopyrido[3,4-*d*]pyridazin-2(1*H*)-yl]méthyl}-1-pyrrolidinyl)éthyl]propanamide ;
le *N*-[2-((2*R*)-2-{[4-[(4-chlorophényl)méthyl]-1-oxopyrido[3,4-*d*]pyridazin-2(1*H*)-yl]méthyl}-1-pyrrolidinyl)éthyl]butanamide ;
le *N*-[2-((2*R*)-2-{[4-[(4-chlorophényl)méthyl]-1-oxopyrido[3,4-*d*]pyridazin-2(1*H*)-yl]méthyl}-1-pyrrolidinyl)éthyl]-2,2-diméthylpropanamide ;
le *N*-[2-((2*R*)-2-{[4-[(4-chlorophényl)méthyl]-1-oxopyrido[3,4-*d*]pyridazin-2(1*H*)-yl]méthyl}-1-pyrrolidinyl)éthyl]-2-méthylpropanamide ;
le *N*-[3-((2*R*)-2-{[4-[(4-chlorophényl)méthyl]-1-oxopyxido[3,4-*d*]pyridazin-2(1*H*)-yl]méthyl}-1-pyrrolidinyl)propyl]propanamide ;
le *N*-[3-((2*R*)-2-{[4-[(4-chlorophényl)méthyl]-1-oxopyrido[3,4-*d*]pyridazin-2(1*H*)-yl]méthyl}-1-pyrrolidinyl)propyl]propanamide ;
le *N*-[3-((2*R*)-2-{[4-[(4-chlorophényl)méthyl]-1-oxopyrido[3,4-*d*]pyridazin-2(1*H*)-yl]méthyl}-1-pyrrolidinyl)propyl]-2-méthylpropanamide ;
le *N*-[3-((2*R*)-2-{[4-[(4-chlorophényl)méthyl]-1-oxopyrido[3,4-*d*]pyridazin-2(1*H*)-yl]méthyl}-1-pyrrolidinyl)propyl]-2,2-diméthylpropanamide ;
la 4-[(4-chlorophényl)méthyl]-2-({(2*R*)-1-[2-(3-méthyl-1,2,4-oxadiazol-5-yl)éthyl]-2-pyrrolidinyl}-méthyl)pyrido[3,4-*d*]pyridazin-1(2*H*)-one ;
la 4-[(4-chlorophényl)méthyl]-2-({(2*R*)-1-[2-(3-éthyl-1,2,4-oxadiazol-5-yl)éthyl]-2-pyrrolidinyl}-méthyl)pyrido[3,4-*d*]pyridazin-1(2*H*)-one ;
la 4-[(4-chlorophényl)méthyl]-2-({(2*R*)-1-(2-{3-[(méthyloxy)méthyl]-1,2,4-oxadiazol-5-yl}éthyl)-2-pyrrolidinyl]méthyl}pyrido[3,4-*d*]pyridazin-1(2*H*)-one ;
la 4-[(4-chlorophényl)méthyl]-2-({(2*R*)-1-[2-(3-propyl-1,2,4-oxadiazol-5-yl)êthyl]-2-pyrrolidinyl}-méthyl)pyrido[3,4-*d*]pyridazin-1(2*H*)-one ;
ou un sel de celui-ci.

10. Composé selon l'une quelconque des revendications 1 à 9 ou sel pharmaceutiquement acceptable de celui-ci.

11. Composé selon l'une quelconque des revendications 1 à 9 ou sel pharmaceutiquement acceptable de celui-ci en vue d'une utilisation en thérapie.

12. Composé selon l'une quelconque des revendications 1 à 9 ou sel pharmaceutiquement acceptable de celui-ci en vue d'une utilisation dans le traitement de maladies inflammatoires et/ou allergiques.

13. Composé selon l'une quelconque des revendications 1 à 9 ou sel pharmaceutiquement acceptable de celui-ci en vue d'une utilisation dans le traitement d'une rhinite allergique.

14. Composition pharmaceutique qui comprend un composé selon l'une quelconque des revendications 1 à 9 ou un sel pharmaceutiquement acceptable de celui-ci, éventuellement avec un ou plusieurs supports et/ou excipients pharmaceutiquement acceptables.

15. Combinaison comprenant un composé selon l'une quelconque des revendications 1 à 9 ou un sel pharmaceutiquement acceptable de celui-ci et un ou plusieurs autres composés thérapeutiques.
